(19) Europäisches Patentamt
European Patent Office
Office européen des brevets

(11) Veröffentlichungsnummer: **0 452 266 B1**

(12) **EUROPÄISCHE PATENTSCHRIFT**

(45) Veröffentlichungstag der Patentschrift: **18.01.95**

(51) Int. Cl.$^6$: **C07D 513/04**, C07D 487/06, C08K 5/3417, C07D 487/04

(21) Anmeldenummer: **91810243.5**

(22) Anmeldetag: **03.04.91**

(54) **Isoindolinon-Verbindungen als Stabilisatoren für organische Materialien.**

(30) Priorität: **12.04.90 CH 1283/90**

(43) Veröffentlichungstag der Anmeldung:
**16.10.91 Patentblatt 91/42**

(45) Bekanntmachung des Hinweises auf die Patenterteilung:
**18.01.95 Patentblatt 95/03**

(84) Benannte Vertragsstaaten:
**BE DE FR GB IT NL**

(56) Entgegenhaltungen:
**GB-A- 887 272**
**GB-A- 1 258 351**

(73) Patentinhaber: **CIBA-GEIGY AG**
**Klybeckstrasse 141**
**CH-4002 Basel (CH)**

(72) Erfinder: **Nesvadba, Peter, Dr.**
**Route du Nord 5**
**CH-1723 Marly (CH)**

EP 0 452 266 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Zusammensetzungen, enthaltend ein oxidativem, thermischem oder/und actinischem Abbau unterliegendes organisches Material und mindestens eine Isoindolinon-Verbindung. Weitere Gegenstände der Erfindung sind die Verwendung dieser Verbindungen als Additive zur Stabilisierung von organischem Material sowie neue Isoindolinon-Verbindungen.

Es wurden bereits Benzofuranon(2)- und Indolinon(2)-Verbindungen als Stabilisatoren in polymeren, organischen Materialien vorgeschlagen (WO-A 80/01566). Kem(per-)chlorierte Indolinon-, Isoindolinon- und Indolindion-Verbindungen wurden in DE-A 3 230 593 als Hochdruckzusatzstoffe für Schmiermittel benannt. Bestimmte Isoindolinone wurden in DE-A 1 770 439 als UV-Stabilisatoren vorgeschlagen.

Isoindolinon-Verbindungen mit einem weiteren, ankondensierten (Hydro)-Pyridinring wurden 1972 beschrieben (C.H. Gaozza et al., J. Het. Chem. 9, 883(1972)), und zwar mit dem Ziel, ihre pharmakologische Aktivität zu überprüfen. Ähnliche Verbindungen wurden später auch auf ihre Eignung als DNA Intercalatoren untersucht (S.E. Piatti ei al. Anales Asoc. Quim. Argentina 70, 651 (1982)). Ferner werden in US-A 3,591,599 polycyclische, schwefelhaltige Verbindungen als entzündungshemmende und schmerzstillende Mittel vorgeschlagen, die das Isoindolinon-Ringsystem enthalten. Es wurden auch pharmazeutisch wirksame Isoindolinonderivate hergestellt, deren bekanntestes das Etomidolin, ein Spasmolyticum, ist (Merck Index, 10th ed., 3829, US-A 3,624, 206). Weiterhin ist die Verwendung von Isoindolinon-Abkömmlingen mit ankondensiertem (Benzo)thiophenring als Trübungsverhinderer in photographischen Silberhalogenid-Emulsionen bekannt (US-A 2,860,985). Außerdem ist eine große Anzahl von Dokumenten bekannt, die 8,9,10,11-Tetrahydro-12-phthaloperinone als Farbstoffe beschreiben. Siehe als Beispiel DE-A 1 569 613.

Weitere polycyclische Verbindungen, die ein ankondensiertes Isoindol-Ringsystem enthalten, sind z.B. in folgenden Literaturstellen untersucht worden: Liebigs Ann. Chem. 1985, 657; J. Org. Chem. 34 (1969), 165; Chem. Pharm. Bull. 20 (1972), 69; Chem. Pharm. Bull. 22 (1974), 2142; US-A 3,994,920; DE-A 1 670 446; US-A 3,507,867; DE-A 2 062 022.

Im Vorliegenden werden nun erstmals Isoindolinon-Verbindungen mit ankondensierten Ringen in Zusammensetzungen mit organischen Materialien zur Verbesserung deren Stabilität und Verarbeitbarkeit vorgeschlagen.

Weiterhin wurden neue Isoindolinon-Verbindungen aufgefunden, die sich ebenfalls ausgezeichnet zum Schutz von organischem Material vor thermischem, oxidativem oder/und actinischem Abbau eignen.

Gegenstand der vorliegenden Erfindung sind Zusammensetzungen enthaltend ein thermischem, oxidativem und/oder actinischem Abbau unterliegendes organisches Material und mindestens eine Verbindung der Formel (I)

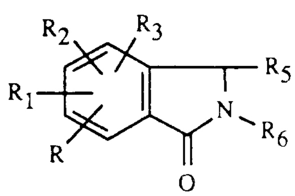

(I),

worin R Wasserstoff, $C_1$-$C_{18}$-Alkyl, $C_1$-$C_{18}$-Alkoxy, Halogen, Nitro, Phenyl-$C_1$-$C_4$-alkoxy, $C_2$-$C_{18}$-Alkanoyl, Benzoyl, ($C_1$-$C_6$-Alkyl)-benzoyl, $C_2$-$C_{18}$-Alkenoyl, -N($R_7$)($R_{7a}$), -OH oder -CO-A ist,

$R_1$ die gleichen Bedeutungsmöglichkeiten wie R hat,

$R_2$ Wasserstoff oder $C_1$-$C_4$-Alkyl und

$R_3$ Wasserstoff oder Halogen bedeuten,

A Hydroxy, $C_1$-$C_{18}$-Alkoxy, $C_5$-$C_{12}$-Cycloalkoxy, Benzyloxy oder mit $C_1$-$C_6$-Alkyl und/oder Halogen substituiertes Benzyloxy, -N($R_7$)($R_{7a}$), -NH-NH-$R_8$ oder -$G_1$-$(G)_n$ ist, n eine Zahl von 1 bis 3 und G einen Rest der Formel

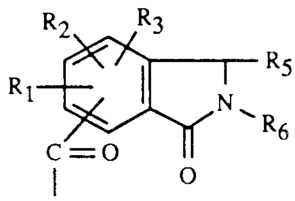

(II)

bedeuten,
$G_1$ im Fall n = 1 fur $-O-R_{20}O-$, $-NH-R_{21}-NH-$ oder -NH-NH-,
im Fall n = 2 für eine Gruppe der Formel

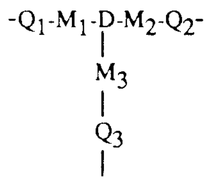

(III)

und im Fall n = 3 für $(-O-CH_2)_4C$ oder

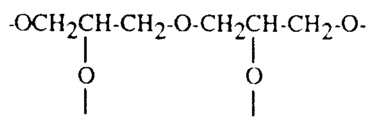

steht, wobei D N, CH oder $C(C_1-C_4\text{-Alkyl})$ ist, $Q_1$, $Q_2$ und $Q_3$ unabhängig voneinander O oder NH und $M_1$, $M_2$ und $M_3$ unabhängig voneinander $C_1-C_6$-Alkylen oder durch -O-unterbrochenes $C_2-C_{12}$-Alkylen bedeuten, oder im Falle D = CH oder $C(C_1-C_4\text{-Alkyl})$ $-M_3-Q_3$ auch für -O- oder im Falle D = N $-M_3-Q_3$ auch für eine direkte Bindung steht, $R_5$ und $R_6$ zusammen eine Gruppe der Formel

(IV) 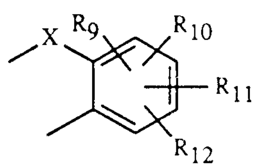 , 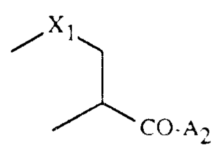 (V),

(VI) 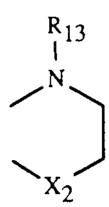 oder 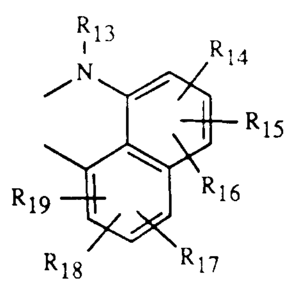 (VII)

bilden,
X -O-, -S-,

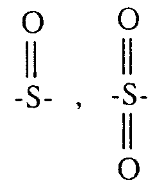

oder

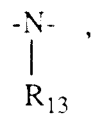

$X_1$ -S-,

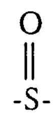

oder

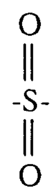

und
$X_2$ $-CH_2-$ oder eine direkte Bindung bedeuten,
$R_7$ und $R_{7a}$ unabhängig voneinander Wasserstoff, $C_1$-$C_{18}$-Alkyl, Phenyl-$C_1$-$C_4$-alkyl, $C_2$-$C_{18}$-Alkanoyl, $C_2$-$C_{18}$-Alkenoyl, $C_2$-$C_4$-Hydroxyalkyl, Benzoyl oder ($C_1$-$C_6$-Alkyl)-benzoyl, oder zusammen mit dem N-Atom, an das sie gebunden sind, Piperidyl, Morpholinyl, Piperazinyl oder 4-Methylpiperazinyl bedeuten,
$R_8$ Wasserstoff, $C_2$-$C_{18}$-Alkanoyl, Benzoyl oder ($C_1$-$C_6$-Alkyl)-benzoyl ist,
$R_9$, $R_{10}$, $R_{11}$ und $R_{12}$ die gleichen Bedeutungsmöglichkeiten wie R, $R_1$, $R_2$ und $R_3$ (in dieser Reihenfolge) haben, wobei an Stelle von -CO-A -CO-$A_1$ zu setzen ist,
$R_{13}$ Wasserstoff, $C_1$-$C_{18}$-Alkyl, $C_2$-$C_{18}$-Alkenyl, $C_5$-$C_{12}$-Cycloalkyl, Phenyl-$C_1$-$C_4$-alkyl, $C_2$-$C_{18}$-Alkanoyl, $C_2$-$C_{18}$-Alkenoyl, Benzoyl, ($C_1$-$C_6$-Alkyl)-benzoyl, Cyano-$C_1$-$C_4$-alkyl, $C_2$-$C_{19}$-Alkoxycarbonyl-$C_1$-$C_4$-alkyl, Carboxy-$C_1$-$C_4$-alkyl, Hydroxy-$C_1$-$C_4$-alkyl oder

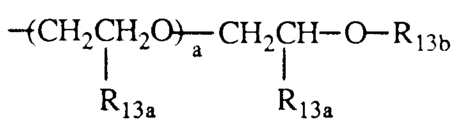

bedeutet,
$R_{13a}$ für Wasserstoff oder Methyl und $R_{13b}$ für Wasserstoff, $C_1$-$C_{18}$-Alkyl, Phenyl-$C_1$-$C_4$-alkyl, $C_1$-$C_{18}$-Alkanoyl, Benzoyl, $C_2$-$C_{18}$-Alkenoyl oder ($C_1$-$C_6$-Alkyl)-benzoyl und a für eine Zahl von 0 bis 6 stehen,
$R_{14}$, $R_{15}$, $R_{16}$, $R_{17}$, $R_{18}$ und $R_{19}$ unabhängig voneinander für Wasserstoff, $C_1$-$C_4$-Alkyl, Halogen, Hydroxy,

$C_1$-$C_{18}$-Alkoxy oder -N($R_{7b}$)($R_{7c}$) stehen, worin $R_{7b}$ und $R_{7c}$ unabhängig voneinander Wasserstoff oder $C_1$-$C_{18}$-Alkyl bedeuten,
$R_{20}$ $C_1$-$C_{12}$-Alkylen, $C_2$-$C_8$-Alkenylen, $C_3$-$C_6$-Alkinylen oder durch O, S oder/und -N($R_{22}$)- unterbrochenes $C_2$-$C_{24}$-Alkylen,
$R_{21}$ $C_1$-$C_{12}$-Alkylen und $R_{22}$ Wasserstoff, $C_1$-$C_{18}$-Alkyl, Phenyl, ($C_1$-$C_6$-Alkyl)-phenyl oder Phenyl-$C_1$-$C_4$-alkyl bedeuten, $A_1$ Hydroxyl, $C_1$-$C_{24}$-Alkoxy, $C_5$-$C_{12}$-Cycloalkoxy, Benzyloxy oder mit $C_1$-$C_6$-Alkyl und/oder Halogen substituiertes Benzyloxy, -N($R_7$)($R_{7a}$),-NH-NH-$R_8$ oder -$G_1$-$(G_2)_n$ ist, und $A_2$ Hydroxyl, $C_1$-$C_{24}$-Alkoxy, $C_5$-$C_{12}$-Cycloalkoxy, Benzyloxy oder mit $C_1$-$C_6$-Alkyl und/oder Halogen substituiertes Benzyloxy, -N($R_7$)($R_{7a}$),-NH-NH-$R_8$ oder -$G_1$-$(G_3)_n$ ist, wobei $G_1$ und n wie oben definiert sind,
$G_2$ für einen Rest der Formel

(VIII) 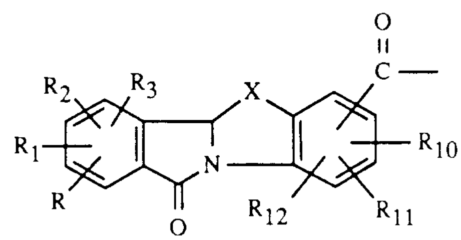

und
$G_3$ für einen Rest der Formel

(IX) 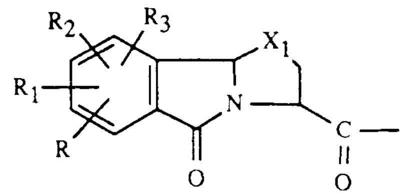

steht,
mit der Maßgabe, daß im Molekül nur ein Substituent -CO-A, CO-$A_1$ bzw. -CO-$A_2$ vorhanden ist, in dem A, $A_1$ bzw. $A_2$ einen Rest -$G_1$-$(G)_n$, -$G_1$-$(G_2)_n$ bzw. $G_1$-$(G_3)_n$ darstellt.

Stellen in obigen Formeln R, $R_1$, $R_9$ $R_{10}$, $R_7$, $R_{7a}$, $R_{7b}$, $R_{7c}$, $R_{13}$, $R_{13b}$, oder $R_{22}$ $C_1$-$C_{18}$-Alkyl dar, so handelt es sich dabei um verzweigte oder unverzweigte Reste. Beispiele hierfür sind Methyl, Ethyl, Propyl, Isopropyl, n-Butyl, Isobutyl, t-Butyl, Pentyl, Isopentyl, Hexyl, Heptyl, 3-Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl oder 1-Methylundecyl; die Reste R, $R_1$, $R_2$, $R_7$, $R_{7a}$, $R_{7b}$, $R_{7c}$, $R_9$, $R_{10}$, $R_{13b}$ und $R_{22}$ weisen dabei vorzugsweise 1-12, insbesondere 1-4 C-Atome auf. Sind $R_2$, $R_{14}$ bis $R_{19}$ $C_1$-$C_4$-Alkyl, kommen dafür z.B. Methyl, Ethyl, Propyl, Isopropyl, n-Butyl, Isobutyl und t-Butyl in Betracht. Weitere hier nicht näher aufgeführte Alkylgruppen in obigen Formeln weisen die gleichen beispielhaften Bedeutungen auf.

Beispiele für $C_1$-$C_{18}$ (bzw. $C_1$-$C_{24}$)-Alkoxygruppen R, $R_1$, $R_9$, $R_{10}$, A, $A_1$, $A_2$ und $R_{14}$-$R_{19}$ leiten sich von den vorstehend für $C_1$-$C_{18}$-Alkylgruppen aufgezählten Resten zuzüglich Eicosyl und Docosyl als weitere Beispiele ab und können formal durch Anhängen des Suffix -oxy definiert werden.

Bedeuten Substituenten in Formel I Halogen, ist darunter Cl, Br, F und J, insbesondere Cl und Br, vor allem Cl zu verstehen.

R, $R_1$, $R_7$, $R_{7a}$, $R_8$, $R_9$, $R_{10}$, $R_{13}$ und $R_{13b}$ als $C_2$-$C_{18}$-Alkanoyl sind Gruppen der Formel -CO-$C_1$-$C_{17}$-Alkyl. Beispiele für solche Reste sind erhältlich, indem man die vorstehend für $C_1$-$C_{18}$-Alkyl angeführten Reste einsetzt (ohne Octadecyl). Für die Reste R, $R_1$, $R_9$ und $R_{10}$ ist $C_2$-$C_5$-Alkanoyl bevorzugt.

R, $R_1$, $R_9$ und $R_{10}$ als Phenyl-$C_1$-$C_4$-alkoxy ist beispielsweise Benzyloxy, Phenethoxy, $\alpha$-Methylbenzyloxy, oder $\alpha$,$\alpha$-Dimethylbenzyloxy. Bevortugt ist Benzyloxy. Die gleichen Substituenten sowie $R_7$, $R_{7a}$, $R_8$, $R_{13}$ und $R_{13b}$ als ($C_1$-$C_6$-Alkyl)benzoyl können im Phenylrest z.B. 1-3, vor allem 1 oder 2, insbesondere 1 $C_1$-$C_6$-Alkylgruppe(n) tragen. Beispiele für Alkyl sind weiter oben angegeben. Bevorzugt ist dabei $C_1$-$C_4$-Alkyl, insbesondere Methyl.

$R_{13}$ als $C_2$-$C_{18}$-Alkenyl kann verzweigt oder unverzweigt sein und beispielsweise Vinyl, Allyl, 2-Methallyl, Hexenyl, Undecenyl, Heptadecenyl und Oleyl bedeuten.

R, $R_1$, $R_7$, $R_{7a}$, $R_9$, $R_{10}$, $R_{13}$ und $R_{13b}$ als $C_3$-$C_{18}$-Alkenoyl bedeuten Reste der Formel -CO-$C_2$-$C_{17}$-Alkenyl. Beispiele für solche Reste sind erhältlich, indem man die vorstehend für $C_2$-$C_{18}$-Alkenyl angeführten Reste einsetzt (ohne Oleyl).

$R_{13}$ als $C_5$-$C_{12}$-Cycloalkyl kann z.B. Cyclopentyl, Cyclohexyl, Cycloheptyl, Cyclooctyl oder Cyclododecyl sein. Cyclopentyl und Cyclohexyl ist bevorzugt, insbesondere Cyclohexyl.

$R_{13}$ als $C_2$-$C_{19}$-Alkoxycarbonyl-$C_1$-$C_4$-alkyl ist insbesondere $C_2$-$C_{19}$-Alkyloxycarbonylmethyl. Der Alkyoxyteil hat vorzugsweise 1 bis 12 C-Atome.

Cycloalkoxy als Substituent A, $A_1$ bzw. $A_2$ ist z.B. Cyclopentyloxy, Cyclohexyloxy, Cyclooctyloxy, Cycloheptyloxy oder Cyclododecyloxy, wobei Cyclopentyloxy und insbesondere Cyclohexyloxy bevorzugt sind.

Substituiertes Benzyloxy als Substituent A, $A_1$ bzw. $A_2$ enthält z.B. 1 bis 3 Substituenten aus der Reihe Halogen (insbesondere Chlor) und $C_1$-$C_6$-Alkyl. Vorzugsweise sind entweder Halogen oder Alkyl als Substituenten vorhanden. Insbesondere sind 1 oder 2, vor allem 1 Substituent zweckmäßig. Alkyl ist vor allem $C_1$-$C_4$-Alkyl, insbesondere Methyl.

$C_1$-$C_6$-Alkylen (Substituenten $M_1$, $M_2$, $M_3$) bzw. $C_1$-$C_{12}$-Alkylen ($R_{20}$, $R_{21}$) kann geradkettig oder verzweigt sein und beispielsweise Methylen, Ethylen, Propylen, Butylen, Hexylen und für $R_{20}$, $R_{21}$ zusätzlich Octylen, Decylen und Dodecylen bedeuten, wobei geradkettige Reste bevorzugt sind.

$R_{20}$ als $C_2$-$C_8$-Alkenylen bzw. $C_3$-$C_6$-Alkinylen ist z.B. Vinylen, Butenylen, Hexenylen und Octenylen bzw. Butinylen.

Beispiele für durch -O- unterbrochenes $C_2$-$C_{12}$-Alkylen ($M_1$, $M_2$, $M_3$) bzw. $C_2$-$C_{24}$-Alkylen ($R_{20}$) sind etwa Methoxyethyl, Ethoxyethyl, Butoxyethyl, Butoxypropyl, $-CH_2OCH_2CH_2OCH_2CH_2-$, $-CH_2CH_2OCH_2CH_2-$, $-CH(CH_3)CH_2OCH_2CH(CH_3)-$,

$$-CH_2CH_2-(OCH_2CH_2)_n-$$

mit n = 1 bis 5 (für $M_1$, $M_2$, $M_3$) bzw. 1 bis 11 (für $R_{20}$).

Beispiele Für $R_{20}$ als durch -S- bzw. -N($R_{22}$)- unterbrochenes $C_2$-$C_{24}$-Alkylen sind Methylthiaethylen, Ethylthiapropylen, Octylthiapropylen bzw. $-CH_2CH_2NHC_4H_8-$, $-(CH_2)_3NHC_8H_{16}-$,

$$-(CH_2)_3-N(CH_3)CH_2CH(C_2H_5)C_4H_8- \ .$$

$R_7$, $R_{7a}$, $R_{13}$, $R_{13b}$ und $R_{22}$ als Phenyl-$C_1$-$C_4$-alkyl bedeutet z.B. Benzyl, Phenethyl, 3-Phenylpropyl, $\alpha$-Methylbenzyl oder $\alpha,\alpha$-Dimethylbenzyl. Bevorzugt ist Benzyl.

$C_2$-$C_4$-Hydroxyalkyl als Substituenten $R_7$, $R_{7a}$ und $R_{13}$ enthält z.B. 1 bis 4 OH-, z.B. 1-2, insbesondere 1 OH-Gruppe. Bevorzugt ist 2-Hydroxyethyl.

$R_{22}$ als ($C_1$-$C_6$-Alkyl)phenyl enthält z.B. 1-3, vor allem 1 oder 2, insbesondere 1 Alkylgruppe (vorzugsweise mit 1 bis 4 C-Atomen, insbesondere Methyl) am Phenylrest. Beispiele sind Tolyl, Dimethylphenyl, Trimethylphenyl, t-Butylphenyl und Di-t-butylphenyl.

Beispiele für Gruppen der Formel

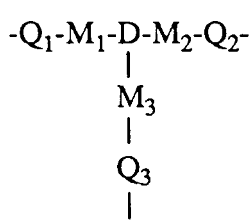

sind die folgenden:
$N(CH_2CH_2O-)_3$, $CH_3C(CH_2O-)_3$, $CH_3CH_2C(CH_2O-)_3$,

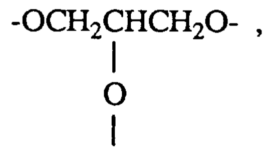

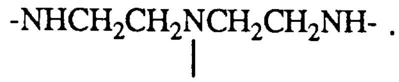

Zweckmäßig bedeuten R und $R_1$ bzw. $R_9$ und $R_{10}$ Wasserstoff, Hydroxy, $C_1$-$C_4$-Alkyl, Chlor, $C_1$-$C_4$-Alkoxy, Benzoyl, $N(R_7)(R_{7a})$ oder -CO-A, wobei $R_7$ und $R_{7a}$ zweckmäßig Wasserstoff, $C_1$-$C_6$-Alkyl, Benzyl, $C_2$-$C_4$-Hydroxyalkyl oder zusammen mit dem N-Atom, an das sie gebunden sind, Piperidyl, Morpholinyl, Piperazinyl oder 4-Methylpiperazinyl bedeuten. Zweckmäßige Verbindungen mit dem Rest -CO-A als R oder $R_1$ sind diejenigen, bei denen A Hydroxyl, $C_1$-$C_{18}$-Alkoxy, $C_5$-$C_6$-Cycloalkoxy, Benzyloxy oder mit $C_1$-$C_4$-Alkyl und/oder Chlor substituiertes Benzyloxy oder $N(R_7)(R_{7a})$ ist. $R_2$ und $R_3$ bzw. $R_{11}$ und $R_{12}$ sind vorzugsweise Wasserstoff. Besonders bevorzugt ist höchstens einer der Reste R, $R_1$, $R_2$ und $R_3$ bzw. $R_9$, $R_{10}$, $R_{11}$ und $R_{12}$ von Wasserstoff verschieden und bedeutet dann insbesondere Hydroxy, $C_1$-$C_4$-Alkyl, Chlor oder $C_1$-$C_4$-Alkoxy.

Von besonderem Interesse sind erfindungsgemäße Zusammensetzungen enthaltend Verbindungen der Formel I, worin
R Wasserstoff, Hydroxy, $C_1$-$C_4$-Alkyl, Chlor, Nitro, $C_1$-$C_4$-Alkoxy, Benzoyl, $-N(R_7)(R_{7a})$ oder -CO-A ist,
$R_1$ die gleichen Bedeutungsmöglichkeiten wie R hat,
$R_2$ Wasserstoff oder $C_1$-$C_4$-Alkyl und
$R_3$ Wasserstoff oder Chlor bedeuten,
A Hydroxyl, $C_1$-$C_{18}$-Alkoxy, $C_5$-$C_6$-Cycloalkoxy, Benzyloxy oder mit $C_1$-$C_4$-Alkyl und/oder Chlor substituiertes Benzyloxy, $-N(R_7)(R_{7a})$ oder $-G_1-(G)_n$ ist, n eine Zahl von 1 bis 3 und G einen Rest der Formel

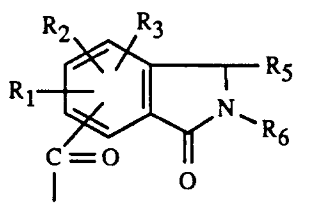

(II)

bedeuten,
$G_1$ im Fall n = 1 für $-O-R_{20}-O-$ oder $-NH-R_{21}-NH-$,
im Fall n = 2 für eine Gruppe der Formel

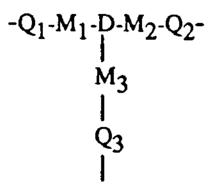

(III)

und im Fall n = 3 für $(-OCH_2)_4C$ steht, wobei D gleich N, CH oder $C(C_1$-$C_4$-Alkyl) ist,
$Q_1$, $Q_2$ und $Q_3$ unabhängig voneinander O oder NH und
$M_1$, $M_2$ und $M_3$ unabhängig voneinander $C_1$-$C_6$-Alkylen oder durch -O- unterbrochenes $C_2$-$C_{12}$-Alkylen bedeuten,
oder im Falle D = CH oder $C(C_1$-$C_4$-Alkyl) $-M_3-Q_3$ auch für -O- oder im Falle D = N $-M_3-Q_3$ auch für eine

direkte Bindung steht,

$R_5$ und $R_6$ zusammen eine Gruppe der Formel IV, V, VI oder VII bilden,

$R_7$ und $R_{7a}$ unabhängig voneinander Wasserstoff, $C_1$-$C_6$-Alkyl, Benzyl, $C_2$-$C_4$-Hydroxyalkyl, oder zusammen mit dem N-Atom, an das sie gebunden sind, Piperidyl, Morpholinyl, Piperazinyl oder 4-Methylpiperazinyl bedeuten,

$R_9$, $R_{10}$, $R_{11}$ und $R_{12}$ die gleichen Bedeutungsmöglichkeiten wie R, $R_1$, $R_2$ und $R_3$ (in dieser Reihenfolge) haben, wobei an Stelle von -CO-A -CO-$A_1$ zu setzen ist,

$R_{13}$ Wasserstoff, $C_1$-$C_{18}$-Alkyl, $C_2$-$C_{18}$-Alkenyl, $C_5$-$C_6$-Cycloalkyl, Phenyl-$C_1$-$C_4$-alkyl, $C_2$-$C_{18}$-Alkanoyl, $C_2$-$C_{18}$-Alkenoyl, Benzoyl, ($C_1$-$C_6$-Alkyl)-benzoyl, $C_2$-$C_{19}$-Alkoxycarbonyl-$C_1$-$C_4$-alkyl, Carboxy-$C_1$-$C_4$-alkyl, Cyano-$C_1$-$C_4$-alkyl oder Hydroxy-$C_2$-$C_4$-alkyl bedeutet,

$R_{14}$, $R_{15}$, $R_{16}$, $R_{17}$, $R_{18}$ und $R_{19}$ unabhängig voneinander fur Wasserstoff, $C_1$-$C_4$-Alkyl, Chlor, Hydroxy, $C_1$-$C_4$-Alkoxy oder -N($R_{7b}$)($R_{7c}$) stehen, worin $R_{7b}$ und $R_{7c}$ unabhängig voneinander Wasserstoff oder $C_1$-$C_6$-Alkyl bedeuten,

$R_{20}$ $C_1$-$C_{12}$-Alkylen, $C_2$-$C_8$-Alkenylen, $C_3$-$C_6$-Alkinylen oder durch O, S oder/und -N($R_{22}$) unterbrochenes $C_2$-$C_{24}$-Alkylen,

$R_{21}$ $C_1$-$C_{12}$-Alkylen und $R_{22}$ Wasserstoff oder $C_1$-$C_4$-Alkyl bedeuten, $A_1$ Hydroxy, $C_1$-$C_{18}$-Alkoxy, $C_5$-$C_6$-Cycloalkoxy, Benzyloxy oder mit $C_1$-$C_4$-Alkyl und/oder Halogen substituiertes Benzyloxy, -N($R_7$)($R_{7a}$) oder -$G_1$-$(G_2)_n$ ist und $A_2$ Hydroxy, $C_1$-$C_{18}$-Alkoxy, $C_5$-$C_6$-Cycloalkoxy, Benzyloxy oder mit $C_1$-$C_4$-Alkyl und/oder Halogen substituiertes Benzyloxy, -N($R_7$)($R_{7a}$) oder -$G_1$-$(G_3)_n$ ist, wobei $G_1$ und n wie oben definiert sind,

$G_2$ für einen Rest der Formel

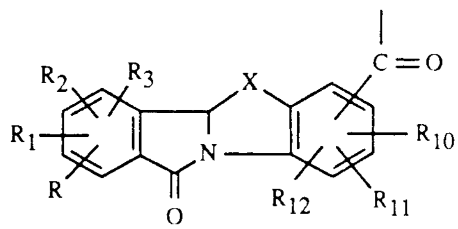

und

$G_3$ für einen Rest der Formel

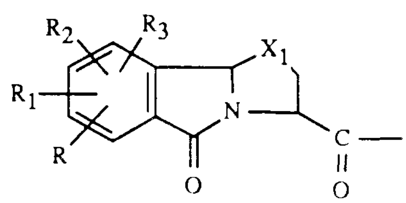

steht.

Zweckmäßige Zusammensetzungen enthalten Verbindungen der Formel I, worin

R Wasserstoff, Chlor, Hydroxy oder $C_1$-$C_4$-Alkyl ist,

$R_1$ Wasserstoff, Chlor, $C_1$-$C_4$-Alkyl oder Hydroxy,

$R_2$ und $R_3$ Wasserstoff sind,

$R_5$ und $R_6$ zusammen eine Gruppe der Formel IV, V, VI oder VII bilden,

$R_9$, $R_{10}$, $R_{11}$ und $R_{12}$ die gleichen Bedeutungsmöglichkeiten wie R, $R_1$, $R_2$ und $R_3$ (in dieser Reihenfolge) haben,

$R_{13}$ Wasserstoff, $C_1$-$C_{18}$-Alkyl, $C_5$-$C_6$-Cycloalkyl, Benzyl, $C_2$-$C_{19}$-Alkoxycarbonyl-$C_1$-$C_4$-alkyl, Carboxy-$C_1$-$C_4$-alkyl, $C_2$-$C_{18}$-Alkanoyl oder Benzoyl bedeutet,

$R_{14}$, $R_{15}$, $R_{16}$, $R_{17}$, $R_{18}$ und $R_{19}$ Wasserstoff sind und

$A_2$ Hydroxy, $C_1$-$C_{18}$-Alkoxy, $C_5$-$C_6$-Cycloalkoxy oder Benzyloxy bedeutet.

Bevorzugt sind Zusammensetzungen, die Verbindungen der Formel I enthalten, worin R und $R_9$ unabhängig voneinander Wasserstoff, $C_1$-$C_4$-Alkyl oder Chlor und $R_1$, $R_2$, $R_3$, $R_{10}$, $R_{11}$, $R_{12}$, $R_{14}$, $R_{15}$, $R_{16}$, $R_{17}$, $R_{18}$ und $R_{19}$ Wasserstoff bedeuten, sowie solche, worin A, $A_1$ und $A_2$ von -$G_1$-$(G)_n$, -$G_1$-$(G_2)_n$ bzw. -$G_1$-$(G_3)_n$ verschieden sind.

Besonders bevorzugte Zusammensetzungen enthalten Verbindungen der Formel I, worin R Wasserstoff, Methyl oder Chlor bedeutet, $R_1$, $R_2$ und $R_3$ Wasserstoff sind, und $R_5$ und $R_6$ zusammen eine Gruppe der Formeln IV, V, VI oder VII darstellen, worin X -S-,

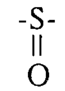

oder

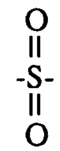 ,

$X_1$ -S-, $X_2$ $-CH_2-$, $R_9$ Wasserstoff, $-CH_3$, Cl oder -COO-($C_1$-$C_{24}$-Alkyl), $R_{10}$, $R_{11}$ und $R_{12}$ Wasserstoff, $R_{13}$ Wasserstoff, $C_1$-$C_{18}$-Alkyl, Benzoyl, $C_2$-$C_{19}$-Alkoxycarbonyl-methyl oder $C_2$-$C_{18}$-Alkanoyl, $R_{14}$ bis $R_{19}$ Wasserstoff und $A_2$ $C_1$-$C_{24}$-Alkoxy bedeuten.

Die Besonderheit von organischen Stoffen liegt im Gegensatz zu den meist bis zu hohen Temperaturen stabilen anorganischen Stoffen darin, daß sie sich leicht unter Einfluß von Wärme, Licht bzw. Strahlung, mechanischer Belastung (insbesondere durch Scherkräfte) und chemischen Reagentien (besonders Luftsauerstoff) zersetzen.

Dem Schutz vor solchen Einflüssen dienen die Verbindungen der Formel I, die in den erfindungsgemäßen Zusammensetzungen zweckmäßig zu 0.01 bis 10, beispielsweise zu 0.05 bis 5, vorzugsweise zu 0.05 bis 3, insbesondere jedoch zu 0.1 bis 2 Gew.-% in den organischen Materialien vorliegen sollen. Es kann sich dabei um eine oder mehrere dieser Verbindungen handeln, und die Gewichtsprozentangaben beziehen sich auf die gesamte Menge dieser Verbindungen. Berechnungsgrundlage ist dabei das Gesamtgewicht des organischen Materials ohne die Verbindungen der Formel I.

Die in den erfindungsgemäßen Zusammensetzungen enthaltenen Materialien sind solche, die gegen oxidativen, thermischen oder/und actinischen Abbau empfindlich sind. Als nicht unter diese organischen Materialien fallend sind lebende Organismen zu verstehen.

Vorzugsweise fallen unter die Definition "organisches Material" nicht: gelatine- und silberhalogenidhaltige photographische Emulsionen.

Beispielhaft für organische Materialien, die erfindungsgemäß mit Hilfe der Verbindungen der Formel I stabilisiert werden können, seien erwähnt:

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentvpen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-($\alpha$-methylstyrol).

5. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xyloldiamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Hamstoff oder Melamin andererseits ableiten, wie Phenol-Formaldehyd-, Harnstoff-Formaldehydund Melamin-Formaldehydharze.
21. Trocknende und nicht-trocknende Alkydharze.
22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
23. Vernetzbare Acrylharze, die sich von substituierten Acrylsäureestern ableiten, wie z.B. von Epoxyacrylaten, Urethan-acrylaten oder Polyester-acrylaten.
24. Alkydharze, Polyesterharze und Acrylatharze, die mit Melaminharzen, Harnstoffharzen, Polyisocyanaten oder Epoxidharzen vernetzt sind.
25. Vernetzte Epoxidharze, die sich von Polyepoxiden ableiten, z.B. von Bis-glycidylethern oder von cycloaliphatischen Diepoxiden.
26. Natürliche Polymere, wie Cellose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6/6 und Copolymere, PA/HDPE, PA/PP, PA/PPO.
28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wäßrige Emulsionen.
29. Wäßrige Emulsionen natürlicher oder synthetischer Kautschuke, wie z.B. Naturkautschuk-Latex oder Latices von carboxylierten Styrol-Butadien-Copolymeren.

Die erfindungsgemäßen Zusammensetzungen enthalten als organisches Material vorzugsweise natürliche, halbsynthetische oder synthetische Polymere, einen Schmierstoff, eine Metallbearbeitungsflüssigkeit oder eine Hydraulikflüssigkeit. Besonders bevorzugt sind Zusammensetzungen, die ein synthetisches Polymer enthalten, insbesondere einen thermoplastischen Kunststoff oder ein Elastomer. Vor allem sind Zusammensetzungen hervorzuheben, die ein Polyolefin als organisches Material enthalten. Beispiele für solche Polymere sind der vorstehenden Aufzählung von geeigneten Materialien zu entnehmen.

Ebenfalls bevorzugt sind Zusammensetzungen, die einen Schmierstoff, eine Metallbearbeitungsflüssigkeit oder eine Hydraulikflüssigkeit, insbesondere einen Schmierstoff, enthalten.

Die in Frage kommenden Schmierstoffe basieren beispielsweise auf mineralischen oder synthetischen Oelen oder Mischungen davon. Die Schmierstoffe sind dem Fachmann geläufig und in der einschlägigen Fachliteratur, wie beispielsweise in Dieter Klamann, "Schmierstoffe und verwandte Produkte" (Verlag Chemie, Weinheim, 1982), in ScheweKobek, "Das Schmiermittel-Taschenbuch" (Dr. Alfred Hüthig-Verlag, Heidelberg, 1974) und in "Ullmanns Enzyklopädie der technischen Chemie", Bd. 13, Seiten 85-94 (Verlag Chemie, Weinheim, 1977) beschrieben.

Die Schmierstoffe sind insbesondere Oele und Fette, beispielsweise basierend auf einem Mineralöl. Bevorzugt sind Oele.

Eine weitere Gruppe von Schmierstoffen, die zur Anwendung gelangen können, sind pflanziiche oder tierische Oele, Fette, Talge und Wachse oder deren Gemische untereinander oder Gemische mit den erwähnten mineralischen oder synthetischen Oelen. Pflanzliche und tierische Oele, Fette, Talge und Wachse sind beispielsweise Palmkernöl, Palmöl, Olivenöl, Rueböl, Rapsöl, Leinöl, Erdnußöl, Sojabohnenöl, Baumwollöl, Sonnenblumenöl, Kurbiskernöl, Kokosnußöl, Maisöl, Rizinusöl, Baumnußöl und Mischungen davon, Fischöle, Talge von Schlachttieren wie Rindertalg, Klauenfett und Knochenöl sowie deren modifizierte, epoxidierte und sulfoxidierte Formen, beispielsweise epoxidiertes Sojabohnenöl.

Die Mineralöle basieren insbesondere auf Kohlenwasserstoffverbindungen.

Beispiele von synthetischen Schmierstoffen umfassen Schmierstoffe auf der Basis der aliphatischen oder aromatischen Carboxylester, der polymeren Ester, der Polyalkylenoxide, der Phosphorsäureester, der Poly-$\alpha$-olefine oder der Silicone, eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropantricaprylat oder Gemische davon, eines Tetraesters von Pentaerythrit mit einer einwertigen Säure oder mit einem Gemisch solcher Säuren, wie z.B. Pentaerythrit-tetracaprylat, oder eines komplexen Esters von einwertigen und zweiwertigen Säuren mit mehrwertigen Alkoholen, z.B. ein komplexer Ester von Trimethylolpropan mit Capryl- und Sebacinsäure oder von einem Gemisch davon. Besonders geeignet sind neben Mineralölen z.B. Poly-α-Olefine, Schmierstoffe auf Esterbasis, Phosphate, Glykole, Polyglykole und Polyalkylenglykole, sowie deren Mischungen mit Wasser.

Metallbearbeitungsflüssigkeiten und Hydraulikflüssigketen können auf Basis der gleichen Substanzen hergestellt werden wie vorstehend für die Schmiermittel beschrieben. Häufig handelt es sich dabei auch um Emulsionen solcher Substanzen in Wasser oder anderen Flüssigkeiten.

Die Einarbeitung in die organischen Materialien kann beispielsweise durch Einmischen der Verbindungen der Formel I und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindungen auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der Verbindungen der Formel I in Polymere besteht in deren Zugabe vor oder während der Polymerisation der entsprechenden Monomeren bzw. vor der Vernetzung. Im Falle der Zugabe vor oder während der Polymerisation können die Verbindungen der Formel I auch als Regulatoren für die Kettenlänge der Polymeren (Kettenabbrecher) wirken.

Die Verbindungen der Formel I oder Mischungen davon können auch in Form eines Masterbatches, der diese Verbindungen beispielsweise in einer Konzentration von 2,5 bis 25 Gew.-% enthalt, den zu stabilisierenden Kunststoffen zugesetzt werden.

Zweckmäßig kann die Einarbeitung der Verbindungen der Formel I nach folgenden Methoden erfolgen:

- als Emulsion oder Dispersion (z.B. zu Latices oder Emulsionspolymeren)
- Als Trockenmischung während des Vermischens von Zusatzkomponenten oder Polymermischungen
- durch direktes Zugeben in die Verarbeitungsapparatur (z.B. Extruder, Innenmischer usw.)
- als Lösung oder Schmelze.

Erfindungsgemäße Polymerzusammensetzungen können in verschiedener Form angewendet bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Erfindungsgemäße Schmierstoffzusammensetzungen finden z.B. Verwendung in Verbrennungsmotoren, z.B. in Kraftfahrzeugen.

Zusätzlich zu den erfindungsgemäßen Verbindungen bzw. Mischungen können die erfindungsgemäßen Zusammensetzungen, insbesondere wenn sie organische, vorzugsweise synthetische, Polymere enthalten, noch weitere übliche Additive enthalten. Beispiele für solche Additive sind:

1. Antioxidantien

1.1. Alkylierte Monophenole, z.B. 2,6-Di-tert.butyl-4-methylphenol, 2-Tert.butyl-4,6-di-methylphenol, 2,6-Di-tert.butyl-4-ethylphenol, 2,6-Di-tert.butyl-4-n-butylphenol,2,6-Di-tert.butyl-4-i-butylphenol, 2,6-Dicyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butylhydrochinon, 2,5-Di-tert.amyl-hydrochinon, 2,6-Diphenyl-4-octadecyloxyphenol.

1.3. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert-butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.-butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methyl-phenyl)-3-n-dodecylmercaptobutan, Ethylenglycol-bis-[3,3-bis-(3'-tert.-butyl-4'-hydroxyphenyl)-butyrat], Bis-(3-tert.butyl-4-hydroxy-5-methyl-phenyl)-di-cyclopentadien, Bis-[2-(3'-tert.butyl-2'-hydroxy-5'-methyl-benzyl)-6-tert.-butyl-4-methyl-phenyl]-terephthalat.

1.5 Benzylverbindungen, z.B. 1,3,5-Tris-(3,5-di-tert.butyl-4-hydroxy-benzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxy-

benzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino )-s-triazin, N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octa- decanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.9 Ester der β-(3,5-Dicyclohexyl-4-hydroxyphenyl)-propionsäure mit ein- der mehrwertigen Alkoholen, wie z.B. mit Methanol, Octadecanol, 1,6-Hexandiol, Neopentylglycol, Thiodethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.10. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bi5-(3,5-di-tert.butyl-4-hydroxyphenylpropionyl)-trimethylendiamin, N,N'-Bis-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-, 3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-di-methoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyldiethanolamin, Nickeldibutyldithiocarbamat, Nickelsalze von 4-Hydroxy-3,5-di-tert.butylbenzylphosphonsäure-monoalkylestem, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(1,2,2,6,6-pentamethylpiperidyl)-sebacat, n-Butyl-3,5-di-tert.butyl-4-hydroxybenzylmalonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.-butyl-oxanilid, 2,2'-Di-dodecyloxy-5,5'di-tert.butyl-oxanilid, 2-Ethoxy-2'-ethyloxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylpheny1)-1,3,5-triazin, 2-(2-Hydroxy-4-dodecyloxyphenyl)-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrarzi n, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der $\beta$-Thio-dipropionsäure, beispielsweise der Lauryl-, Stearyl-, Myristyl- oder Tridecylester, Mercaptobenzimidazol, das Zinksälz des 2-Mercaptobenzimidazols, Zink-dibutyl-dithiocarbamat, Dioctadecyldisulfid, Pentaerythrit-tetrakis-($\beta$-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.

10. Sonstige Zusätze, wie z.B. Weichmacher, Gleitmittel, Emulgatoren, Pigmente, Optische Aufheller, Flammschutzmittel, Antistatika, Treibmittel.

Handelt es sich bei den erfindungsgemäßen Zusammensetzungen um solche auf Basis von Schmierstoffen und Hydraulikflüssigkeiten bzw. Metallbearbeitungsflüssigkeiten, können diese ebenfalls weitere Additive enthalten, die zugegeben werden, um gewisse Gebrauchseigenschaften zu verbessern, wie z.B. weitere Antioxidantien, Metalldesaktivatoren, Rostinhibitoren, Viskositäts-Index-Verbesserer, Stockpunkterniedriger, Dispergiermittel/Tenside und Verschleißschutzadditive.

Beispiele für Antioxidantien sind der weiter oben wiedergegebenen Auflistung unter dem Titel "1. Antioxidantien", insbesondere Punkte 1.1 bis 1.10 zu entnehmen. Beispiele für weitere zusätzliche Additive sind die folgenden:

Beispiele für aminische Antioxidantien:

N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylaminophenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, 2,3-Dihydro-3,3-dimethyl-4H-1,4-benzothiazin, Phenothiazin, N-Allylphenothiazin.

Beispiele für weitere Antioxidantien: Aliphatische oder aromatische Phosphite, Ester der Thiodipropionsäure oder der Thiodiessigsäure, oder Salze der Dithiocarbamid- oder Dithiophosphorsäure.

Beispiele für Metall-Desaktivatoren, z.B. für Kupfer, sind: Triazole, Benztriazole und deren Derivate, Tolutriazole und deren Derivate, 2-Mercaptobenzthiazol, 2-Mercaptobenztriazol, 2,5-Dimercaptobenztriazol, 2,5-Dimercaptobenzthiadiazol,5,5'-Methylenbisbenztriazol, 4,5,6,7-Tetrahydrobenztriazol, Saticyliden-propylendiamin, Salicylaminoguanidin und dessen Salze.

Beispiele für Rost-Inhibitoren sind:

a) Organische Säuren, ihre Ester, Metallsalze und Anhydride, z.B.: N-Oleoyl-sarcosin, Sorbitan-monooleat, Blei-naphthenat, Alkenylbersteinsäureanhydrid, z.B. Dodecenylbernsteinsäure-anhydrid, Alkenylbernsteinsäure-Teilester und -Teilamide, 4-Nonylphenoxy-essigsäure.

b) Stickstofffialtige Verbindungen, z.B.:

i. Primäre, sekundäre oder tertiäre aliphatische oder cycloaliphatische Amine und AminSalze von organischen und anorganischen Säuren, z.B. öllösliche Alkylammoniumcarboxylate.

ii. Heterocyclische Verbindungen, z.B.: Substituierte Imidazoline und Oxazoline.

c) Phosphorhaltige Verbindungen, z.B.: Aminsalze von Phosphorsäurepartialestern oder Phosphonsäurepartialestern, Zinkdialkyldithiophosphate.

d) Schwefelhaltige Verbindungen, z.B.: Barium-dinonylnaphthalin-sulfonate, Calciumpetroleum-sulfonate.

Beispiele für Viskositätsindex-Verbesserer sind: Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunkterniedriger sind: Polymethacrylat, alkylierte Naphthalinderivate.

Beispiele für Dispergiermittel/Tenside sind: Polybutenylbernsteinsäureamide oder -imide, Polybutenylphosphonsäurederivate, basische Magnesium-, Calcium-, und Bariumsulfonate und -phenolate.

Beispiele für Verschleißschutz-Additive sind: Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldi- und tri-sulfide, Triphenylphosphorothionate, Diethanolaminomethyltolyltriazol, Di(2-ethylhexyl)aminomethyltolyltriazol.

Besonders vorteilhaft ist es, die Verbindungen der Formel I in Kombination mit organischen Phosphiten oder Phosphoniten einzusetzen, insbesondere bei der Stabilisierung von thermoplastischen Polymeren. Gegenstand der Erfindung sind daher auch Zusammensetzungen, die mindestens eine Verbindung der Formel I und mindestens ein organisches Phosphit oder/und Phosphonit enthalten. Beispiele für solche Phosph(on)ite sind solche der Formeln

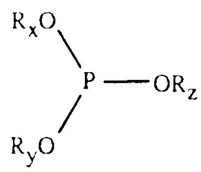

(A)

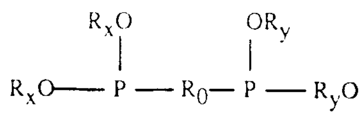

(B)

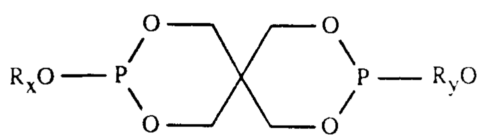

(C)

worin $R_x$, $R_y$ und $R_z$ unabhängig voneinander $C_6$-$C_{20}$-Alkyl, $C_5$-$C_8$-Cycloalkyl, Phenyl oder durch ein bis drei $C_1$-$C_{12}$-Alkyl substituiertes Phenyl bedeutet und

$R_0$ unsubstituiertes oder durch $C_1$-$C_{12}$-Alkyl substituiertes Phenylen, Naphthylen oder Diphenylen oder einen Rest -O-$R_v$-O- bedeutet, worin

$R_v$ unsubstituiertes oder durch $C_1$-$C_{12}$-Alkyl substituiertes Phenylen, Naphthylen oder Diphenylen oder $R_0$ einen Rest -Phen-$R_w$-Phen- bedeutet, worin Phen Phenylen bedeutet und

$R_w$ -O-, -S-, $-SO_2-$, $-CH_2-$, oder $-C(CH_3)_2-$ bedeutet.

Unter den Verbindungen der Formel A sind solche bevorzugt, worin $R_x$, $R_y$ und $R_z$ $C_6$-$C_{20}$-Alkyl, Phenyl oder durch ein bis drei, insbesondere ein bis zwei $C_1$-$C_{12}$-Alkylgruppen substituiertes Phenyl bedeuten.

Unter den Verbindungen der Formel B sind solche bevorzugt, worin $R_x$ und $R_y$ Phenyl oder durch ein bis zwei $C_1$-$C_{12}$-Alkylgruppen substituiertes Phenyl bedeuten und $R_0$ einen Diphenylen-Rest bedeutet.

Unter den Verbindungen der Formel C sind solche bevorzugt, worin $R_x$ und $R_y$ Phenyl oder durch ein bis drei $C_1$-$C_{12}$-Alkylgruppen substituiertes Phenyl bedeuten.

Bevorzugt verwendet man Phosphite und Phosphonite, die mindestens eine Gryppe P-O-Ar enthalten, wobei Ar einen Mono- oder Dialkylphenylrest bedeutet. Beispiele dafür sind:

Triphenylphosphit, Decyl-diphenylphosphit, Phenyl-didecylphosphit, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)pentaerythritdiphosphit, Tristearyl-sorbit-triphosphit, Tetrakis-(2,4-di-tert.butylphenyl)-4,4'-biphenylen-diphosphonit, Bis-(2,6-di-tert.butyl-4-methyl-phenyl)-pentaerythrit-diphosphit

Die Menge des Zusatzes an Phosph(on)it richtet sich nach der Menge an zugesetzter Verbindung der Formel I. Im allgemeinen verwendet man 0.01 bis 1 Gew.-%, insbesondere 0.05 bis 0.5 Gew.-%, bezogen auf das Polymere.

Die Erfindung betrifft ferner neue Isoindolinonverbindungen, die der Formel X

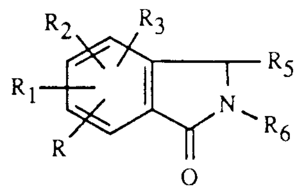

(X)

entsprechen,
worin R Wasserstoff, $C_1$-$C_{18}$-Alkyl, $C_1$-$C_{18}$-Alkoxy, Halogen, Nitro, Phenyl-$C_1$-$C_4$-alkoxy, $C_2$-$C_{18}$-Alkanoyl, Benzoyl, ($C_1$-$C_6$-Alkyl)-benzoyl, $C_2$-$C_{18}$-Alkenoyl, -N($R_7$)($R_{7a}$), -OH oder -CO-A ist,
$R_1$ die gleichen Bedeutungsmöglichkeiten wie R hat,
$R_2$ Wasserstoff oder $C_1$-$C_4$-Alkyl und
$R_3$ Wasserstoff oder Halogen bedeuten,
A Hydroxy, $C_1$-$C_{18}$-Alkoxy, $C_5$-$C_{12}$-Cycloalkoxy, Benzyloxy oder mit $C_1$-$C_4$-Alkyl und/oder Halogen substituiertes Benzyloxy, -(N($R_7$)($R_{7a}$), -NH-NH-$R_8$ oder $G_1$-$(G)_n$ ist,
n eine Zahl von 1 bis 3 und G einen Rest der Formel

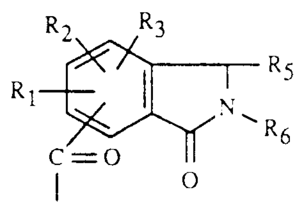

(II)

bedeuten,
$G_1$ im Fall n = 1 für -O-$R_{20}$-O-, -NH-$R_{21}$-NH- oder -NH-NH-,
im Fall n = 2 für eine Gruppe der Formel

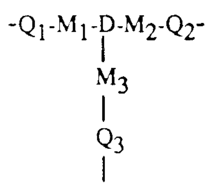

(III)

und im Fall n = 3 für $(\text{-O-CH}_2)_4$C oder

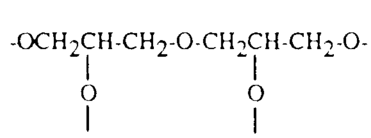

steht, wobei D N, CH oder C($C_1$-$C_4$-Alkyl) ist, $Q_1$, $Q_2$ und $Q_3$ unabhängig voneinander O oder NH und $M_1$, $M_2$, und $M_3$ unabhängig voneinander $C_1$-$C_6$-Alkylen oder durch -O-unterbrochenes $C_2$-$C_{12}$-Alkylen bedeuten, oder im Falle D = CH oder C($C_1$-$C_4$-Alkyl) -$M_3$-$Q_3$ auch für -O- oder im Falle D = N -$M_3$-$Q_3$ auch für eine direkte Bindung steht, $R_5$ und $R_6$ zusammen eine Gruppe der Formel IV, V oder VII bilden

(IV) 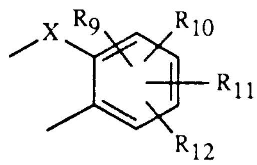 , 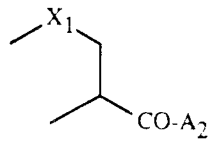 (V),

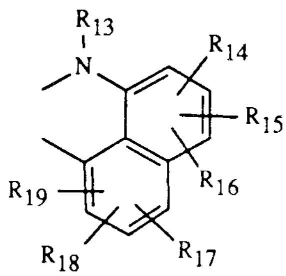 (VII)

X-S-,

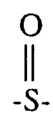

oder

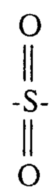 ,

$X_1$ -S-,

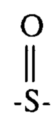

oder

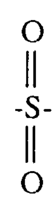

bedeuten
wobei, wenn X S oder S = O ist und einer der Substituenten $R_9$-$R_{12}$ H, Alkoxy oder Halogen bedeutet, die restlichen 3 dieser Substituenten nicht gleichzeitig für H stehen können,
$R_7$ und $R_{7a}$ unabhängig voneinander Wasserstoff, $C_1$-$C_{18}$-Alkyl, Phenyl-$C_1$-$C_4$-alkyl, $C_2$-$C_{18}$-Alkanoyl, $C_2$-$C_{18}$-Alkenoyl, $C_2$-$C_4$-Hydroxyalkyl, Benzoyl oder ($C_1$-$C_6$-Alkyl)-benzoyl, oder zusammen mit dem N-Atom,

an das sie gebunden sind, Piperidyl, Morpholinyl, Piperazinyl oder 4-Methylpiperazinyl bedeuten,
$R_8$ Wasserstoff, $C_2$-$C_{18}$-Alkanoyl, Benzoyl oder ($C_1$-$C_6$-Alkyl)-benzoyl ist,
$R_9$, $R_{10}$, $R_{11}$ und $R_{12}$ die gleichen Bedeutungsmöglichkeiten wie R, $R_1$, $R_2$ und $R_3$ (in dieser Reihenfolge) haben, wobei an Stelle von -CO-A -CO-$A_1$ zu setzen ist,
$R_{13}$ Wasserstoff, $C_1$-$C_{18}$-Alkyl, $C_2$-$C_{18}$-Alkenyl, $C_5$-$C_{12}$-Cycloalkyl, Phenyl-$C_1$-$C_4$-alkyl, $C_2$-$C_{18}$-Alkanoyl, $C_2$-$C_{18}$-Alkenoyl, Benzoyl, ($C_1$-$C_6$-Alkyl)-benzoyl, Benzyl, $C_2$-$C_{19}$-Alkoxycarbonyl-$C_1$-$C_4$-alkyl, Carboxy-$C_1$-$C_4$-alkyl, Cyano-$C_1$-$C_4$-alkyl, Hydroxy-$C_2$-$C_4$-alkyl oder

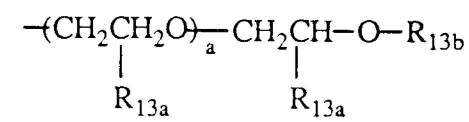

bedeutet,
$R_{13a}$ für Wasserstoff oder Methyl und $R_{13b}$ für Wasserstoff, $C_1$-$C_{18}$-Alkyl, Phenyl-$C_1$-$C_4$-alkyl, $C_2$-$C_{18}$-Alkanoyl, Benzoyl, $C_2$-$C_{18}$-Alkenoyl oder ($C_1$-$C_6$-Alkyl)-benzoyl und a für eine Zahl von 0 bis 6 stehen,
$R_{14}$, $R_{15}$, $R_{16}$, $R_{17}$, $R_{18}$ und $R_{19}$ unabhängig voneinander für Wasserstoff, $C_1$-$C_4$-Alkyl, Halogen, Hydroxy, $C_1$-$C_{18}$-Alkoxy oder -N($R_{7b}$)($R_{7c}$) stehen, worin $R_{7b}$ und $R_{7c}$ unabhängig voneinander Wasserstoff oder $C_1$-$C_{18}$-Alkyl bedeuten,
$R_{20}$ $C_1$-$C_{12}$-Alkylen, $C_2$-$C_8$-Alkenylen, $C_3$-$C_6$-Alkinylen oder durch O, S oder/und -N($R_{22}$)- unterbrochenes $C_2$-$C_{24}$-Alkylen,
$R_{21}$ $C_1$-$C_{12}$-Alkylen und $R_{22}$ Wasserstoff, $C_1$-$C_{18}$-Alkyl, Phenyl, ($C_1$-$C_6$-Alkyl)-phenyl oder Phenyl-$C_1$-$C_4$-alkyl bedeuten, $A_1$ Hydroxy, $C_1$-$C_{24}$-Alkoxy, $C_5$-$C_{12}$-Cycloalkoxy, Benzyloxy oder mit $C_1$-$C_4$-Alkyl und/oder Halogen substituiertes Benzyloxy, -N($R_7$)($R_{7a}$),-NH-NH-$R_8$ oder -$G_1$-$(G_2)_n$ ist, und $A_2$ Hydroxy, $C_1$-$C_{24}$-Alkoxy, $C_5$-$C_{12}$-Cycloalkoxy, Benzyloxy oder mit $C_1$-$C_4$-Alkyl und/oder Halogen substituiertes Benzyloxy, -N($R_7$)($R_{7a}$),-NH-NH-$R_8$ oder -$G_1$-$(G_3)_n$ ist, wobei $G_1$ und n wie oben definiert sind,
$G_2$ für einen Rest der Formel

(VIII) 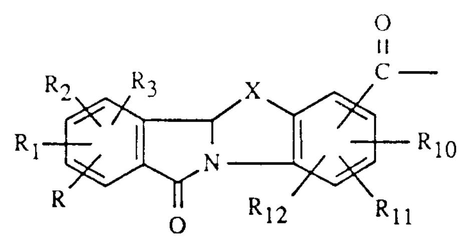

und
$G_3$ für einen Rest der Formel

(IX) 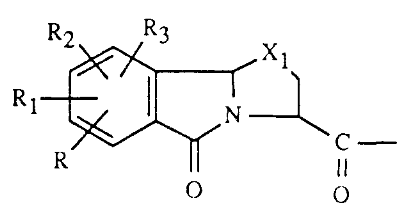

steht,
mit den Maßgaben, (1) daß im Molekül nur ein Substituent -CO-A, CO-$A_1$ bzw. -CO-$A_2$ vorhanden ist, in dem A, $A_1$ bzw. $A_2$ einen Rest -$G_1$(G)$_n$, -$G_1$-$(G_2)_n$ bzw. -$G_1$-$(G_3)_n$ darstellt und (2) daß im Fall X = S höchstens einer der Substituenten R und $R_1$ für $C_1$-$C_{18}$-Alkoxy steht.

Zweckmäßig sind Verbindungen der Formel X, worin R Wasserstoff, Hydroxy, $C_1$-$C_4$-Alkyl, Chlor, Nitro, $C_1$-$C_4$-Alkoxy, Benzoyl, -N($R_7$)($R_{7a}$) oder -CO-A ist,
$R_1$ die gleichen Bedeutungsmöglichkeiten wie R hat,
$R_2$ Wasserstoff, Chlor oder $C_1$-$C_4$-Alkyl und

$R_3$ Wasserstoff oder Chlor bedeuten,
A Hydroxyl, $C_1$-$C_{18}$-Alkoxy, $C_5$-$C_6$-Cycloalkoxy, Benzyloxy oder mit $C_1$-$C_4$-Alkyl und/oder Chlor substituiertes Benzyloxy, -N($R_7$)($R_{7a}$) oder -$G_1$-$(G)_n$ ist, n eine Zahl von 1 bis 3 und G einen Rest der Formel

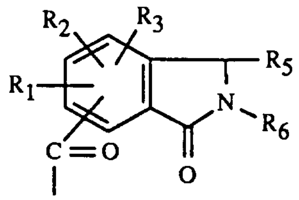

(II)

bedeuten,
$G_1$ im Fall n = 1 für -O-$R_{20}$-O- oder -NH-$R_{21}$-NH-,
im Fall n = 2 für eine Gruppe der Formel

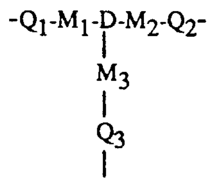

(III)

und im Fall n = 3 für $(-OCH_2)_4C$ steht, wobei D gleich N, CH oder C($C_1$-$C_4$-Alkyl) ist,
$Q_1$, $Q_2$ und $Q_3$ unabhängig voneinander O oder NH und
$M_1$, $M_2$ und $M_3$ unabhängig voneinander $C_1$-$C_6$-Alkylen oder durch -O- unterbrochenes $C_2$-$C_{12}$-Alkylen bedeuten,
oder im Falle D = CH oder C($C_1$-$C_4$-Alkyl) -$M_3$-$Q_3$ auch für -O- oder im Falle D = N -$M_3$-$Q_3$ auch für eine direkte Bindung steht,
$R_5$ und $R_6$ zusammen eine Gruppe der Formel IV, V oder VII bilden,
$R_7$ und $R_{7a}$ unabhängig voneinander Wasserstoff, $C_1$-$C_6$-Alkyl, Benzyl, $C_2$-$C_4$-Hydroxyalkyl, oder zusammen mit dem N-Atom, an das sie gebunden sind, Piperidyl, Morpholinyl, Piperazinyl oder 4-Methylpiperazinyl bedeuten,
$R_9$, $R_{10}$, $R_{11}$ und $R_{12}$ die gleichen Bedeutungsmöglichkeiten wie R, $R_1$, $R_2$ und $R_3$ (in dieser Reihenfolge) haben, wobei an Stelle von -CO-A -CO-$A_1$ zu setzen ist,
$R_{13}$ Wasserstoff, $C_1$-$C_{18}$-Alkyl, $C_2$-$C_{18}$-Alkenyl, $C_5$-$C_6$-Cycloalkyl, Phenyl-$C_1$-$C_4$-alkyl, $C_2$-$C_{18}$-Alkanoyl, $C_2$-$C_{18}$-Alkenoyl, Benzoyl, ($C_1$-$C_6$-Alkyl)-benzoyl, Cyano-$C_1$-$C_4$-alkyl $C_2$-$C_{19}$-Alkoxycarbonyl-$C_1$-$C_4$-alkyl, Carboxy-$C_1$-$C_4$-alkyl oder Hydroxy-$C_2$-$C_4$-alkyl bedeutet,
$R_{14}$, $R_{15}$, $R_{16}$, $R_{17}$, $R_{18}$ und $R_{19}$ unabhängig voneinander für Wasserstoff, $C_1$-$C_4$-Alkyl, Chlor, Hydroxy, $C_1$-$C_4$-Alkoxy oder -N($R_{7b}$)($R_{7c}$) stehen, worin $R_{7b}$ und $R_{7c}$ unabhängig voneinander Wasserstoff oder $C_1$-$C_6$-Alkyl bedeuten,
$R_{20}$ $C_1$-$C_{12}$-Alkylen, $C_2$-$C_8$-Alkenylen, $C_3$-$C_6$-Alkinylen oder durch O, S oder/und -N($R_{22}$) unterbrochenes $C_2$-$C_{24}$-Alkylen,
$R_{21}$ $C_1$-$C_{12}$-Alkylen und $R_{22}$ Wasserstoff oder $C_1$-$C_4$-Alkyl bedeuten, $A_1$ Hydroxy, $C_1$-$C_{18}$-Alkoxy, $C_5$-$C_6$-Cycloalkoxy, Benzyloxy oder mit $C_1$-$C_4$-Alkyl und/oder Halogen substituiertes Benzyloxy, -N($R_7$)($R_{7a}$) oder -$G_1$-$(G_2)_n$ ist und $A_2$ Hydroxy, $C_1$-$C_{18}$-Alkoxy, $C_5$-$C_6$-Cycloalkoxy, Benzyloxy oder mit $C_1$-$C_4$-Alkyl und/oder Halogen substituiertes Benzyloxy, -N($R_7$)($R_{7a}$) oder -$G_1$-$(G_3)_n$ ist, wobei $G_1$ und n wie oben definiert sind,
$G_2$ für einen Rest der Formel

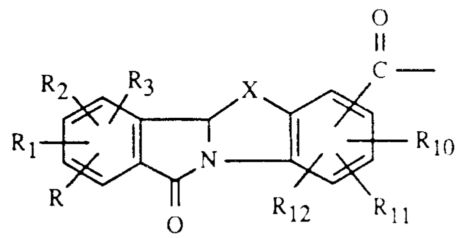

und

$G_3$ für einen Rest der Formel

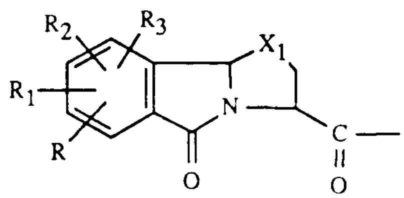

steht.

Bevorzugt sind Verbindungen der Formel X, worin R Wasserstoff, Chlor, Hydroxy oder $C_1$-$C_4$-Alkyl ist, $R_1$ Wasserstoff, Chlor, $C_1$-$C_4$-Alkyl oder Hydroxy, $R_2$ und $R_3$ Wasserstoff sind, $R_5$ und $R_6$ zusammen eine Gruppe der Formel IV, V oder VII bilden, $R_9$, $R_{10}$, $R_{11}$ und $R_{12}$ die gleichen Bedeutungsmöglichkeiten wie R, $R_1$, $R_2$ und $R_3$ (in dieser Reihenfolge) haben, $R_{13}$ Wasserstoff, $C_1$-$C_{18}$-Alkyl, $C_5$-$C_6$-Cycloalkyl, Benzyl, $C_2$-$C_{18}$-Alkanoyl, $C_2$-$C_{19}$-Alkoxycarbonyl-$C_1$-$C_4$-alkyl oder Benzoyl bedeutet, $R_{14}$, $R_{15}$, $R_{16}$, $R_{17}$, $R_{18}$ und $R_{19}$ Wasserstoff sind, und $A_2$ Hydroxy, $C_1$-$C_{18}$-Alkoxy, $C_5$-$C_6$-Cycloalkoxy oder Benzyloxy bedeutet, insbesondere solche, worin R Wasserstoff, Methyl oder Chlor bedeutet, $R_1$, $R_2$ und $R_3$ Wasserstoff sind, und $R_5$ und $R_6$ zusammen eine Gruppe der Formeln IV, V oder VII darstellen, worin X -S-,

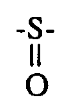

oder

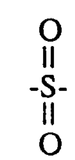

,

$X_1$ -S-, $R_9$ Wasserstoff, $-CH_3$, Cl oder -COO-($C_1$-$C_{24}$-Alkyl), $R_{10}$, $R_{11}$ und $R_{12}$ Wasserstoff, $R_{13}$ Wasserstoff, $C_1$-$C_{18}$-Alkyl, Benzoyl $C_2$-$C_{19}$-Alkoxycarbonyl-methyl oder $C_2$-$C_{18}$-Alkanoyl, $R_{14}$ bis $R_{19}$ Wasserstoff und $A_2$ $C_1$-$C_{24}$-Alkoxy bedeuten.

Besonders bevorzugt sind Verbindungen der Formel X, worin R und $R_9$ unabhängig voneinander Wasserstoff, $C_1$-$C_4$-Alkyl oder Chlor und $R_1$, $R_2$, $R_3$, $R_4$, $R_{10}$, $R_{11}$, $R_{12}$, $R_{14}$, $R_{15}$, $R_{16}$, $R_{17}$, $R_{18}$ und $R_{19}$ Wasserstoff bedeuten.

Ein weiterer Gegenstand der Erfindung ist die Verwendung von Verbindungen nach den Formeln I und X zum Stabilisieren organischen Materials gegen oxidativen, thermischen und/oder actinischen Abbau.

Bevorzugt ist die Verwendung von Verbindungen gemäß. den Formeln I und X als Stabilisatoren in Schmierstoffen, Metallbearbeitungs- und Hydraulikflüssigkeiten sowie in synthetischen, natürlichen oder halbsynthetischen Polymeren.

Die Erfindung besteht demzufolge auch in einem Verfahren zum Stabilisieren organischen Materials, insbesondere von Schmierstoffen, Metallbearbeitungs- und Hydraulikflüssigkeiten sowie natürlichen, synthetischen oder halbsynthetischen Polymeren, dadurch gekennzeichnet, däß man diesem Material als Stabilisatoren Verbindungen der Formeln I bzw. X zusetzt bzw. auf diese aufbringt.

Die Herstellung der erfindungsgemäßen Verbindungen sowie der in den erfindungsgemäßen Verbindungen eingesetzten Isoindolinonverbindungen erfolgt beispielsweise nach den in C.H. Gaozza et al., J. Heterocycl Chem 9, 883 (1972) und US-A 3,591,599 beschriebenen Verfahren und kann durch das folgende Schema zusammengefäßt werden:

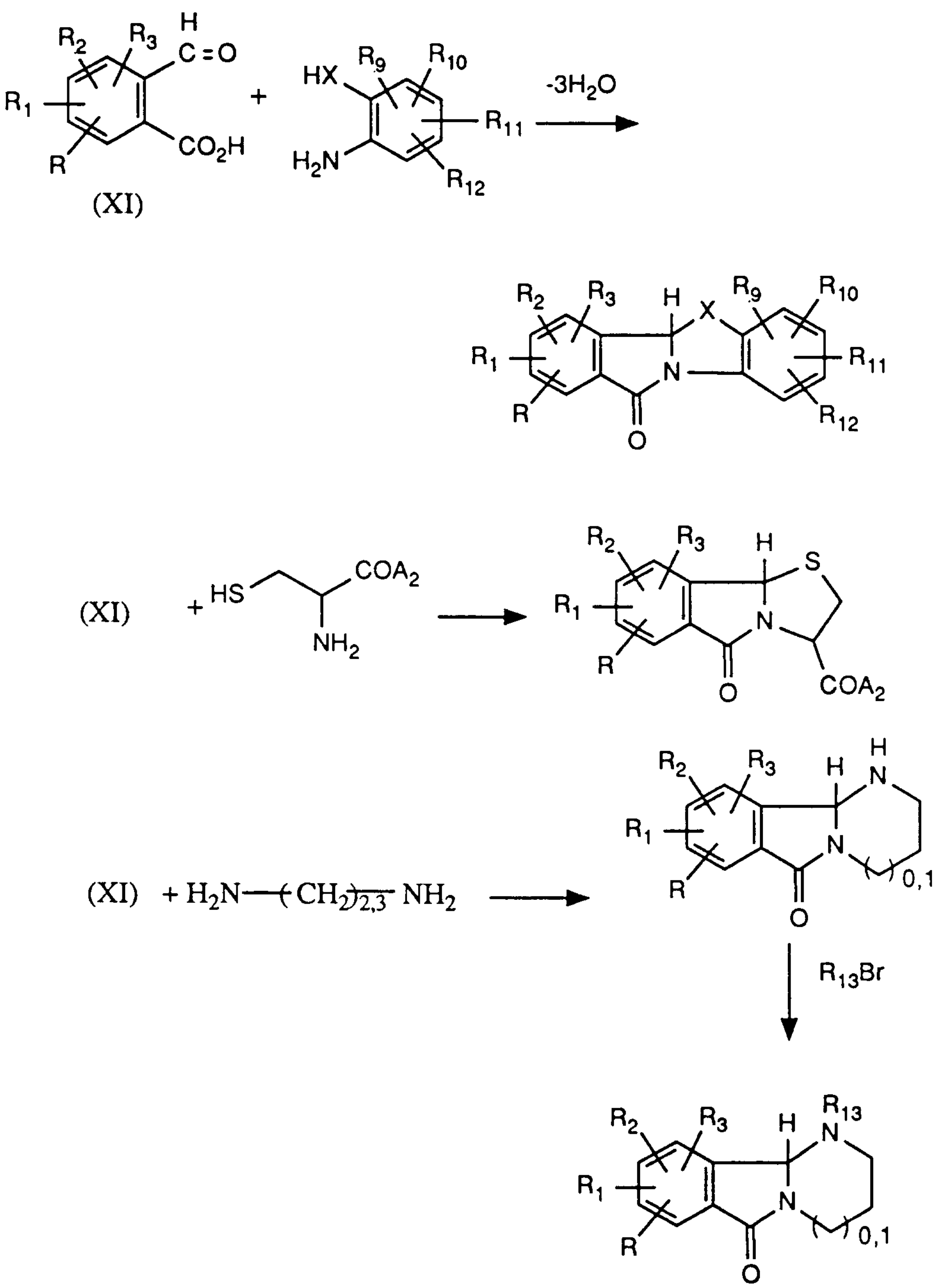

(XI) +

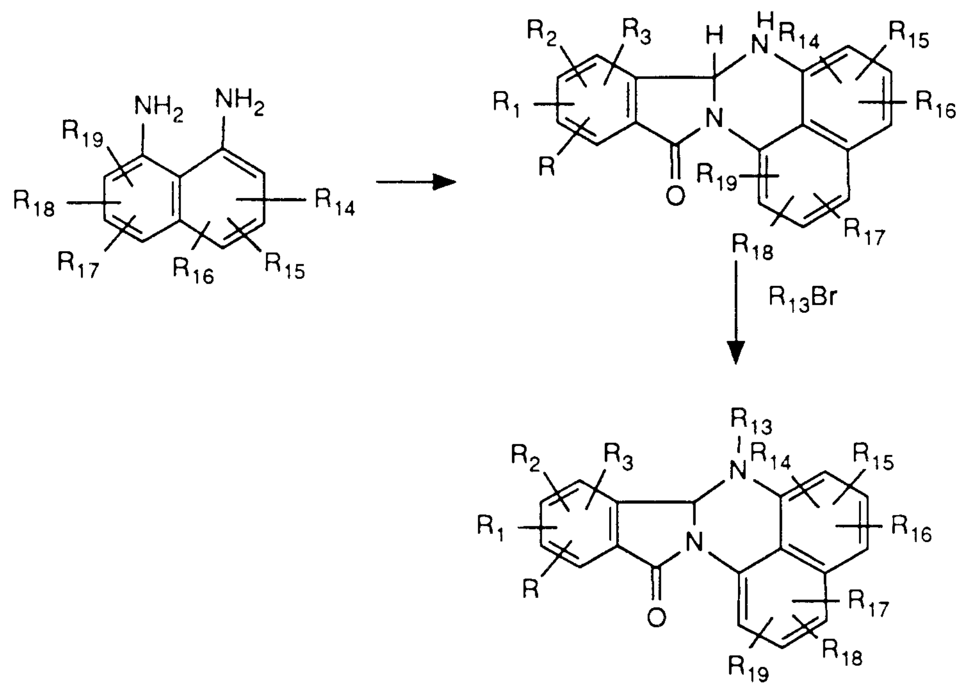


Die Umsetzungen erfolgen zweckmäßig unter Zusatz von üblichen, unter den Reaktionsbedingungen inerten Lösungsmitteln, vorzugsweise Ethanol. Die schwefelhaltigen Produkte können mit Oxidationsmitteln zu den entsprecheneden Sulfoxid- und Sulfon-Verbindungen oxidiert werden. Bevorzugt ist z.B. m-Chlorperbenzoesäure als Oxidationsmittel.

Selbstverständlich können allfällige Substituenten nach erfolgtem Ringaufbau nach üblichen, in der organischen Chemie bekannten Verfahren ineinander umgewandelt bzw. eingeführt werden.

Zur Herstellung von Verbindungen der Formel I, in denen in Resten -COA, -COA$_1$ bzw. -COA$_2$ A, A$_1$ bzw. A$_2$ -G$_1$-(G)$_n$, -G$_1$-(G$_2$)$_n$ bzw. -G$_1$-(G$_3$)$_n$ bedeuten, geht man z.B. von den entsprechenden Säuren (A, A$_1$, A$_2$ = OH) aus und führt die Umsetzung (Oligomerisierung) aus daraus gebildeten Estern oder Amiden durch.

Wird über Ester oligomerisiert, so enfolgt dies z.B. durch Umwandlung der freien Säure (A, A$_1$, A$_2$ = OH) in das entsprechende Säurechlorid (z.B. mit $PCl_5$, $SOCl_2$) und Reaktion mit dem dem Rest G$_1$ entsprechenden zwei- , drei- oder vier-wertigen Alkohol, vorzugsweise unter Basenkatalyse. Die Säure kann mit dem entsprechenden höherwertigen Alkohol auch direkt in der Wärme, oder aber schonend durch Behandlung mit Dicyclohexylcarbodiimid (DCC) in Gegenwart des höherwertigen Alkohols umgesetzt werden (Tetrahedron Letters 1978, 4475-8), bzw. durch weitere schonende, z.B. in Tetrahedron 36, 2409 (1980) beschriebene Verfahren. Eine weitere Möglichkeit zum Erhalt der über Estergruppen oligomerisierten Verbindungen besteht in der Umesterung ausgehend von den niederen Monoestern mit Diol, Triol, Tetraol. Dafür geeignete Katalysatoren sind z.B. starke Säuren wie $H_2SO_4$, HCl, p-Toluolsulfonsäure, Methansulfonsäure, saure Ionenaustauscher, oder auch Basen, wie z.B. $LiNH_2$, $CH_3ONa$ oder aber Dialkylzinnoxide oder Titantetraalkoxylate. Der niedere Alkohol wird dabei durch Destillation aus dem Reaktionsgemisch entfernt. Die Katalysatormenge beträgt in der Regel 0.1-5 Mol%, bezogen auf den Monoester.

Wird über Amide oligomerisiert, so werden wie oben beschrieben die Säurechloride gebildet, die dann mit dem einem Rest G$_1$ entsprechenden Di- oder Triamin umgesetzt werden. Bei empfindlichen Verbindungen eignet sich beispielsweise ebenfalls die Methode mit DCC, neben den anderen, in Tetrahedron 36, 2409 (1980) beschriebenen Verfahren. Des weiteren können auch niedere Ester (A, A$_1$, A$_2$ = Methyl, Ethyl, Isopropyl, Propyl etc.) direkt in der Wärme mit den genannten Aminen zu entsprechenden Amiden umgesetzt werden.

Die Reaktionen können mit oder ohne Zusatz von üblichen, inerten Lösungsmitteln durchgeführt werden. Einige der beschriebenen Herstellungsverfahren sind in den nachfolgenden Beispielen ausführlich erläutert.

Die für die oben beschriebenen Herstellungsverfahren benötigten Ausgangsverbindungen sind bekannt oder können in an sich bekannter Weise nach üblichen Methoden hergestellt werden.

Die nachfolgenden Beispiele erläutern die Erfindung weiter. Soweit nichts anderes angegeben ist, bedeuten darin sowie in der übrigen Beschreibung Teile- und Prozentangaben Gewichtsteile und Gewichtsprozent.

Beispiel 1: (Verbindung 1, Tabelle 1)

12.5 g 2-Aminothiophenol und 15.0 g Phthalaldehydsäure werden in 100 ml Ethanol unter Stickstoff während 12 Std. gekocht. Danach wird das Reaktionsgemisch abgekühlt, der ausgeschiedene Feststoff abgesaugt und aus Ethanol-Methylenchlorid (1:1) umkristallisiert. Man erhält so 18.2 g farblose Kristalle vom Schmelzbereich 173-174 °C, die der Struktur von Verbindung 1 entsprechen.

Beispiel 2: (Verbindung 2, Tabelle 1)

6.25 g 3-Amino-4-mercaptobenzoesäure und 5.54 g Phthalaldehydsäure werden in 40 ml Ethanol und 10 ml Wasser unter Stickstoff während 17 Std. gekocht. Danach wird das Reaktionsgemisch abgekühlt und der augeschiedene Feststoff abgesaugt und getrocknet. Man erhält so 6.9 g weißer Kristalle vom Schmelzpunkt 256-260 °C. 4.8 g dieser Verbindung werden in 50 ml Methylenchlorid mit 4.58 g Stearylalkohol, 3.5 g Dicyclohexylcarbodiimid und 0.1 g 4-Dimethylaminopyridin bei Raumtemperatur 20 Std. gerührt. Das Reaktionsgemisch wird anschließend filtriert, das Filtrat am Rotationsverdampfer eingedampft und der Rückstand aus Acetonitril umkristallisiert. Man erhält so 6.7 g farbloser Kristalle vom Schmelzbereich 93-95 °C, die der Struktur von Verbindung 2 entsprechen.

Beispiel 3: (Verbindung 3, Tabelle 1)

1.86 g L-Cystein-ethylester-hydrochlorid und 1.50 g Phthalaldehydsäure werden in 20 ml Ethanol unter Stickstoff während 4 Std. gekocht. Das Reaktionsgemisch wird danach am Rotationsverdampfer eingedampft, der Rückstand mit 2.70g Stearylalkohol und 0.05 g p-Toluolsulfonsäure versetzt und anschließend 90 Min. bei 200 °C im schwachen Vakuum (20 k Pa) gerührt. Nach Abkühlen, Säulenchromatographie an Kieselgel (Methylenchlorid:Hexan = 19:1) und Umkristallisation aus Acetonitril werden 3 g der Verbindung 3 mit einem Schmelzpunkt von 51-3 °C erhalten.

Beispiel 4: (Verbindung 4, Tabelle 1)

12.0 g der Verbindung 1 in 120 ml Methylenchlorid werden auf -30 °C abgekühlt und bei dieser Temperatur mit 10.2 g m-Chlorperbenzoesäure (85 %) versetzt. Die Temperatur des Reaktionsgemisches wird danach im Laufe von 3 Std. auf +20 °C erhöht. Das Gemisch wird anschließend mit 1-N-KOH und dann mit Wasser gewaschen und am Rotationsverdampfer eingedampft. Aus dem Rückstand erhält man durch Chromatographie an Kieselgel (Methylenchlorid:Ethylacetat = 19:1) 8.35 g der Verbindung 4 mit einem Schmelzpunktbereich von 178-82 °C.

Beispiel 5: (Verbindung 5, Tabelle 1)

9.59 g der Verbindung 1 werden bei 20 °C während 20 Std. mit 24.0 g m-Chlorperbenzoesäure (85 %) wie in Beispiel 4 beschrieben oxidiert. Durch Kristallisation aus Toluol werden 9.4 g der Verbindung 5 erhalten, die zwischen 214-216 °C schmilzt.

Beispiel 6: (Verbindung 6, Tabelle 1)

7.41 g Diaminopropan und 15.01 g Phthalaldehydsäure werden in 100 ml Ethanol gelöst und das Gemisch wird unter Stickstoff während 3 Std. gekocht. Danach wird das Lösungsmittel abdestilliert und der Rückstand aus Toluol-Hexan (1:1) umkristallisiert. Man erhält so 16.41 g der Verbindung 6, die zwischen 128-130 °C schmilzt.

Beispiel 7: (Verbindung 7, Tabelle 1)

9.4 g der Verbindung 6,15.56 g Cetylbromid und 0.1 g Kaliumjodid werden in 50 ml Dimethylformamid unter Stickstoff während 15 Std. gekocht. Danach wird das Reaktionsgemisch abgekühlt, mit 50 ml Methylenchlorid verdünnt und 3x mit 500 ml Wasser gewaschen. Aus dem Rückstand nach Abdampfen des

Methylenchlorids werden durch Chromatographie an Kieselgel (Methylenchlorid-Ethylacetat 1:1) und anschließende Kristallisation aus Hexan 5.7 g der Verbindung 7 vom Schmelzbereich 50-51 ° C erhalten.

Beispiel 8: (Verbindung 8, Tabelle 1)

6.63 g der Verbindung 6 werden in 20 ml Pyridin gelöst und danach mit 4.92 g Benzoylchlorid und 0.5 g 4-Dimethylaminopyridin versetzt. Das Reaktionsgemisch wird 3 Std. bei Raumtemperatur gerührt, anschließend wird es mit 100 ml Methylenchlorid verdünnt, mit Wasser, verdünnter Salzsäure und erneut mit Wasser gewaschen. Der Rückstand nach Abdampfen des Lösungsmittels wird aus Methanol kristallisiert. Man erhält so 7.4 g der Verbindung 8 vom Schmelzpunkt 143-5 ° C.

Beispiele 9 und 10: (Verbindungen 9 und 10, Tabelle 1)

15.82 g 1,8-Diaminonaphthalin und 15.02 g Phthalaldehydsäure werden in 150 ml Ethanol gelöst und die Lösung wird 1 Std. unter Stickstoff gekocht. Das Reaktionsgemisch wird danach abgekühlt und der ausgefallene Feststoff abgesaugt, mit Ethanol gewaschen und getrocknet. Man erhält so 24.7 g des Zwischenproduktes vom Schmelzbereich 230-240 ° C (Verbindung 9).

10.0 g des obigen Zwischenproduktes werden in 30 ml Dimethylformamid gelöst und die Lösung wird danach mit 12.33 g Cetylbromid und 1.0 g Kaliumjodid versetzt und während 24 Std. unter Stickstoff gekocht. Das Reaktionsgemisch wird danach mit 100 ml Methylenchlorid verdünnt und mit Wasser gewaschen. Aus dem Rückstand nach Abdampfen des Lösungsmittels werden durch Chromatographie an Kieselgel (Methylenchlorid) und Kristallisation aus Ethanol 12.8 g der Verbindung 10 erhalten, die zwischen 54-56 ° C schmilzt.

Beispiel 11: (Verbindung 11, Tabelle 1)

28.2 g der Verbindung 6,45 ml Bromessigsäuremethylester, 1 g Natriumiodid und 20.8 g Kaliumcarbonat werden langsam zum Rückfluß erwärmt und 15 min gekocht. Das Reaktionsgemisch wird mit 100 ml Toluol verdünnt und filtriert. Nach Eindampfen des Filtrats im Vakuum (150 mbar) chromatographiert man den Rückstand an Kieselgel (Ethylacetat/Isopropanol 3:1) und kristallisiert aus Toluol/Hexan (1:1) um. Man erhält 21.3 g der Verbindung 11. Schmelzpunkt 70-73 ° C.

Beispiel 12: (Verbindung 12, Tabelle 1)

7.8 g der Verbindung 11, 5.65 g n-Dodecanol und 0.13 g Dibutylzinnoxid werden im schwachen Vakuum (0.15 bar) während 4 h auf 150 ° C erwärmt. Nach Abkühlen und Chromatographie an Kieselgel (Dichlormethan/Ethylacetat 4:1) und Umkristallisieren aus Acetonitril erhält man 8.7 g der Verbindung 12. Schmelzpunkt 44-45 ° C.

Tabelle 1

| Nr. | Verbindung | Smp. °C | *C* *H* *N* *S* [berechnet/gefunden] % |
|---|---|---|---|
| 1 | 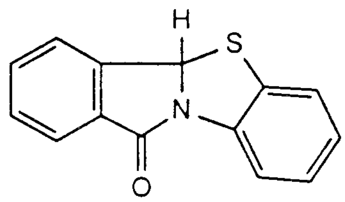 | 173-4 | 70,27 3,79 5,85 13,40<br>70,31 3,82 5,90 13,42 |
| 2 | 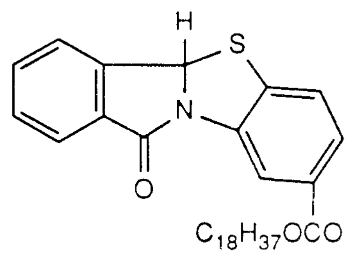 | 93-5 | 73,98 8,47 2,61 5,98<br>73,49 8,53 2,53 6,09 |
| 3 | 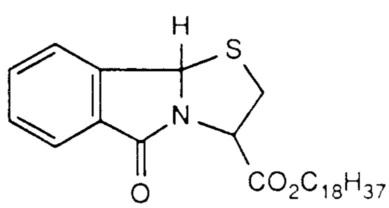 | 51-5 | 71,41 9,30 2,87 6,57<br>72,12 9,19 2,79 6,48 |
| 4 | 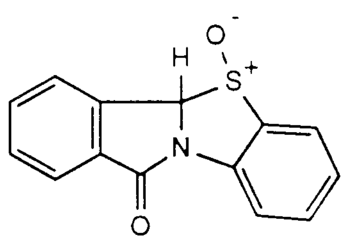 | 178-82 | 65,87 3,55 5,49 12,56<br>65,85 3,68 5,21 12,84 |
| 5 | 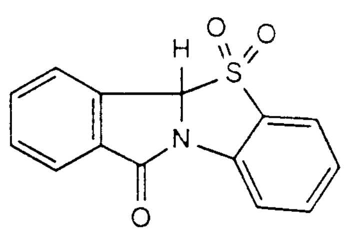 | 214-16 | 61,98 3,34 5,16 11,82<br>62,01 3,32 4,91 11,80 |

Tabelle 1 (Fortsetzung)

| Nr. | Verbindung | Smp. °C | C H N S [berechnet/gefunden] % |
|---|---|---|---|
| 6 | 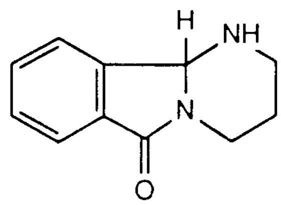 | 128-130 | 70,19 6,43 14,88 --<br>70,18 6,55 15,02 -- |
| 7 | 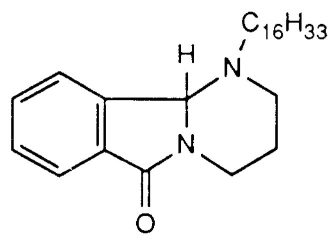 | 50-1 | 78,59 10,75 6,79 --<br>78,51 10,73 6,72 -- |
| 8 | 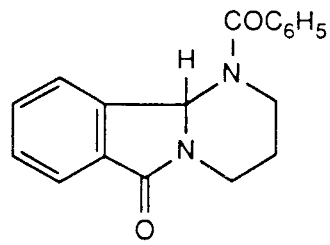 | 143-5 | 73,96 5,52 9,59 --<br>73,74 5,52 9,70 -- |
| 9 | 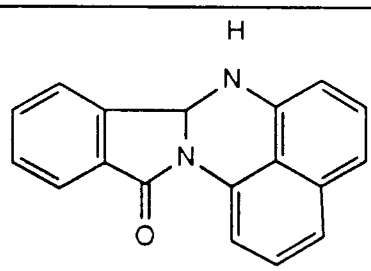 | 230-240 | |
| 10 | 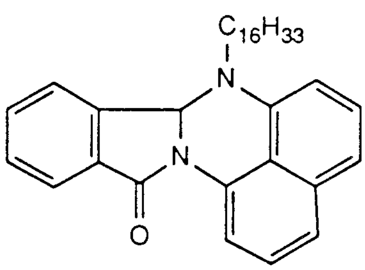 | 54-6 | 82,21 8,93 5,64 --<br>82,16 9,01 5,63 -- |

Tabelle 1 (Fortsetzung)

| Nr. | Verbindung | Smp. °C | C H N S [berechnet/gefunden] % |
|---|---|---|---|
| 11 | 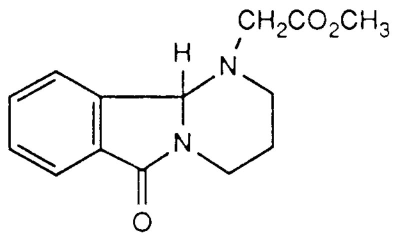 | 70-73 | 64.60 6.20 10.76 --<br>64.65 6.20 10.92 -- |
| 12 | 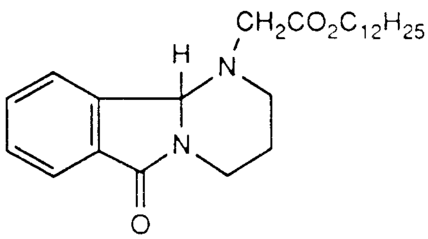 | 44-45 | 72.60 9.02 6.77 --<br>72.42 9.29 6.63 -- |

Beispiel 13: Test auf Stabilisierung eines industriellen Öles gegen oxidativen Abbau (TFOUT: Thin Film Oxygen Uptake Test)

Dieser Test ist eine modifizierte Form des Rotan Bomb Test für Mineralöle (ASTM D 272). Eine detaillierte Beschreibung findet sich bei C.S.Ku, S.M.Hsu, Lubrication Engineering 40 (1984) 75-83. Das Testöl ist in diesem Fall ein kommerzielles Motorenöl 15W40, mit ca. der Hälfte des üblichen Gehaltes an Zinkdithiophosphaten (0.75 % ZnDTP, 550 ppm P, 1160 ppm Zn). Das zu testende Additiv (Verbindung der Formel I) wird im Öl in Gegenwart von Wasser (2 %), einer oxidierten/nitrierten Benzinfraktion (4 %) und eines Gemisches von flüssigen Metallnaphthenaten (4 %) bei 6.1 bar Sauerstoffdruck und 160 °C auf seine stabilisierende Wirkung getestet. Das Wasser und die beiden flüssigen Katalysatoren für den Test werden unter der Bezeichnung Standard Reference Material 1817 vom National Bureau of Standards (NBS) bezogen, mit Bescheinigung für die Analyse. Der Test ist abgeschlossen, wenn im Druck/Zeit-Diagramm ein deutlicher Knick die einsetzende Oxidation am Ende der Induktionsperiode (min.) anzeigt.
Eine lange Induktionsperiode bedeutet eine gute stabilisierende Wirkung des Additives. Die Resultate sind in der Tabelle 2 zusammengefaßt.

Tabelle 2

| Verbindung aus Beispiel | Zusatzmenge (Gew. %) | Induktionsperiode (min.) |
|---|---|---|
| 9 | 0,5 | 153 |
| Referenz | ohne Additiv | 83 |

Beispiel 14: Test auf Stabilisierung des Schmelzindex von Polypropylen bei Mehrfachextrusion

1.3 kg Polypropylenpulver (Schmelzindex 3.2 g/10 min. gemessen bei 230 °C mit 2.16 kg) werden gemischt mit 0.05 % Calciumstearat und 0.05 % Irganox 1010 und 0.05 % Verarbeitungsstabilisator. Diese Mischung wird in einem Extruder mit einem Zylinderdurchmesser von 20 mm und einer Länge von 20 d = 400 mm mit 100 Umdrehungen pro Min. extrudiert, wobei die 3 Heizzonen auf die folgenden Temperaturen eingestellt werden: 260 °C, 270 °C, 280 °C. Das Extrudat wird zur Kühlung durch ein Wasserbad gezogen und anschließend granuliert. Dieses Granulat wird ein zweites und ein drittes Mal extrudiert. Nach diesen 3

Extrusionen wird der Schmelzindex gemessen (bei 230 °C mit 2.16 kg). Große Zunahme des Schmelzindex bedeutet starken Kettenabbau, also schlechte Stabilisierung. Die Resultate sind in der Tabelle 3 zusammengefäßt.

Tabelle 3

| Verbindung aus Beispiel | Schmelzindex |
|---|---|
| 1 | 4,9 |
| 2 | 5,5 |
| 3 | 9,3 |
| 5 | 6,7 |
| 6 | 11,1 |
| 7 | 8,7 |
| ohne Additiv | 16,5 |

Beispiel 15: Test auf Erhaltung der Schlagzähigkeit von ABS (Acrylnitril-Butadien-Styrol-Copolymer)

ABS (Terluran® 996S, BASF) mit einer Basisstabilisierung bestehend aus 0.6 % 4,4'-Thiobis(2-t-butyl-5-methylphenol), 0.1 % 1,1,3-Tris(2'-methyl-4'-hydroxy-5'-t-butylphenyl)butan und 0.2 % Dilaurylthiodipropionat wird mit 0.15 % der Verbindung 1 (Tabelle 1) und 0.3 % Tris(2,4-di-t-butylphenyl)phosphit (= Verbindung A) vermischt. Diese Mischung wird in einem Plamver-Extruder mit 100 Umdrehungen pro Min. extrudiert, wobei die 3 Heizzonen auf die folgenden Temperaturen eingestellt werden: 260 °C, 270 °C, 280 °C.
Aus dem 1., 3. und 5. Extrudat werden mit einer Aarburg-Spritzgußmaschine (Heizzonentemperaturen: 230 °C/240 °C/240 °C, Formtemperatur: 60 °C, Zyklusdauer: 35 Sekunden) Probekörper angefertigt. Diese werden gemäß DIN 53753 auf die Erhaltung der Schlagzähigkeit mit folgendem Resultat geprüft:

Tabelle 4

| Stabilisator | Kerbschlagzähigkeit nach Charpy (DIN 53753) (KJ/m$^2$) | | |
|---|---|---|---|
| | 1x | 3x | 5x |
| -- | 49±6 | 36±5 | 29±5 |
| 0.15% Verbindung 1 + 0.3% Verbindung A | 57±5 | 56±5 | 51±7 |

Die Ergebnisse zeigen, daß die Kerbschlagzähigkeit durch die Zugabe der beiden Additive deutlich besser erhalten wird.

Beispiel 16: Test auf Stabilisierung von Butadien (BR) und Butadienstyrolcopolymer (SBS) während der Brabenderverarbeitung

Polybutadien [BR BUNA CB HX 529C, enthaltend als Basisstabilisierung 0.3% 2,6-Di-tert-butyl-4-methylphenol (BHT)] beziehungsweise Butadienstyrolcopolymer [SBS Finapren 416, enthaltend als Basisstabilisierung BHT und Trinonylphosphit (TNPP)] werden in der Mischkammer eines Brabender Plastographen mit dem zu testenden Verarbeitungsstabilisator vermischt. Die Mischungen werden 60 Minuten lang bei der in Tabelle 5 angegebenen Temperatur und 60 UpM geknetet. Während dieser Zeit wird der Knetwiderstand als Drehmoment kontinuierlich registriert. Infolge der Vernetzung des Polymeren erfolgt im Laufe der Knetzeit nach anfänglicher Konstanz eine rasche Zunahme des Drehmoments. Die Wirksamkeit der Stabilisatoren äußert sich in einer Verlängerung der Konstantzeit. Die erhaltenen Werte sind Tabelle 5 zu entnehmen.

Tabelle 5

| Verbindung aus Tabelle 1 | Zusatzmenge % | Butadien 160 °C Induktionszeit [min] | SBS 200 °C Induktionszeit [min] |
|---|---|---|---|
| Referenz | ohne Additiv | 9 | 7.5 |
| **1** | 0.25 | 23.5 | 40 |
| **1** + **A*** (Gemisch 1Teil **1** 2 Teile **A**) | 0.25 | 20 | 33 |
| **7** | 0.40 | -- | 30.5 |

*) A: Tri(2,4-di-tert-butylphenyl)phosphit

## Patentansprüche

**1.** Zusammensetzung enthaltend ein thermischem, oxidativem und/oder actinischem Abbau unterliegendes organisches Material und mindestens eine Verbindung der Formel (I)

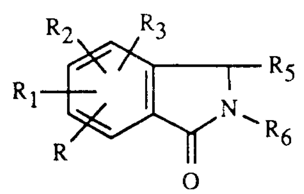

(I),

worin R Wasserstoff, $C_1$-$C_{18}$-Alkyl, $C_1$-$C_{18}$-Alkoxy, Halogen, Nitro, Phenyl-$C_1$-$C_4$-alkoxy, $C_2$-$C_{18}$-Alkanoyl, Benzoyl, ($C_1$-$C_6$-Alkyl)-benzoyl, $C_2$-$C_{18}$-Alkenoyl, -N($R_7$)($R_{7a}$), -OH oder -CO-A ist,
$R_1$ die gleichen Bedeutungsmöglichkeiten wie R hat,
$R_2$ Wasserstoff oder $C_1$-$C_4$-Alkyl und
$R_3$ Wasserstoff oder Halogen bedeuten,
A Hydroxy, $C_1$-$C_{18}$-Alkoxy, $C_5$-$C_{12}$-Cycloalkoxy, Benzyloxy oder mit $C_1$-$C_6$-Alkyl und/oder Halogen substituiertes Benzyloxy, -N($R_7$)($R_{7a}$), -NH-NH-$R_8$ oder -$G_1$-$(G)_n$ ist,
n eine Zahl von 1 bis 3 und G einen Rest der Formel

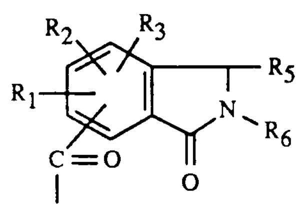

(II)

bedeuten,
$G_1$ im Fall n = 1 für -O-$R_{20}$-O-, -NH-$R_{21}$-NH- oder -NH-NH-,
im Fall n = 2 für eine Gruppe der Formel

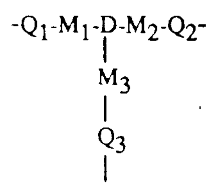

(III)

und im Fall n = 3 für $(-O-CH_2)_4C$ oder

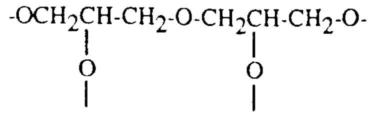

steht, wobei D N, CH oder C($C_1$-$C_4$-Alkyl) ist, $Q_1$, $Q_2$ und $Q_3$ unabhängig voneinander O oder NH und $M_1$, $M_2$ und $M_3$ unabhängig voneinander $C_1$-$C_6$-Alkylen oder durch -O-unterbrochenes $C_2$-$C_{12}$-Alkylen bedeuten, oder im Falle D = CH oder C($C_1$-$C_4$-Alkyl) -$M_3$-$Q_3$ auch für -O- oder im Falle D = N -$M_3$-$Q_3$ auch für eine direkte Bindung steht, $R_5$ und $R_6$ zusammen eine Gruppe der Formel

(IV) 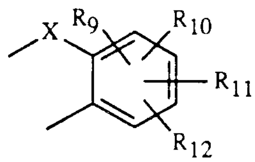 , 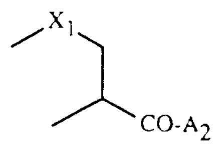 (V),

(VI) 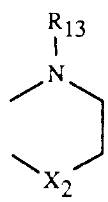 oder 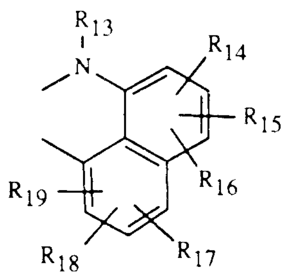 (VII)

bilden,
X -O-, -S-,

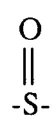 , 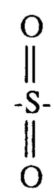

oder

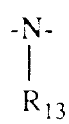 ,

$X_1$ -S-,

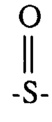

oder

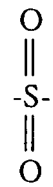

und

$X_2$ $-CH_2-$ oder eine direkte Bindung bedeuten,

$R_7$ und $R_{7a}$ unabhängig voneinander Wasserstoff, $C_1$-$C_{18}$-Alkyl, Phenyl-$C_1$-$C_4$-alkyl, $C_2$-$C_{18}$-Alkanoyl, $C_2$-$C_{18}$-Alkenoyl, $C_2$-$C_4$-Hydroxyalkyl, Benzoyl oder ($C_1$-$C_6$-Alkyl)-benzoyl, oder zusammen mit dem N-Atom, an das sie gebunden sind, Piperidyl, Morpholinyl, Piperazinyl oder 4-Methylpiperazinyl bedeuten,

$R_8$ Wasserstoff, $C_2$-$C_{18}$-Alkanoyl, Benzoyl oder ($C_1$-$C_6$-Alkyl)-benzoyl ist,

$R_9$, $R_{10}$, $R_{11}$ und $R_{12}$ die gleichen Bedeutungsmöglichkeiten wie R, $R_1$, $R_2$ und $R_3$ (in dieser Reihenfolge) haben, wobei an Stelle von -CO-A -CO-$A_1$ zu setzen ist,

$R_{13}$ Wasserstoff, $C_1$-$C_{18}$-Alkyl, $C_2$-$C_{18}$-Alkenyl, $C_5$-$C_{12}$-Cycloalkyl, Phenyl-$C_1$-$C_4$-alkyl, $C_2$-$C_{18}$-Alkanoyl, $C_2$-$C_{18}$-Alkenoyl, Benzoyl, ($C_1$-$C_6$-Alkyl)-benzoyl, Cyano-$C_1$-$C_4$-alkyl, $C_2$-$C_{19}$-Alkoxycarbonyl-$C_1$-$C_4$-alkyl, Carboxy-$C_1$-$C_4$-alkyl, Hydroxy-$C_2$-$C_4$-alkyl oder

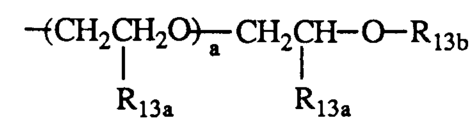

bedeutet,

$R_{13a}$ für Wasserstoff oder Methyl und $R_{13b}$ für Wasserstoff, $C_1$-$C_{18}$-Alkyl, Phenyl-$C_1$-$C_4$-alkyl, $C_2$-$C_{18}$-Alkanoyl, Benzoyl, $C_2$-$C_{18}$-Alkenoyl oder ($C_1$-$C_6$-Alkyl)-benzoyl und a für eine Zahl von 0 bis 6 stehen,

$R_{14}$, $R_{15}$, $R_{16}$, $R_{17}$, $R_{18}$ und $R_{19}$ unabhängig voneinander für Wasserstoff, $C_1$-$C_4$-Alkyl, Halogen, Hydroxy, $C_1$-$C_{18}$-Alkoxy oder -N($R_{7b}$)($R_{7c}$) stehen, worin $R_{7b}$ und $R_{7c}$ unabhängig voneinander Wasserstoff oder $C_1$-$C_{18}$-Alkyl bedeuten,

$R_{20}$ $C_1$-$C_{12}$-Alkylen, $C_2$-$C_8$-Alkenylen, $C_3$-$C_6$-Alkinylen oder durch O, S oder/und -N($R_{22}$)- unterbrochenes $C_2$-$C_{24}$-Alkylen,

$R_{21}$ $C_1$-$C_{12}$-Alkylen und $R_{22}$ Wasserstoff, $C_1$-$C_{18}$-Alkyl, Phenyl, ($C_1$-$C_6$-Alkyl)-phenyl oder Phenyl-$C_1$-$C_4$-alkyl bedeuten, $A_1$ Hydroxy, $C_1$-$C_{24}$-Alkoxy, $C_5$-$C_{12}$-Cycloalkoxy, Benzyloxy oder mit $C_1$-$C_6$-Alkyl und/oder Halogen substituiertes Benzyloxy, -N($R_7$)($R_{7a}$),-NH-NH-$R_8$ oder -$G_1$-$(G_2)_n$ ist, und $A_2$ Hydroxy, $C_1$-$C_{24}$-Alkoxy, $C_5$-$C_{12}$-Cycloalkoxy, Benzyloxy oder mit $C_1$-$C_6$-Alkyl und/oder Halogen substituiertes Benzyloxy, -N($R_7$)($R_{7a}$),-NH-NH-$R_8$ oder -$G_1$-$(G_3)_n$ ist, wobei $G_1$ und n wie oben definiert sind,

$G_2$ für einen Rest der Formel

(VIII) 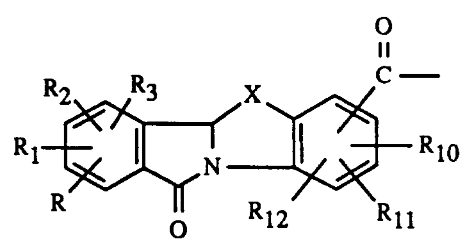

und

$G_3$ für einen Rest der Formel

(IX) 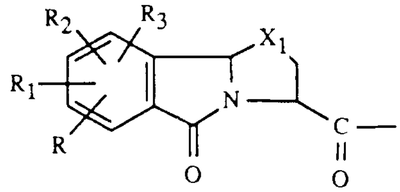

steht,

mit der Maßgabe, daß im Molekül nur ein Substituent -CO-A, CO-$A_1$ bzw. -CO-$A_2$ vorhanden ist, in dem A, $A_1$ bzw. $A_2$ einen Rest -$G_1$-$(G)_n$, -$G_1$-$(G_2)_n$ bzw. $G_1$-$(G_3)_n$ darstellt.

**2.** Zusammensetzung nach Anspruch 1, worin R Wasserstoff, Hydroxy, $C_1$-$C_4$-Alkyl, Chlor, Nitro, $C_1$-$C_4$-Alkoxy, Benzoyl, -N($R_7$)($R_{7a}$) oder -CO-A ist,
$R_1$ die gleichen Bedeutungsmöglichkeiten wie R hat,
$R_2$ Wasserstoff oder $C_1$-$C_4$-Alkyl und
$R_3$ Wasserstoff oder Chlor bedeuten,
A Hydroxyl, $C_1$-$C_{18}$-Alkoxy, $C_5$-$C_6$-Cycloalkoxy, Benzyloxy oder mit $C_1$-$C_4$-Alkyl und/oder Chlor substituiertes Benzyloxy, -N($R_7$)($R_{7a}$) oder -$G_1$-$(G)_n$ ist, n eine Zahl von 1 bis 3 und G einen Rest der Formel

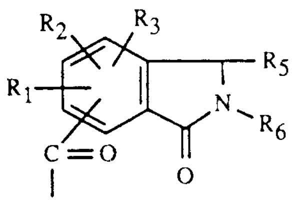 (II)

bedeuten,
$G_1$ im Fall n = 1 für -O-$R_{20}$-O- oder -NH-$R_{21}$-NH-,
im Fall n = 2 für eine Gruppe der Formel

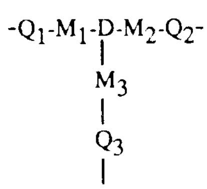 (III)

und im Fall n = 3 für $(-OCH_2)_4C$ steht, wobei D gleich N, CH oder C($C_1$-$C_4$-Alkyl) ist, $Q_1$, $Q_2$ und $Q_3$ unabhängig voneinander O oder NH und
$M_1$, $M_2$ und $M_3$ unabhängig voneinander $C_1$-$C_6$-Alkylen oder durch -O- unterbrochenes $C_2$-$C_{12}$-Alkylen bedeuten,
oder im Falle D = CH oder C($C_1$-$C_4$-Alkyl) -$M_3$-$Q_3$ auch für -O-oder im Falle D = N -$M_3$-$Q_3$ auch für eine direkte Bindung steht,
$R_5$ und $R_6$ zusammen eine Gruppe der Formel IV, V, VI oder VII bilden,
$R_7$ und $R_{7a}$ unabhängig voneinander Wasserstoff, $C_1$-$C_6$-Alkyl, Benzyl, $C_2$-$C_4$-Hydroxyalkyl, oder zusammen mit dem N-Atom, an das sie gebunden sind, Piperidyl, Morpholinyl, Piperazinyl oder 4-Methylpiperazinyl bedeuten,
$R_9$, $R_{10}$, $R_{11}$ und $R_{12}$ die gleichen Bedeutungsmöglichkeiten wie R, $R_1$, $R_2$ und $R_3$ (in dieser Reihenfolge) haben, wobei an Stelle von -CO-A -CO-$A_1$ zu setzen ist,
$R_{13}$ Wasserstoff, $C_1$-$C_{18}$-Alkyl, $C_2$-$C_{18}$-Alkenyl, $C_5$-$C_6$-Cycloalkyl, Phenyl-$C_1$-$C_4$-alkyl, $C_2$-$C_{18}$-Alkanoyl, $C_2$-$C_{18}$-Alkenoyl, Benzoyl, ($C_1$-$C_6$-Alkyl)-benzoyl, Cyano-$C_1$-$C_4$-alkyl $C_2$-$C_{19}$-Alkoxycarbonyl-$C_1$-$C_4$-alkyl, Carboxy-$C_1$-$C_4$-alkyl oder Hydroxy-$C_2$-$C_4$-alkyl bedeutet,
$R_{14}$, $R_{15}$, $R_{16}$, $R_{17}$, $R_{18}$ und $R_{19}$ unabhängig voneinander für Wasserstoff, $C_1$-$C_4$-Alkyl, Chlor, Hydroxy,

$C_1$-$C_4$-Alkoxy oder -N($R_{7b}$)($R_{7c}$) stehen, worin $R_{7b}$ und $R_{7c}$ unabhängig voneinander Wasserstoff oder $C_1$-$C_6$-Alkyl bedeuten,
$R_{20}$ $C_1$-$C_{12}$-Alkylen, $C_2$-$C_8$-Alkenylen, $C_3$-$C_6$-Alkinylen oder durch O, S oder/und -N($R_{22}$) unterbrochenes $C_2$-$C_{24}$-Alkylen,
$R_{21}$ $C_1$-$C_{12}$-Alkylen und $R_{22}$ Wasserstoff oder $C_1$ -$C_4$-Alkyl bedeuten, $A_1$ Hydroxy, $C_1$-$C_{18}$-Alkoxy, $C_5$-$C_6$-Cycloalkoxy, Benzyloxy oder mit $C_1$-$C_4$-Alkyl und/oder Halogen substituiertes Benzyloxy, -N($R_7$)-($R_{7a}$) oder -$G_1$-($G_2$)$_n$ ist und $A_2$ Hydroxy, $C_1$-$C_{18}$-Alkoxy, $C_5$-$C_6$-Cycloalkoxy, Benzyloxy oder mit $C_1$-$C_4$-Alkyl und/oder Halogen substituiertes Benzyloxy, -N($R_7$)($R_{7a}$) oder -$G_1$-($G_3$)$_n$ ist, wobei $G_1$ und n wie oben definiert sind,
$G_2$ für einen Rest der Formel

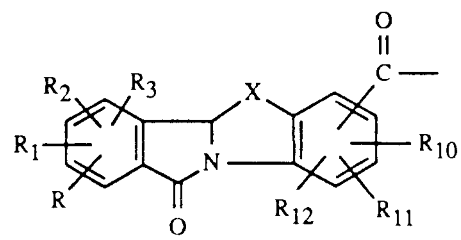

und
$G_3$ für einen Rest der Formel

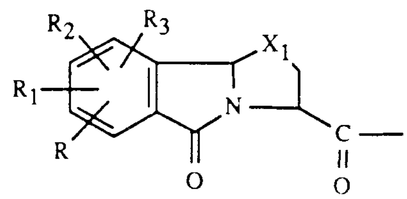

steht.

3. Zusammensetzung nach Anspruch 1, worin
R Wasserstoff, Chlor, Hydroxy oder $C_1$-$C_4$-Alkyl ist,
$R_1$ Wasserstoff, Chlor, $C_1$-$C_4$-Alkyl oder Hydroxy,
$R_2$ und $R_3$ Wasserstoff sind,
$R_5$ und $R_6$ zusammen eine Gruppe der Formel IV, V, VI oder VII bilden,
$R_9$, $R_{10}$, $R_{11}$ und $R_{12}$ die gleichen Bedeutungsmöglichkeiten wie R, $R_1$, $R_2$ und $R_3$ (in dieser Reihenfolge) haben,
$R_{13}$ Wasserstoff, $C_1$-$C_{18}$-Alkyl, $C_5$-$C_6$-Cycloalkyl, Benzyl, $C_2$-$C_{19}$-Alkoxycarbonyl-$C_1$-$C_4$-alkyl, Carboxy-$C_1$-$C_4$-alkyl, $C_2$-$C_{18}$-Alkanoyl oder Benzoyl bedeutet,
$R_{14}$, $R_{15}$, $R_{16}$, $R_{17}$, $R_{18}$ und $R_{19}$ Wasserstoff sind und
$A_2$ Hydroxy, $C_1$-$C_{18}$-Alkoxy, $C_5$-$C_6$-Cycloalkoxy oder Benzyloxy bedeutet.

4. Zusammensetzung nach Anspruch 1, worin R und $R_9$ unabhängig voneinander Wasserstoff, $C_1$-$C_4$-Alkyl oder Chlor und $R_1$, $R_2$, $R_3$, $R_{10}$, $R_{11}$, $R_{12}$, $R_{14}$, $R_{15}$, $R_{16}$, $R_{17}$, $R_{18}$ und $R_{19}$ Wasserstoff bedeuten.

5. Zusammensetzung nach Anspruch 1, worin A, $A_1$ und $A_2$ von -$G_1$-(G)$_n$, -$G_1$-($G_2$)$_n$ bzw. -$G_1$-($G_3$)$_n$ verschieden sind.

6. Zusammensetzung nach Anspruch 1, worin R Wasserstoff, Methyl oder Chlor bedeutet,
$R_1$, $R_2$ und $R_3$ Wasserstoff sind, und $R_5$ und $R_6$ zusammen eine Gruppe der Formeln IV, V, VI oder VII darstellen, worin X -S-,

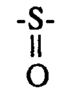

oder

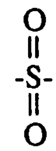 ,

$X_1$ -S-, $X_2$ $-CH_2-$, $R_9$ Wasserstoff, $-CH_3$, Cl oder -COO-($C_1$-$C_{24}$-Alkyl), $R_{10}$, $R_{11}$ und $R_{12}$ Wasserstoff, $R_{13}$ Wasserstoff, $C_1$-$C_{18}$-Alkyl, Benzoyl, $C_2$-$C_{19}$-Alkoxycarbonyl-methyl oder $C_2$-$C_{18}$-Alkanoyl, $R_{14}$ bis $R_{19}$ Wasserstoff und $A_2$ $C_1$-$C_{24}$-Alkoxy bedeuten.

**7.** Zusammensetzung nach Anspruch 1, worin das organische Material ein Schmierstoff, eine Metallbearbeitungsflüssigkeit, eine Hydraulikflüssigkeit oder ein natürliches oder (halb)synthetisches, insbesondere ein synthetisches, Polymer ist.

**8.** Zusammensetzung nach Anspruch 7, worin das organische Material ein thermoplastischer Kunststoff oder ein Elastomer ist.

**9.** Zusammensetzung nach Anspruch 1, die zusätzlich weitere Stabilisatoren wie Antioxidantien, Lichtschutzmittel und Verarbeitungsstabilisatoren (Hitzestabilisatoren), insbesondere ein organisches Phosphit, enthält.

**10.** Verbindungen der Formel X

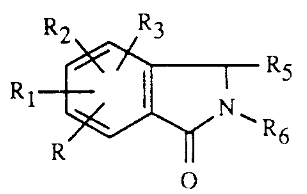

(X),

worin R Wasserstoff, $C_1$-$C_{18}$-Alkyl, $C_1$-$C_{18}$-Alkoxy, Halogen, Nitro, Phenyl-$C_1$-$C_4$-alkoxy, $C_2$-$C_{18}$-Alkanoyl, Benzoyl ($C_1$-$C_6$-Alkyl)-benzoyl, $C_2$-$C_{18}$-Alkenoyl, -N($R_7$)($R_{7a}$), -OH oder -CO-A ist,
$R_1$ die gleichen Bedeutungsmöglichkeiten wie R hat,
$R_2$ Wasserstoff oder $C_1$-$C_4$-Alkyl und
$R_3$ Wasserstoff oder Halogen bedeuten,
A Hydroxy, $C_1$-$C_{18}$-Alkoxy, $C_5$-$C_{12}$-Cycloalkoxy, Benzyloxy oder mit $C_1$-$C_4$-Alkyl und/oder Halogen substituiertes Benzyloxy, -N($R_7$)($R_{7a}$), -NH-NH-$R_8$ oder -$G_1$-$(G)_n$ ist,
n eine Zahl von 1 bis 3 und G einen Rest der Formel

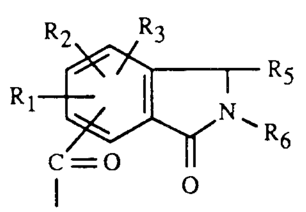

(II)

bedeuten,
$G_1$ im Fall n = 1 für -O-$R_{20}$-O-, -NH-$R_{21}$-NH- oder -NH-NH-,
im Fall n = 2 für eine Gruppe der Formel

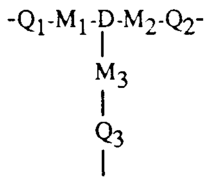

(III)

und im Fall n = 3 für $(-O-CH_2)_4C$ oder

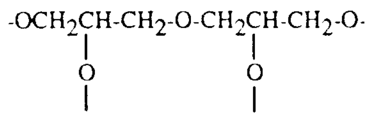

steht, wobei D N, CH oder $C(C_1-C_4\text{-Alkyl})$ ist, $Q_1$, $Q_2$ und $Q_3$ unabhängig voneinander O oder NH und $M_1$, $M_2$ und $M_3$ unabhängig voneinander $C_1$-$C_6$-Alkylen oder durch -O-unterbrochenes $C_2$-$C_{12}$-Alkylen bedeuten, oder im Falle D = CH oder $C(C_1-C_4\text{-Alkyl})$ $-M_3-Q_3$ auch für -O- oder im Falle D = N $-M_3-Q_3$ auch für eine direkte Bindung steht,

$R_5$ und $R_6$ zusammen eine Gruppe der Formel IV, V oder VII bilden

(IV) 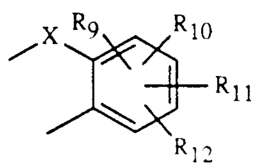 , 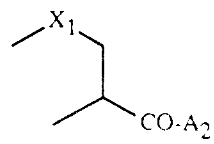 (V),

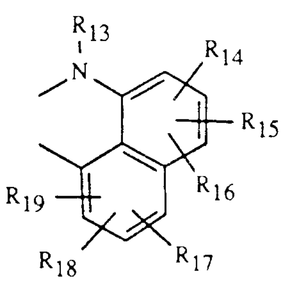 (VII)

X-S-,

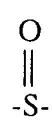

oder

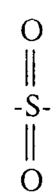 ,

$X_1$ -S-,

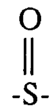

oder

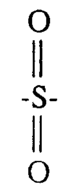

bedeuten

wobei, wenn X S oder S=O ist und einer der Substituenten $R_9$-$R_{12}$ H, Alkoxy oder Halogen bedeutet, die restlichen 3 dieser Substituenten nicht gleichzeitig für H stehen können,

$R_7$ und $R_{7a}$ unabhängig voneinander Wasserstoff, $C_1$-$C_{18}$-Alkyl, Phenyl-$C_1$-$C_4$-alkyl, $C_2$-$C_{18}$-Alkanoyl, $C_2$-$C_{18}$-Alkenoyl, $C_2$-$C_4$-Hydroxyalkyl, Benzoyl oder ($C_1$-$C_6$-Alkyl)-benzoyl, oder zusammen mit dem N-Atom, an das sie gebunden sind, Piperidyl, Morpholinyl, Piperazinyl oder 4-Methylpiperazinyl bedeuten,

$R_8$ Wasserstoff, $C_2$-$C_{18}$-Alkanoyl, Benzoyl oder ($C_1$-$C_6$-Alkyl)-benzoyl ist,

$R_9$, $R_{10}$, $R_{11}$ und $R_{12}$ die gleichen Bedeutungsmöglichkeiten wie R, $R_1$, $R_2$ und $R_3$ (in dieser Reihenfolge) haben, wobei an Stelle von -CO-A -CO-$A_1$ zu setzen ist,

$R_{13}$ Wasserstoff, $C_1$-$C_{18}$-Alkyl, $C_2$-$C_{18}$-Alkenyl, $C_5$-$C_{12}$-Cycloalkyl, Phenyl-$C_1$-$C_4$-alkyl, $C_2$-$C_{18}$-Alkanoyl, $C_2$-$C_{18}$-Alkenoyl, Benzoyl, ($C_1$-$C_6$-Alkyl)-benzoyl, Cyano-$C_1$-$C_4$-alkyl, $C_2$-$C_{19}$-Alkoxycarbonyl-$C_1$-$C_4$-alkyl, Carboxy-$C_1$-$C_4$-alkyl, Hydroxy-$C_2$-$C_4$-alkyl oder

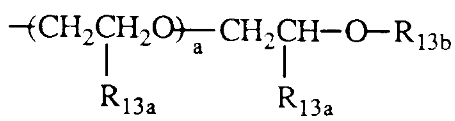

bedeutet,

$R_{13a}$ für Wasserstoff oder Methyl und $R_{13b}$ für Wasserstoff, $C_1$-$C_{18}$-Alkyl, Phenyl-$C_1$-$C_4$-alkyl, $C_2$-$C_{18}$-Alkanoyl, Benzoyl, $C_2$-$C_{18}$-Alkenoyl oder ($C_1$-$C_6$-Alkyl)-benzoyl und a für eine Zahl von 0 bis 6 stehen,

$R_{14}$, $R_{15}$, $R_{16}$, $R_{17}$, $R_{18}$ und $R_{19}$ unabhängig voneinander für Wasserstoff, $C_1$-$C_4$-Alkyl, Halogen, Hydroxy, $C_1$-$C_{18}$-Alkoxy oder -N($R_{7b}$)($R_{7c}$) stehen, worin $R_{7b}$ und $R_{7c}$ unabhängig voneinander Wasserstoff oder $C_1$-$C_{18}$-Alkyl bedeuten,

$R_{20}$ $C_1$-$C_{12}$-Alkylen, $C_2$-$C_8$-Alkenylen, $C_3$-$C_6$-Alkinylen oder durch O, S oder/und -N($R_{22}$)- unterbrochenes $C_2$-$C_{24}$-Alkylen,

$R_{21}$ $C_1$-$C_{12}$-Alkylen und $R_{22}$ Wasserstoff, $C_1$-$C_{18}$-Alkyl, Phenyl, ($C_1$-$C_6$-Alkyl)-phenyl oder Phenyl-$C_1$-$C_4$-alkyl bedeuten, $A_1$ Hydroxy, $C_1$-$C_{24}$-Alkoxy, $C_5$-$C_{12}$-Cycloalkoxy, Benzyloxy oder mit $C_1$-$C_4$-Alkyl und/oder Halogen substituiertes Benzyloxy, -N($R_7$)($R_{7a}$),-NH-NH-$R_8$ oder -$G_1$-($G_2$)$_n$ ist, und $A_2$ Hydroxy, $C_1$-$C_{24}$-Alkoxy, $C_5$-$C_{12}$-Cycloalkoxy, Benzyloxy oder mit $C_1$-$C_4$-Alkyl und/oder Halogen substituiertes Benzyloxy, -N($R_7$)($R_{7a}$),-NH-NH-$R_8$ oder -$G_1$-($G_3$)$_n$ ist, wobei $G_1$ und n wie oben definiert sind,

$G_2$ für einen Rest der Formel

(VIII) 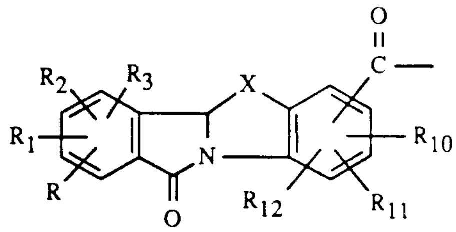

und

$G_3$ für einen Rest der Formel

(IX) 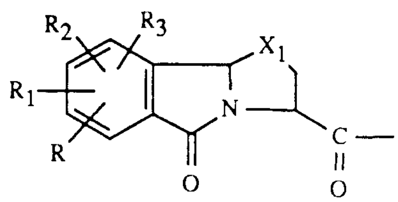

steht,

mit den Maßgaben, (1) daß im Molekül nur ein Substituent -CO-A, CO-$A_1$ bzw. -CO-$A_2$vorhanden ist, in dem A, $A_1$ bzw. $A_2$ einen Rest -$G_1$-$(G)_n$, -$G_1$-$(G_2)_n$ bzw. $G_1$-$(G_3)_n$ darstellt und (2) däß im Fall X = S höchstens einer der Substituenten R und $R_1$ für $C_1$-$C_{18}$-Alkoxy steht.

**11.** Verbindungen nach Anspruch 10, worin R Wasserstoff, Hydroxy, $C_1$-$C_4$-Alkyl, Chlor, Nitro, $C_1$-$C_4$-Alkoxy, Benzoyl, -N($R_7$)($R_{7a}$) oder -CO-A ist,
$R_1$ die gleichen Bedeutungsmöglichkeiten wie R hat,
$R_2$ Wasserstoff oder $C_1$-$C_4$-Alkyl und
$R_3$ Wasserstoff oder Chlor bedeuten,
A Hydroxyl, $C_1$-$C_{18}$-Alkoxy, $C_5$-$C_6$-Cycloalkoxy, Benzyloxy oder mit $C_1$-$C_4$-Alkyl und/oder Chlor substituiertes Benzyloxy, -N($R_7$)($R_{7a}$) oder -$G_1$-$(G)_n$ ist, n eine Zahl von 1 bis 3 und G einen Rest der Formel

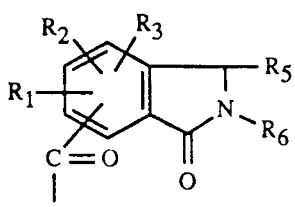 (II)

bedeuten,
$G_1$ im Fall n = 1 für -O-$R_{20}$-O- oder -NH-$R_{21}$-NH-,
im Fall n = 2 für eine Gruppe der Formel

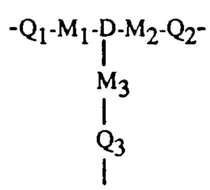 (III)

und im Fall n = 3 für $(-OCH_2)_4C$ steht, wobei D gleich N, CH oder C($C_1$-$C_4$-Alkyl) ist,
$Q_1$, $Q_2$ und $Q_3$ unabhängig voneinander O oder NH und

$M_1$, $M_2$ und $M_3$ unabhängig voneinander $C_1$-$C_6$-Alkylen oder durch -O- unterbrochenes $C_2$-$C_{12}$-Alkylen bedeuten,
oder im Falle D = CH oder C($C_1$-$C_4$-Alkyl) -$M_3$-$Q_3$ auch für -O- oder im Falle D = N -$M_3$-$Q_3$ auch für eine direkte Bindung steht,
$R_5$ und $R_6$ zusammen eine Gruppe der Formel IV, V oder VII bilden,
$R_7$ und $R_{7a}$ unabhängig voneinander Wasserstoff, $C_1$-$C_6$-Alkyl, Benzyl, $C_2$-$C_4$-Hydroxyalkyl, oder zusammen mit dem N-Atom, an das sie gebunden sind, Piperidyl, Morpholinyl, Piperazinyl oder 4-Methylpiperazinyl bedeuten,
$R_9$, $R_{10}$, $R_{11}$ und $R_{12}$ die gleichen Bedeutungsmöglichkeiten wie R, $R_1$, $R_2$ und $R_3$ (in dieser Reihenfolge) haben, wobei an Stelle von -CO-A -CO-$A_1$ zu setzen ist,
$R_{13}$ Wasserstoff, $C_1$-$C_{18}$-Alkyl, $C_2$-$C_{18}$-Alkenyl, $C_5$-$C_6$-Cycloalkyl, Phenyl-$C_1$-$C_4$-alkyl, $C_2$-$C_{18}$-Alkanoyl, $C_2$-$C_{18}$-Alkenoyl, Benzoyl, ($C_1$-$C_6$-Alkyl)-benzoyl, $C_2$-$C_{19}$-Alkoxycarbonyl-$C_1$-$C_4$-alkyl, Carboxy-$C_1$-$C_4$-alkyl, Cyano-$C_1$-$C_4$-alkyl oder Hydroxy-$C_2$-$C_4$-alkyl bedeutet,
$R_{14}$, $R_{15}$, $R_{16}$, $R_{17}$, $R_{18}$ und $R_{19}$ unabhängig voneinander für Wasserstoff, $C_1$-$C_4$-Alkyl, Chlor, Hydroxy, $C_1$-$C_4$-Alkoxy oder -N($R_{7b}$)($R_{7c}$) stehen, worin $R_{7b}$ und $R_{7c}$ unabhängig voneinander Wasserstoff oder $C_1$-$C_6$-Alkyl bedeuten,
$R_{20}$ $C_1$-$C_{12}$-Alkylen, $C_2$-$C_8$-Alkenylen, $C_3$-$C_6$-Alkinylen oder durch O, S oder/und -N($R_{22}$) unterbrochenes $C_2$-$C_{24}$-Alkylen,
$R_{21}$ $C_1$-$C_{12}$-Alkylen und $R_{22}$ Wasserstoff oder $C_1$-$C_4$-Alkyl bedeuten, $A_1$ Hydroxy, $C_1$-$C_{18}$-Alkoxy, $C_5$-$C_6$-Cycloalkoxy, Benzyloxy oder mit $C_1$-$C_4$-Alkyl und/oder Halogen substituiertes Benzyloxy, -N($R_7$)-($R_{7a}$) oder -$G_1$-$(G_2)_n$ ist und $A_2$ Hydroxy, $C_1$-$C_{18}$-Alkoxy, $C_5$-$C_6$-Cycloalkoxy, Benzyloxy oder mit $C_1$-$C_4$-Alkyl und/oder Halogen substituiertes Benzyloxy, -N($R_7$)($R_{7a}$) oder -$G_1$-$(G_3)_n$ ist, wobei $G_1$ und n wie oben definiert sind,
$G_2$ für einen Rest der Formel

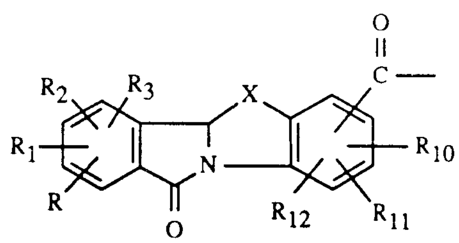

und
$G_3$ für einen Rest der Formel

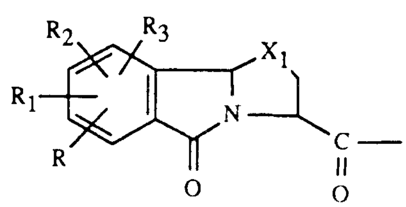

steht.

**12.** Verbindungen nach Anspruch 10, worin
R Wasserstoff, Chlor, Hydroxy oder $C_1$-$C_4$-Alkyl ist,
$R_1$ Wasserstoff, Chlor, $C_1$-$C_4$-Alkyl oder Hydroxy,
$R_2$ und $R_3$ Wasserstoff sind,
$R_5$ und $R_6$ zusammen eine Gruppe der Formel IV, V oder VII bilden,
$R_9$, $R_{10}$, $R_{11}$ und $R_{12}$ die gleichen Bedeutungsmöglichkeiten wie R, $R_1$, $R_2$ und $R_3$ (in dieser Reihenfolge) haben,
$R_{13}$ Wasserstoff, $C_1$-$C_{18}$-Alkyl, $C_5$-$C_6$-Cycloalkyl, Benzyl, $C_2$-$C_{18}$-Alkanoyl, $C_2$-$C_{19}$-Alkoxycarbonyl-$C_1$-$C_4$-alkyl oder Benzoyl bedeutet,

$R_{14}$, $R_{15}$, $R_{16}$, $R_{17}$, $R_{18}$ und $R_{19}$ Wasserstoff sind und
$A_2$ Hydroxy, $C_1$-$C_{18}$-Alkoxy, $C_5$-$C_6$-Cycloalkoxy oder Benzyloxy bedeutet.

**13.** Verbindungen nach Anspruch 12, worin R Wasserstoff, Methyl oder Chlor bedeutet, $R_1$, $R_2$ und $R_3$ Wasserstoff sind, und $R_5$ und $R_6$ zusammen eine Gruppe der Formeln IV, V oder VII darstellen, worin X -S-,

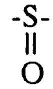

oder

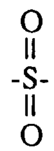 ,

$X_1$ -S-, $R_9$ Wasserstoff, $-CH_3$, Cl oder -COO-($C_1$-$C_{24}$-Alkyl), $R_{10}$, $R_{11}$ und $R_{12}$ Wasserstoff, $R_{13}$ Wasserstoff, $C_1$-$C_{18}$-Alkyl, Benzoyl $C_2$-$C_{19}$-Alkoxycarbonyl-methyl oder $C_2$-$C_{18}$-Alkanoyl, $R_{14}$ bis $R_{19}$ Wasserstoff und $A_2$ $C_1$-$C_{24}$-Alkoxy bedeuten.

**14.** Verbindungen nach Anspruch 10, worin R und $R_9$ unabhängig voneinander Wasserstoff, $C_1$-$C_4$-Alkyl oder Chlor und $R_1$, $R_2$, $R_3$, $R_{10}$, $R_{11}$, $R_{12}$, $R_{14}$, $R_{15}$, $R_{16}$, $R_{17}$, $R_{18}$ und $R_{19}$ Wasserstoff bedeuten.

**15.** Verwendung von Verbindungen der Formeln I und X gemäss den Ansprüchen 1 und 10 zum Stabilisieren organischen Materials gegen oxidativen, thermischen und/oder actinischen Abbau.

## Claims

**1.** A composition comprising an organic material liable to thermal, oxidative and/or actinic degradation and at least one compound of the formula (I)

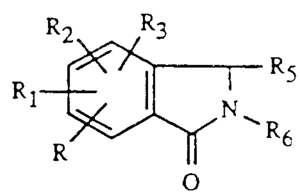

(I),

in which R is hydrogen, $C_1$-$C_{18}$alkyl, $C_1$-$C_{18}$alkoxy, halogen, nitro, phenyl$C_1$-$C_4$alkoxy, $C_2$-$C_{18}$alkanoyl, benzoyl, ($C_1$-$C_6$alkyl)benzoyl, $C_2$-$C_{18}$alkenoyl, -N($R_7$)($R_{7a}$), -OH or -CO-A, $R_1$ has the same possibilities of meaning as R, $R_2$ is hydrogen or $C_1$-$C_4$alkyl and $R_3$ is hydrogen or halogen, A is hydroxyl, $C_1$-$C_{18}$alkoxy, $C_5$-$C_{12}$cycloalkoxy, benzyloxy or benzyloxy substituted by $C_1$-$C_4$alkyl and/or halogen, -N-($R_7$)($R_{7a}$), -NH-NH-$R_8$ or -$G_1$-$(G)_n$,n is a number from 1 to 3 and G is a radical of the formula

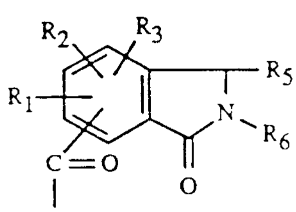

(II)

$G_1$ in the case where n = 1 is $-O-R_{20}-O-$, $-NH-R_{21}-NH-$ or -NH-NH-, in the case where n = 2 it is a group of the formula

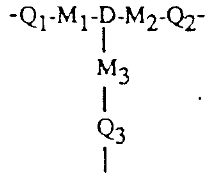 (III)

and in the case where n = 3 it is $(-O-CH_2)_4C$ or

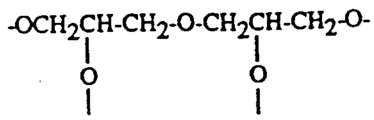

where D is N, CH or $C(C_1-C_4alkyl)$, $Q_1$, $Q_2$ and $Q_3$ independently of one another are O or NH and $M_1$, $M_2$ and $M_3$ independently of one another are $C_1-C_6$alkylene or $C_2-C_{12}$alkylene which is interrupted by -O-, or in the case where D = CH or $C(C_1-C_4alkyl)$ $-M_3-Q_3$ is otherwise -O- or in the case where D = N $-M_3-Q_3$ is otherwise a direct bond,
$R_5$ and $R_6$ together form a group of the formula

(IV) 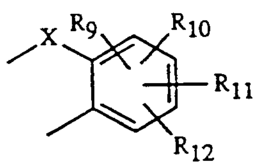 , 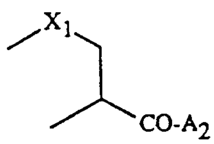 (V),

(VI) 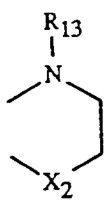 or 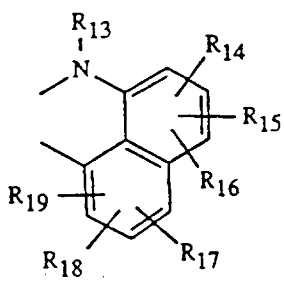 (VII)

X -O-, -S-,

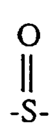 , 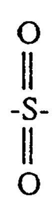

or

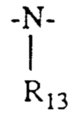

$X_1$ -S-,

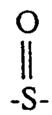

or

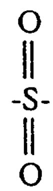

and

$X_2$ is $-CH_2$ or a direct bond, $R_7$ and $R_{7a}$ independently of one another are hydrogen, $C_1$-$C_{18}$alkyl, phenyl-$C_1$-$C_4$alkyl, $C_2$-$C_{18}$alkanoyl, $C_2$-$C_{18}$alkenoyl, $C_2$-$C_4$hydroxyalkyl, benzoyl or ($C_1$-$C_6$alkyl)benzoyl or, together with the N atom to which they are bonded, are piperidyl, morpholinyl, piperazinyl or 4-methylpiperazinyl, $R_8$ is hydrogen, $C_2$-$C_{18}$alkanoyl, benzoyl or ($C_1$-$C_6$alkyl)benzoyl, $R_9$, $R_{10}$, $R_{11}$ and $R_{12}$ have the same possibilities of meaning as R, $R_1$, $R_2$ and $R_3$ (in this sequence), where -CO-A is to be replaced by -CO-$A_1$, $R_{13}$ is hydrogen, $C_1$-$C_{18}$alkyl, $C_2$-$C_{18}$alkenyl,$C_5$-$C_{12}$cycloalkyl, phenyl-$C_1$-$C_4$alkyl, $C_2$-$C_{18}$alkanoyl, $C_2$-$C_{18}$alkenoyl, benzoyl, ($C_1$-$C_6$alkyl)benzoyl, cyano-$C_1$-$C_4$alkyl, $C_2$-$C_{19}$alkoxycarbonyl-$C_1$-$C_4$alkyl, carboxy-$C_1$-$C_4$alkyl, hydroxy-$C_2$-$C_4$alkyl or

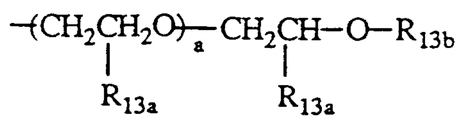 ,

$R_{13a}$ is hydrogen or methyl and $R_{13b}$ is hydrogen, $C_1$-$C_{18}$alkyl, phenyl-$C_1$-$C_4$alkyl, $C_1$-$C_{18}$alkanoyl, benzoyl, $C_2$-$C_{18}$alkenoyl or ($C_1$-$C_6$alkyl)benzoyl and a is a number from 0 to 6, $R_{14}$, $R_{15}$, $R_{16}$, $R_{17}$, $R_{18}$ and $R_{19}$ independently of one another are hydrogen, $C_1$-$C_4$alkyl, halogen, hydroxyl, $C_1$-$C_{18}$alkoxy or -N($R_{7b}$)($R_{7c}$), in which $R_{7b}$ and $R_{7c}$ independently of one another are hydrogen or $C_1$-$C_{18}$alkyl, $R_{20}$ is $C_1$-$C_{12}$alkylene, $C_2$-$C_8$alkenylene, $C_3$-$C_6$alkynylene or $C_2$-$C_{24}$alkylene which is interrupted by O, S and/or -N($R_{22}$), $R_{21}$ is $C_1$-$C_{12}$alkylene and $R_{22}$ is hydrogen, $C_1$-$C_{18}$alkyl, phenyl, $C_1$-$C_6$alkyl)phenyl or phenyl-$C_1$-$C_4$alkyl, $A_1$ is hydroxyl $C_1$-$C_{24}$alkoxy, $C_5$-$C_{12}$cycloalkoxy, benzyloxy or benzyloxy substituted by $C_1$-$C_6$alkyl and/or halogen, -N($R_7$)($R_{7a}$), -NH-NH-$R_8$ or $G_1$-$(G_2)_n$, and $A_2$ is hydroxyl, $C_1$-$C_{24}$alkoxy, $C_5$-$C_{12}$cycloalkoxy, benzyloxy or benzyloxy substituted by $C_1$-$C_6$alkyl and/or halogen, -N-($R_7$)($R_{7a}$), -NH-NH-$R_8$ or $G_1$-$(G_3)_n$, where $G_1$ and n are as defined above, $G_2$ is a radical of the formula

(VIII) 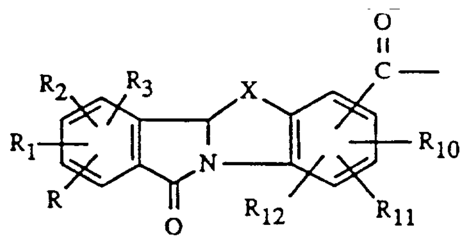

and
$G_3$ is a radical of the formula

(IX) 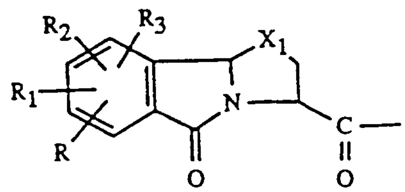

with the proviso that only one substituent -CO-A, CO-$A_1$ or -CO-$A_2$ is present in the molecule, in which A, $A_1$ or $A_2$ is a radical -$G_1$-$(G)_n$, -$G_1$-$(G_2)_n$ or -$G_1$-$(G_3)_n$.

**2.** A composition according to claim 1, in which R is hydrogen, hydroxyl, $C_1$-$C_4$alkyl, chlorine, nitro, $C_1$-$C_4$alkoxy, benzoyl, -N($R_7$)($R_{7a}$) or -CO-A, $R_1$ has the same possibilities of meaning as R, $R_2$ is hydrogen or $C_1$-$C_4$alkyl and $R_3$ is hydrogen or chlorine, A is hydroxyl, $C_1$-$C_{18}$alkoxy, $C_5$-$C_6$cycloalkoxy, benzyloxy or benzyloxy substituted by $C_1$-$C_4$alkyl and/or chlorine, -N($R_7$)($R_{7a}$) or -$G_1$-$(G)_n$, n is a number from 1 to 3 and G is a radical for the formula

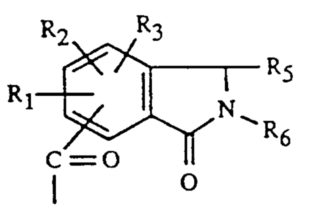 (II)

$G_1$ in the case where n = 1 is -O-$R_{20}$-O- or -NH-$R_{21}$-NH-,
in the case where n = 2 it is a group of the formula

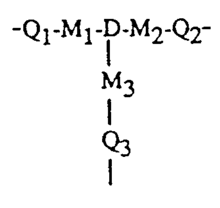 (III)

and in the case where n = 3 it is $(-OCH_2)_4C$, where D is equal to N, CH or C($C_1$-$C_4$alkyl), $Q_1$, $Q_2$ and $Q_3$ independently of one another are O or NH and $M_1$, $M_2$ and $M_3$ independently of one another are $C_1$-$C_6$alkylene or $C_2$-$C_{12}$alkylene interrupted by -O-, or in the case where D = CH or C($C_1$-$C_4$alkyl) -$M_3$-$Q_3$ is otherwise -O- or in the case where D = N -$M_3$-$Q_3$ is otherwise a direct bond, $R_5$ and $R_6$ together form a group of the formula IV, V, VI or VII, $R_7$ and $R_{7a}$ independently of one another are hydrogen, $C_1$-$C_6$alkyl, benzyl, $C_2$-$C_4$hydroxyalkyl or, together with the N atom to which they are bonded, are piperidyl, morpholinyl, piperazinyl or 4-methylpiperazinyl, $R_9$, $R_{10}$, $R_{11}$ and $R_{12}$ have the same possibilities of meaning as R, $R_1$, $R_2$ and $R_3$ (in this sequence), where -CO-A is to be replaced by -CO-$A_1$, $R_{13}$ is hydrogen, $C_1$-$C_{18}$alkyl, $C_2$-$C_{18}$alkenyl,$C_5$-$C_6$cycloalkyl, phenyl-$C_1$-$C_4$alkyl, $C_2$-$C_{18}$al-

kanoyl, $C_2$-$C_{18}$alkenoyl, benzoyl, ($C_1$-$C_6$alkyl)benzoyl, cyano-$C_1$-$C_4$alkyl, $C_2$-$C_{19}$alkoxycarbonyl-$C_1$-$C_4$alkyl, carboxy-$C_1$-$C_4$alkyl or hydroxy-$C_2$-$C_4$alkyl, $R_{14}$ $R_{15}$, $R_{16}$, $R_{17}$, $R_{18}$ and $R_{19}$ independently of one another are hydrogen, $C_1$-$C_4$alkyl, chlorine, hydroxyl, $C_1$-$C_4$alkoxy or -N($R_{7b}$($R_{7c}$), in which $R_{7b}$ and $R_{7c}$ independently of one another are hydrogen or $C_1$-$C_6$alkyl, $R_{20}$ is $C_1$-$C_{12}$alkylene, $C_2$-$C_8$alkenylene, $C_3$-$C_6$alkynylene or $C_2$-$C_{24}$alkylene interrupted by O, S and/or -N($R_{22}$), $R_{21}$ is $C_1$-$C_{12}$alkylene and $R_{22}$ is hydrogen or $C_1$-$C_4$alkyl, $A_1$ is hydroxyl, $C_1$-$C_{18}$alkoxy, $C_5$-$C_6$cycloalkoxy, benzyloxy orbenzyloxy substituted by $C_1$-$C_4$alkyl and/or hydrogen, -N($R_7$)($R_{7a}$) or -$G_1$-$(G_2)_n$ and $A_2$ is hydroxyl, $C_1$-$C_{18}$alkoxy, $C_5$-$C_6$cycloalkoxy, benzyloxy or benzyloxy substituted by $C_1$-$C_4$alkyl and/or halogen, -N($R_7$)($R_{7a}$) or -$G_1$-$(G_3)_n$, where $G_1$ and n are as defined above, $G_2$ is a radical of the formula

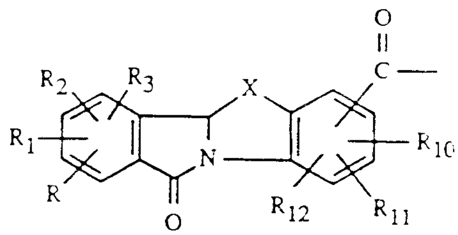

and
$G_3$ is a radical of the formula

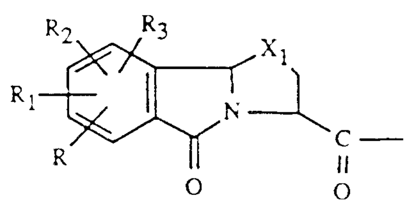

**3.** A composition according to claim 1, in which R is hydrogen, chlorine, hydroxyl or $C_1$-$C_4$alkyl, $R_1$ is hydrogen, chlorine, $C_1$-$C_4$alkyl or hydroxyl, $R_2$ and $R_3$ are hydrogen, $R_5$ and $R_6$ together form a group of the formula IV, V, VI or VII, $R_9$, $R_{10}$, $R_{11}$ and $R_{12}$ have the same possibilities of meaning as R, $R_1$, $R_2$ and $R_3$ (in this sequence), $R_{13}$ is hydrogen, $C_1$-$C_{18}$alkyl, $C_5$-$C_6$cycloalkyl, benzyl, $C_2$-$C_{19}$alkoxycarbonyl-$C_1$-$C_4$alkyl, carboxy-$C_1$-$C_4$alkyl, $C_2$-$C_{18}$alkanoyl or benzoyl, $R_{14}$, $R_{15}$, $R_{16}$, $R_{17}$, $R_{18}$ and $R_{19}$ are hydrogen and $A_2$ is hydroxyl, $C_1$-$C_{18}$alkoxy, $C_5$-$C_6$cycloalkoxy or benzoyloxy.

**4.** A composition according to claim 1, in which R and $R_9$ independently of one another are hydrogen, $C_1$-$C_4$alkyl or chlorine and $R_1$, $R_2$, $R_3$, $R_{10}$, $R_{11}$, $R_{12}$, $R_{14}$, $R_{15}$, $R_{16}$, $R_{17}$, $R_{18}$ and $R_{19}$ are hydrogen.

**5.** A composition according to claim 1, in which A, $A_1$ and $A_2$ are different from -$G_1$-$(G)_n$, -$G_1$-$(G_2)_n$ or -$G_1$-$(G_3)_n$.

**6.** A composition according to claim 1, in which R is hydrogen, methyl or chlorine, $R_1$, $R_2$ and $R_3$ are hydrogen, and $R_5$ and $R_6$ together are a group of the formula IV, V, VI or VII in which X is -S-,

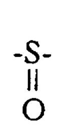

or

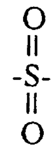

$X_1$ is -S-, $X_2$ is $-CH_2-$, $R_9$ is hydrogen, $-CH_3$, Cl or -COO- ($C_1$-$C_{24}$alkyl), $R_{10}$, $R_{11}$ and $R_{12}$ are hydrogen, $R_{13}$ is hydrogen, $C_1$-$C_{18}$alkyl, benzoyl, $C_2$-$C_{19}$alkoxycarbonyl-methyl or $C_2$-$C_{18}$alkanoyl, $R_{14}$ to $R_{19}$ are hydrogen and $A_2$ is $C_1$-$C_{24}$alkoxy.

**7.** A composition according to claim 1, in which the organic material is a lubricant, a metalworking fluid, a hydraulic fluid or a natural or (semi) synthetic polymer, in particular a synthetic polymer.

**8.** A composition according to claim 7, in which the organic material is a thermoplastic or an elastomer.

**9.** A composition according to claim 1, which additionally contains other stabilisers such as antioxidants, light stabilisers and processing stabilisers (heat stabilisers), in particular an organic phosphite.

**10.** A compound of the formula X

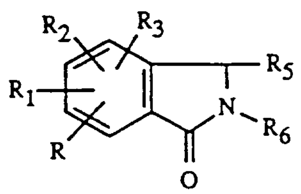

(X),

in which R is hydrogen, $C_1$-$C_{18}$alkyl, $C_1$-$C_{18}$alkoxy, halogen, nitro, phenyl-$C_1$-$C_4$alkoxy, $C_2$-$C_{18}$alkanoyl, benzoyl, ($C_1$-$C_6$alkyl)benzoyl, $C_2$-$C_{18}$alkenoyl, $-N(R_7)(R_{7a})$, -OH or -CO-A, $R_1$ has the same possibilities of meaning as R, $R_2$ is hydrogen or $C_1$-$C_4$alkyl and $R_3$ is hydrogen or halogen, A is hydroxyl, $C_1$-$C_{18}$alkoxy, $C_5$-$C_{12}$cycloalkoxy, benzyloxy or benzyloxy substituted by $C_1$-$C_4$alkyl and/or halogen, $-N(R_7)(R_{7a})$, $-NH-NH-R_8$ or $-G_1-(G)_n$,n is a number from 1 to 3 and G is a radical of the formula

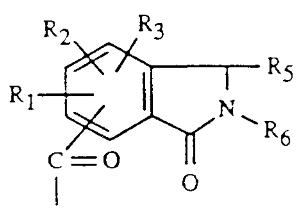

(II)

$G_1$ in the case where n = 1 is $-O-R_{20}-O-$, $-NH-R_{21}-NH-$ or -NH-NH-, in the case where n = 2 it is a group of the formula

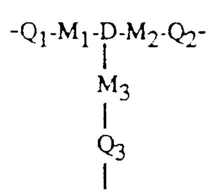

(III)

and in the case where n = 3 it is $(-O-CH_2)_4C$ or

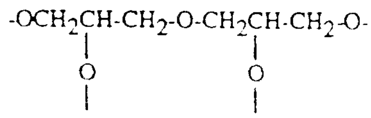

where D is N, CH or $C(C_1-C_4alkyl)$, $Q_1$, $Q_2$ and $Q_3$ independently of one another are O or NH and $M_1$, $M_2$ and $M_3$ independently of one another are $C_1-C_6$alkylene or $C_2-C_{12}$alkylene which is interrupted by -O-, or in the case where D = CH or $C(C_1-C_4alkyl)$ $-M_3-Q_3$ is otherwise -O- or in the case where D = N $-M_3-Q_3$ is otherwise a direct bond, $R_5$ and $R_6$ together form a group of the formula IV, V or VII

(IV) 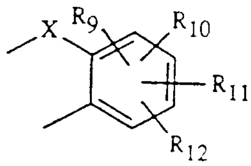 , 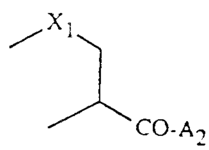 (V),

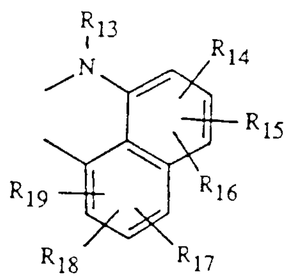 (VII)

X is -S-,

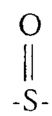

or

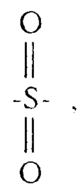 ,

$X_1$ is -S-,

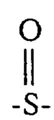

or

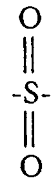

where if X is S or S=O and one of the substituents $R_9$-$R_{12}$ is H, alkoxy or halogen, the remaining 3 of these substituents cannot simultaneously be H, $R_7$ and $R_{7a}$ independently of one another are hydrogen, $C_1$-$C_{18}$alkyl, phenyl-$C_1$-$C_4$alkyl, $C_2$-$C_{18}$alkanoyl, $C_2$-$C_{18}$alkenoyl, $C_2$-$C_4$hydroxyalkyl, benzoyl or ($C_1$-$C_6$alkyl)benzoyl or, together with the N atom to which they are bonded, are piperidyl, morpholinyl, piperazinyl or 4-methylpiperazinyl, $R_8$ is hydrogen, $C_2$-$C_{18}$alkanoyl, benzoyl or ($C_1$-$C_6$alkyl)benzoyl, $R_9$, $R_{10}$, $R_{11}$ and $R_{12}$ have the same possibilities of meaning as R, $R_1$, $R_2$ and $R_3$ (in this sequence), where -CO-A is to be replaced by -CO-$A_1$, $R_{13}$ is hydrogen, $C_1$-$C_{18}$alkyl, $C_2$-$C_{18}$alkenyl,$C_5$-$C_{12}$cycloalkyl, phenyl-$C_1$-$C_4$alkyl, $C_2$-$C_{18}$alkanoyl, $C_2$-$C_{18}$alkenoyl, benzoyl, ($C_1$-$C_6$alkyl)benzoyl, cyano-$C_1$-$C_4$alkyl, $C_2$-$C_{19}$alkoxycarbonyl-$C_1$-$C_4$alkyl, carboxy-$C_1$-$C_4$alkyl, hydroxy-$C_2$-$C_4$alkyl or

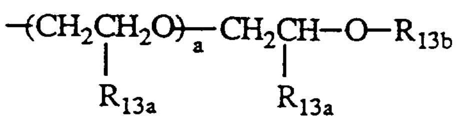 ,

$R_{13a}$ is hydrogen or methyl and $R_{13b}$ is hydrogen, $C_1$-$C_{18}$alkyl, phenyl-$C_1$-$C_4$alkyl, $C_2$-$C_{18}$alkanoyl, benzoyl, $C_2$-$C_{18}$alkenoyl or ($C_1$-$C_6$alkyl)benzoyl and a is a number from 0 to 6, $R_{14}$, $R_{15}$, $R_{16}$, $R_{17}$, $R_{18}$ and $R_{19}$ independently of one another are hydrogen, $C_1$-$C_4$alkyl, halogen, hydroxyl, $C_1$-$C_{18}$alkoxy or -N($R_{7b}$)($R_{7c}$), in which $R_{7b}$ and $R_{7c}$ independently of one another are hydrogen or $C_1$-$C_{18}$alkyl, $R_{20}$ is $C_1$-$C_{12}$alkylene, $C_2$-$C_8$alkenylene, $C_3$-$C_6$alkynylene or $C_2$-$C_{24}$alkylene interrupted by O, S and/or -N-($R_{22}$)-, $R_{21}$ is $C_1$-$C_{12}$alkylene and $R_{22}$ is hydrogen, $C_1$-$C_{18}$alkyl, phenyl, ($C_1$-$C_6$-alkyl)phenyl or phenyl-$C_1$-$C_4$alkyl, $A_1$ is hydroxyl, $C_1$-$C_{24}$alkoxy, $C_5$-$C_{12}$cycloalkoxy, benzyloxy or benzyloxy substituted by $C_1$-$C_4$alkyl and/or halogen, -N($R_7$)($R_{7a}$), -NH-NH-$R_8$ or -$G_1$-$(G_2)_n$, and $A_2$ is hydroxyl, $C_1$-$C_{24}$alkoxy, $C_5$-$C_{12}$cycloalkoxy, benzyloxy or benzyloxy substituted by $C_1$-$C_4$alkyl and/or halogen, -N($R_7$)($R_{7a}$), -NH-NH-$R_8$ or -$G_1$-$(G_3)_n$, where $G_1$ and n are as defined above, $G_2$ is a radical of the formula

(VIII) 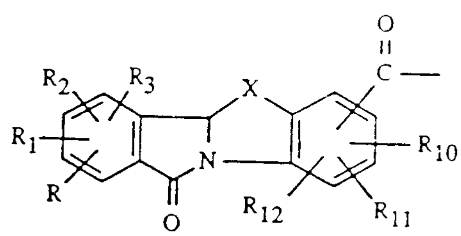

and

$G_3$ is a radical of the formula

(IX) 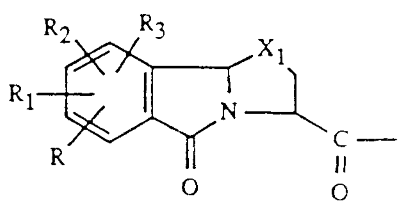

with the provisos (1) that only one substituent -CO-A, CO-$A_1$ or -CO-$A_2$ is present in the molecule, in which A, $A_1$ or $A_2$ is a radical -$G_1$-$(G)_n$, -$G_1$-($G_2$)n or -$G_1$-$(G_3)_n$ and (2) that in the case where X = S, a maximum of one of the substituents R and $R_1$ is $C_1$-$C_{18}$alkoxy.

11. A compound according to claim 10, in which R is hydrogen, hydroxyl, $C_1$-$C_4$alkyl, chlorine, nitro, $C_1$-$C_4$alkoxy, benzoyl, -N($R_7$)($R_{7a}$) or -CO-A, $R_1$ has the same possibilities of meaning as R, $R_2$ is hydrogen, or $C_1$-$C_4$alkyl and $R_3$ is hydrogen or chlorine, A is hydroxyl, $C_1$-$c_{18}$alkoxy, $C_5$-$C_6$cycloalkoxy, benzyloxy or benzyloxy substituted by $C_1$-$C_4$alkyl and/or chlorine, -N($R_7$)($R_{7a}$) or -$G_1$-$(G)_n$, n is a number from 1 to 3 and G is a radical of the formula

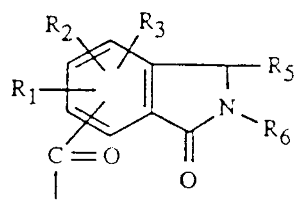

(II)

$G_1$ in the case where n = 1 is -O-$R_{20}$-O- or -NH-$R_{21}$-NH-, in the case where n = 2 it is a group of the formula

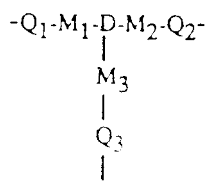

(III)

and in the case where n = 3 it is $(-OCH_2)_4C$, where D is equal to N, CH or C($C_1$-$C_4$alkyl), $Q_1$, $Q_2$ and $Q_3$ independently of one another are O or NH and $M_2$, $M_2$ and $M_3$ independently of one another are $C_1$-$C_6$alkylene or $C_2$-$C_{12}$alkylene interrupted by -O-, or in the case where D = CH or C($C_1$-$C_4$alkyl) -$M_3Q_3$ is otherwise -O- or in the case where D = N -$M_3$-$Q_3$ is otherwise a direct bond, $R_5$ and $R_6$ together form a group of the formula IV, V or VII, $R_7$ and $R_{7a}$ independently of one another are hydrogen, $C_1$-$C_6$alkyl, benzyl, $C_2$-$C_4$hydroxyalkyl or, together with the N atom to which they are bonded, are piperidyl, morpholinyl, piperazinyl or 4-methylpiperazinyl, $R_9$, $R_{10}$, $R_{11}$ and $R_{12}$ have the same possibilities of meaning as R, $R_1$, $R_2$ and $R_3$ (in this sequence), where -CO-A is to be replaced by -CO-$A_1$, $R_{13}$ is hydrogen, $C_1$-$C_{18}$alkyl, $C_2$-$C_{18}$alkenyl,$C_5$-$C_6$cycloalkyl, phenyl-$C_1$-$C_4$alkyl, $C_2$-$C_{18}$alkanoyl, $C_2$-$C_{18}$alkenoyl, benzoyl, ($C_1$-$C_6$alkyl)benzoyl, $C_2$-$C_{19}$alkoxycarbonyl-$C_1$-$C_4$alkyl, carboxy-$C_1$-$C_4$alkyl, cyano-$C_1$-$C_4$alkyl or hydroxy-$C_2$-$C_4$alkyl, $R_{14}$, $R_{15}$, $R_{16}$, $R_{17}$, $R_{18}$ and $R_{19}$ independently of one another are hydrogen, $C_1$-$C_4$alkyl, chlorine, hydroxyl, $C_1$-$C_4$alkoxy or -N($R_{7b}$)($R_{7c}$) in which $R_{7b}$ and $R_{7c}$ independently of one another are hydrogen or $C_1$-$C_6$alkyl, $R_{20}$ is $C_1$-$C_{12}$alkylene, $C_2$-$C_8$alkenylene, $C_3$-$C_6$alkynylene or $C_2$-$C_{24}$alkylene interrupted by O, S and/or -N($R_{22}$), $R_{21}$ is $C_1$-$C_{12}$alkylene and $R_{22}$ is hydrogen or $C_1$-$C_4$alkyl, $A_1$ is hydroxyl, $C_1$-$C_{18}$alkoxy, $C_5$-$C_6$cycloalkoxy, benzyloxy or benzyloxy substituted by $C_1$-$C_4$alkyl and/or halogen, -N($R_7$)($R_{7a}$) or -$G_1$-$(G_2)_n$ and $A_2$ is hydroxyl, $C_1$-$C_{18}$alkoxy, $C_5$-$C_6$cycloalkoxy, benzyloxy or benzyloxy substituted by $C_1$-$C_4$alkyl and/or halogen, -N($R_7$)($R_{7a}$) or -$G_1$-$(G_3)_n$, where $G_1$ and n are as defined above, $G_2$ is a radical of the formula

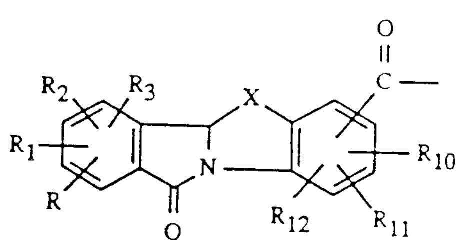

and

$G_3$ is a radical of the formula

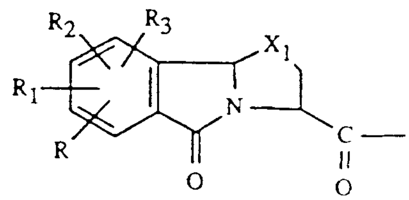

**12.** A compound according to claim 10, in which R is hydrogen, chlorine, hydroxyl or $C_1$-$C_4$alkyl, $R_1$ is hydrogen, chlorine, $C_1$-$C_4$alkyl or hydroxyl, $R_2$ and $R_3$ are hydrogen, $R_5$ and $R_6$ together form a group of the formula IV, V or VII, $R_9$, $A_{10}$, $A_{11}$ and $A_{12}$ have the same possibilities of meaning as R, $R_1$, $R_2$ and $R_3$ (in this sequence), $R_{13}$ is hydrogen, $C_1$-$C_{18}$alkyl, $C_5$-$C_6$cycloalkyl, benzyl, $C_2$-$C_{18}$alkanoyl, $C_2$-$C_{19}$alkoxycarbonyl-$C_1$-$C_4$alkyl or benzoyl, $R_{14}$, $R_{15}$, $R_{16}$, $R_{17}$, $R_{18}$ and $R_{19}$ are hydrogen, and $A_2$ is hydroxyl, $C_1$-$C_{18}$alkoxy, $C_5$-$C_6$cycloalkoxy or benzyloxy.

**13.** A compound according to claim 12, in which R is hydrogen, methyl or chlorine, $R_1$, $R_2$ and $R_3$ are hydrogen, and $R_5$ and $R_6$ together are a group of the formula IV, V or VII, in which X is -S-,

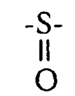

or

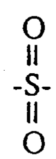 ,

$X_1$ is -S-, $R_9$ is hydrogen, $-CH_3$, Cl or -COO-($C_1$-$C_{24}$alkyl), $R_{10}$, $R_{11}$ and $R_{12}$ are hydrogen, $C_1$-$C_{18}$alkyl, benzoyl, $C_2$-$C_{19}$alkoxycarbonylmethyl or $C_2$-$C_{18}$alkanoyl, $R_{14}$ to $R_{19}$ are hydrogen and $A_2$ is $C_1$-$C_{24}$alkoxy.

**14.** A compound according to claim 10, in which R and $R_9$ independently of one another are hydrogen, $C_1$-$C_4$alkyl or chlorine and $R_1$, $R_2$, $R_3$, $R_{10}$, $R_{11}$, $R_{12}$, $R_{14}$, $R_{15}$, $R_{16}$, $R_{17}$, $R_{18}$ and $R_{19}$ are hydrogen.

**15.** The use of compounds of the formulae I and X according to claims 1 and 10 for stabilising organic material against oxidative, thermal and/or actinic degradation.

**Revendications**

**1.** Composition contenant un matériau organique, soumis à une dégradation thermique, par oxydation et/ou actinique, et au moins un composé de formule (I)

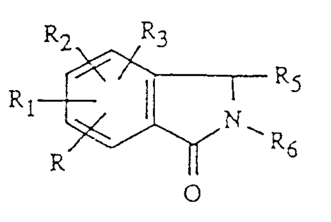

(I),

dans laquelle :

R est un hydrogène ou un radical alkyle en $C_1$-$C_{18}$,alcoxy en $C_1$-$C_{18}$, halogéno, nitro, phényl-(alcoxy en $C_1$-$C_4$), alcanoyle en $C_2$-$C_{18}$, benzoyle, (alkyle en $C_1$-$C_6$)-benzoyle, alcénoyle en $C_2$-$C_{18}$, -N($R_7$)($R_{7a}$), -OH ou -CO-A,

$R^1$ a les mêmes possibilités de signification que R,

$R^2$ est un hydrogène ou un radical alkyle en $C_1$-$C_4$,et

$R^3$ est un hydrogène ou un halogène,

A est un radical hydroxy, alcoxy en $C_1$-$C_{18}$, cycloalcoxy en $C_5$-$C_{12}$, benzyloxy ou encore benzyloxy substitué par des substituants alkyle en $C_1$-$C_6$ et/ou halogéno, -N($R_7$)($R_{7a}$), -NH-NH-$R_8$ ou encore -$G_1$-$(G)_n$,

n est un nombre de 1 à 3, et G est un résidu de formule

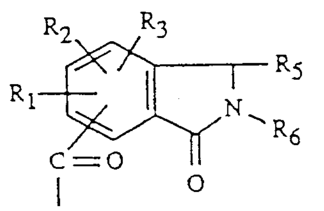

(II)

$G_1$, quand n = 1 est un radical -O-$R_{20}$-O-, -NH-$R_{21}$-NH- ou -NH-NH-,
quand n = 2, un groupe de formule

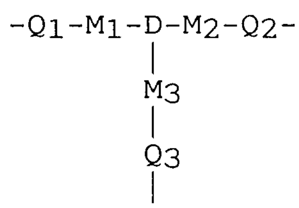

(III)

et quand n = 3, un radical $(-O-CH_2)_4C$ ou encore

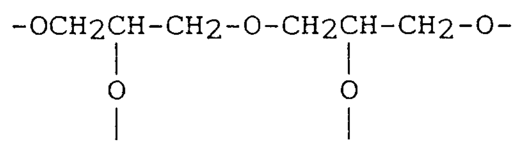

où D est N, CH ou un radical C(alkyle en $C_1$-$C_4$),$Q_1$, $Q_2$ et $Q_3$, indépendamment les uns des autres, correspondent chacun à O ou NH, et $M_1$, $M_2$ ou $M_3$, indépendamment les uns des autres, sont chacun un radical alkylène en $C_1$-$C_6$ ou encore alkylène en $C_2$-$C_{12}$ interrompu par -O-ou encore, quand D est CH ou un radical C(alkyle en $C_1$-$C_4$),-$M_3$-$Q_3$ peut aussi être -O-, ou encore, quand D est N, -$M_3$-$Q_3$ peut aussi être une liaison directe,

$R_5$ et $R_6$ forment ensemble un groupe de formule

(IV) 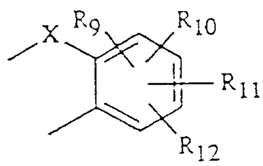 , 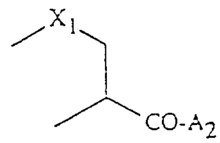 (V),

(VI) 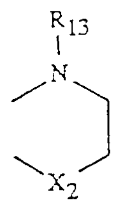 ou 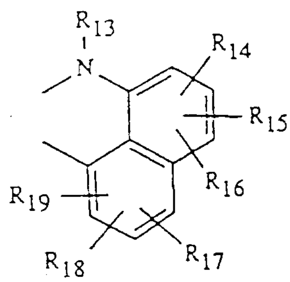 (VII)

X est -O-, -S-,

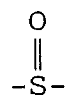 , 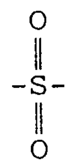

ou

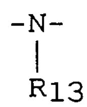

$X_1$ est -S-,

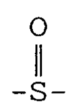

ou

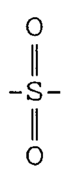 ,

et

$X_2$ est $-CH_2-$ ou une liaison directe,

$R_7$ et $R_{7a}$, indépendamment l'un de l'autre, sont chacun un hydrogène ou un radical alkyle en $C_1$-$C_{18}$, phényl-(alkyle en $C_1$-$C_4$), alcanoyle en $C_2$-$C_{18}$, alcénoyle en $C_2$-$C_{18}$, hydroxyalkyle en $C_2$-$C_4$, benzoyle ou (alkyle en $C_1$-$C_6$)-benzoyle, ou encore, avec l'atome d'azote auquel ils sont liés, sont des

radicaux pipéridyle, morpholinyle, pipérazinyle ou 4-méthylpipérazinyle,

$R_8$ est un hydrogène ou un radical alcanoyle en $C_2$-$C_{18}$, benzoyle ou (alkyle en $C_1$-$C_6$)-benzoyle,

$R_9$, $R_{10}$, $R_{11}$ et $R_{12}$ ont les mêmes possibilités de signification que R, $R_1$, $R_2$ et $R_3$ dans cet ordre, auquel cas, on peut remplacer -CO-A par -CO-$A_1$,

$R_{13}$ est un hydrogène ou un radical alkyle en $C_1$-$C_{18}$, alcényle en $C_2$-$C_{18}$, cycloalkyle en $C_5$-$C_{12}$, phényl(alkyle en $C_1$-$C_4$), alcanoyle en $C_2$-$C_{18}$, alcénoyle en $C_2$-$C_{18}$, benzoyle (alkyle en $C_1$-$C_6$)-benzoyle, cyano-(alkyle en $C_1$-$C_4$), (alcoxy en $C_2$-$C_{19}$)carbonyl-(alkyle en $C_1$-$C_4$), carboxy-(alkyle en $C_1$-$C_4$), hydroxy-(alkyle en $C_1$-$C_4$) ou

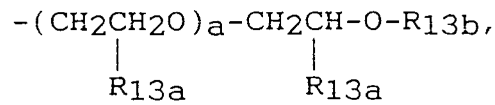

$R_{13a}$ est un hydrogène ou le radical méthyle, et $R_{13b}$ est un hydrogène ou un radical alkyle en $C_1$-$C_{18}$, phényl-(alkyle en $C_1$-$C_4$), alcanoyle en $C_1$-$C_{18}$, benzoyle, alcénoyle en $C_2$-$C_{18}$ ou encore (alkyle en $C_1$-$C_6$)-benzoyle, et a est un nombre de 0 à 6,

$R_{14}$, $R_{15}$, $R_{16}$, $R_{17}$, $R_{18}$ et $R_{19}$, indépendamment les uns des autres, sont chacun un hydrogène ou un radical alkyle en $C_1$-$C_4$, halogéno, hydroxy, alcoxy en $C_1$-$C_{18}$ ou encore -N($R_{7b}$)($R_{7c}$), où $R_{7b}$ et $R_{7c}$ sont chacun indépendamment de l'autre un hydrogène ou un radical alkyle en $C_1$-$C_{18}$,

$R_{20}$ est un radical alkylène en $C_1$-$C_{12}$, alcénylène en $C_2$-$C_8$, alcynyléne en $C_3$-$C_6$, ou encore alkylène en $C_2$-$C_{24}$ interrompu par O, S et/ou par -N($R_{22}$)-,

$R_{21}$ est un radical alkylène en $C_1$-$C_{12}$, et $R_{22}$ est un hydrogène ou un radical alkyle en $C_1$-$C_{18}$, phényle, (alkyle en $C_1$-$C_6$)-phényle, ou encore phényl-(alkyle en $C_1$-$C_4$), $A_1$ est un radical hydroxyle, alcoxy en $C_1$-$C_{24}$, cycloalcoxy en $C_5$-$C_{12}$, benzyloxy ou encore benzyloxy substitué par des substituants alkyle en $C_1$-$C_6$ et/ou halogéno, -N($R_7$)($R_{7a}$), -NH-NH-$R_8$ ou -$G_1$-$(G_2)_n$, et $A_2$ est un radical hydroxyle, alcoxy en $C_1$-$C_{24}$, cycloalcoxy en $C_5$-$C_{12}$, benzyloxy ou encore benzyloxy substitué par des substituants alkyle en $C_1$-$C_6$ et/ou halogéno, -N($R_7$)-($R_{7a}$), -NH-NH-$R_8$ ou encore -$G_1$-($G_3$)n, où $G_1$ et n sont tels que définis ci-dessus,

$G_2$ est un résidu de formule

(VIII) 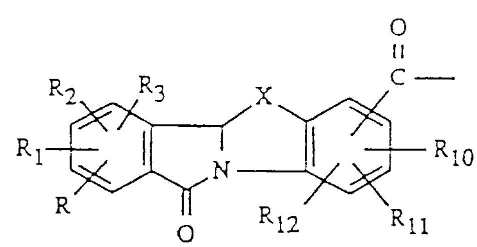

et $G_3$ est un résidu de formule

(IX) 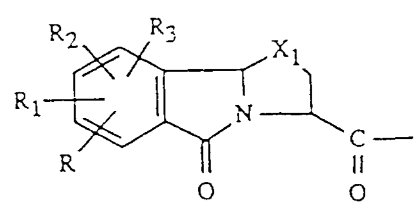

du moment que, dans la molécule, on a un seul substituant -CO-A, CO-$A_1$ ou -CO-$A_2$, dans lequel A, $A_1$ et $A_2$ sont respectivement des radicaux -$G_1$-$(G)_n$ et $G_1$-$(G_2)_n$.

**2.** Composition selon la revendication 1, dans laquelle :

R est un hydrogène ou un radical hydroxy, alkyle en $C_1$-$C_4$, chloro, nitro, alcoxy en $C_1$-$C_4$, benzoyle, -N($R_7$)-($R_{7a}$) ou -CO-A,

$R_1$ a les mêmes possibilités de signification que R,

$R_2$ est un hydrogène ou un radical alkyle en $C_1$-$C_4$, et

$R_3$ est un hydrogène ou le radical chloro,

A est un radical hydroxyle, alcoxy en $C_1$-$C_{18}$, cycloalcoxy en $C_5$-$C_6$, benzyloxy ou encore benzyloxy substitué par des substituants alkyle en $C_1$-$C_4$ et/ou chloro, -N($R_7$)($R_{7a}$) ou encore -$G_1$-$(G)_n$, n est un nombre de 1 à 3 et G est un radical de formule

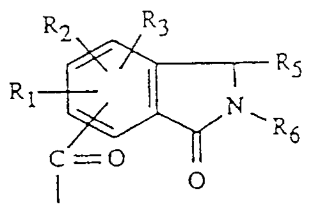

(II)

$G_1$, quand n = 1, est un radical -O-$R_{20}$-O- ou -NH-$R_{21}$-NH-,

quand n = 2, un groupe de formule

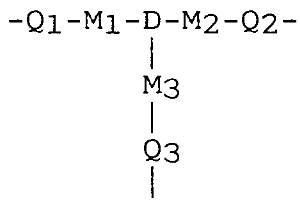

(III)

et quand n = 3, le radical $(-OCH_2)_4C$, où D est N, CH ou encore C(alkyle en $C_1$-$C_4$),

$Q_1$, $Q_2$ et $Q_3$, indépendamment les uns des autres sont chacun O ou NH et

$M_1$, $M_2$ et $M_3$, indépendamment les uns des autres, sont chacun un radical alkylène en $C_1$-$C_6$ ou un radical alkylène en $C_2$-$C_{12}$ interrompu par -O-,

ou bien encore, quand D est CH ou C(alkyle en $C_1$-$C_4$),-$M_3$-$Q_3$ peut aussi représenter -O-, ou encore, quand D est N, -$M_3$-$Q_3$ peut aussi être une liaison directe,

$R_5$ et $R_6$ forment ensemble un groupe de formules (IV), (V), (VI) ou (VII),

$R_7$ et $R_{7a}$, indépendamment l'un de l'autre, sont chacun un hydrogène ou un radical alkyle en $C_1$-$C_6$, benzyle, hydroxyalkyle en $C_2$-$C_4$, ou encore, avec l'atome d'azote auquel ils sont liés, représentent des radicaux pipéridyle, morpholinyle, pipérazinyle ou 4-méthylpipérazinyle,

$R_9$, $R_{10}$, $R_{11}$ et $R_{12}$ ont les mêmes possibilités de signification que R, $R_1$, $R_2$ et $R_3$ (dans cet ordre), auquel cas on peut utiliser à la place de -CO-A -CO-$A_1$,

$R_{13}$ est un hydrogène ou un radical alkyle en $C_1$-$C_{18}$, alcényle en $C_2$-$C_{18}$, cycloalkyle en $C_5$-$C_6$, phényl-(alkyle en $C_1$-$C_4$), alcanoyle en $C_2$-$C_{18}$, alcénoyle en $C_2$-$C_{18}$, benzoyle, (alkyle en $C_1$-$C_6$)-benzoyle (alcoxy en $C_2$-$C_{19}$)carbonyl-(alkyle en $C_1$-$C_4$), carboxy(alkyle en $C_1$-$C_4$), cyano-(alkyle en $C_1$-$C_4$) ou encore hydroxy-(alkyle en $C_2$-$C_4$),

$R_{14}$, $R_{15}$, $R_{16}$, $R_{17}$, $R_{18}$ et $R_{19}$, indépendamment les uns des autres, sont chacun un hydrogène ou un radical alkyle en $C_1$-$C_4$, chloro, hydroxy, alcoxy en $C_1$-$C_4$ ou encore -N($R_{7b}$)($R_{7c}$), où $R_{7b}$ et $R_{7c}$, indépendamment l'un de l'autre, sont chacun un hydrogène ou un radical alkyle en $C_1$-$C_6$,

$R_{20}$ est un radical alkylène en $C_1$-$C_{12}$, alcénylène en $C_2$-$C_8$, alcynylène en $C_3$-$C_6$, ou encore en $C_3$-$C_{24}$ interrompu par O, S et/ou -N($R_{22}$),

$R_{21}$ est un radical alkyléne en $C_1$-$C_{12}$, et $R_{22}$ est un hydrogène ou un radical en $C_1$-$C_4$, $A_1$ est un radical hydroxy, alcoxy en $C_1$-$C_{18}$, cycloalcoxy en $C_5$-$C_6$, benzyloxy ou encore benzyloxy substitué par des substituants alkyle en $C_1$-$C_4$ et/ou halogéno, -N($R_7$)($R_{7a}$) ou -$G_1$-($G_2$)n, et $A_2$ est un radical hydroxy, alcoxy en $C_1$-$C_{18}$,cycloalcoxy en $C_5$-$C_6$, benzyloxy ou encore benzyloxy substitué par des substituants alkyle en $C_1$-$C_4$ et/ou halogéno, -N($R_7$)($R_{7a}$) ou -$G_1$-($G_3$)n, où $G_1$ et n sont tels que définis ci-dessus,

$G_2$ est un radical de formule

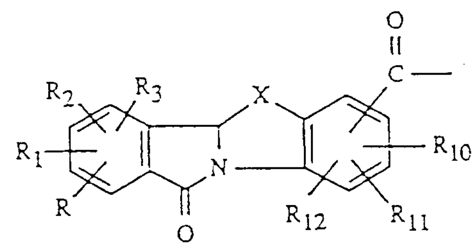

et $G_3$ est un radical de formule

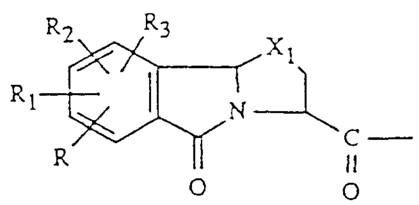

**3.** Composition selon la revendication 1, dans laquelle :
R est un hydrogène ou un radical chloro, hydroxy ou alkyle en $C_1$-$C_4$,
$R_1$ est un hydrogène ou un radical chloro, alkyle en $C_1$-$C_4$ ou hydroxy,
$R_2$ et $R_3$ sont des hydrogènes,
$R_5$ et $R_6$ forment ensemble un groupe de formules (IV), (V), (VI), (VII),
$R_9$, $R_{10}$, $R_{11}$ et $R_{12}$ ont les mêmes possibilités de signification que R, $R_1$, $R_2$ et $R_3$ (dans cet ordre),
$R_{13}$ est un hydrogène ou un radical alkyle en $C_1$-$C_{18}$, cycloalkyle en $C_5$-$C_6$, benzyle, (alcoxy en $C_2$-$C_{19}$)-carbonyl-(alkyle en $C_1$-$C_4$), carboxy-(alkyle en $C_1$-$C_4$), alcanoyle en $C_2$-$C_{18}$ ou benzoyle,
$R_{14}$, $R_{15}$, $R_{16}$, $R_{17}$, $R_{18}$ et $R_{19}$ sont chacun un hydrogène, et
$A_2$ est un radical hydroxy, alcoxy en $C_1$-$C_{18}$, cycloalcoxy en $C_5$-$C_6$ ou benzyloxy.

**4.** Composition selon la revendication 1, dans laquelle R et $R_9$, indépendamment l'un de l'autre, sont chacun un hydrogène ou un radical alkyle en $C_1$-$C_4$ ou chloro, et $R_1$, $R_2$, $R_3$, $R_{10}$, $R_{11}$, $R_{12}$, $R_{14}$, $R_{15}$, $R_{16}$, $R_{17}$, $R_{18}$ et $R_{19}$ sont des hydrogènes.

**5.** Composition selon la revendication 1, dans laquelle A, $A_1$ et $A_2$ sont différents respectivement de $-G_1-(G)_n$, de $-G_1-(G_2)_n$ et de $-G_1-(G_3)_n$.

**6.** Composition selon la revendication 1, dans laquelle R est un hydrogène ou le radical méthyle ou chloro, $R_1$, $R_2$ et $R_3$ sont des hydrogénes, et $R_5$ et $R_6$ forment ensemble un groupe de formules (IV), (V), (VI) ou (VII), où X est -S-,

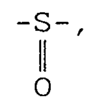

ou encore

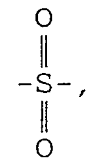

$X_1$ est -S-,

$X_2$ est $-CH_2-$, $R_9$ est un hydrogène, $-CH_3$, Cl ou -COO-(alkyle en $C_1$-$C_{24}$), $R_{10}$, $R_{11}$ et $R_{12}$ sont des hydrogènes, $R_{13}$ est un hydrogène ou un radical alkyle en $C_1$-$C_{18}$, benzoyle, (alcoxy en $C_2$-$C_{19}$)-carbonylméthyle ou encore alcanoyle en $C_2$-$C_{18}$, $R_{14}$ à $R_{19}$ sont des hydrogènes, et $A_2$ est un radical alcoxy en $C_1$-$C_{24}$.

**7.** Composition selon la revendication 1, dans laquelle la matiére organique est un lubrifiant, un fluide pour l'usinage des métaux, un fluide hydraulique ou un polymère naturel ou (semi-)synthétique, en particulier synthétique.

**8.** Composition selon la revendication 7, dans laquelle la matière organique est un matériau thermoplastique ou un élastomère.

**9.** Composition selon la revendication 1, qui contient en outre d'autres stabilisants, tels que des antioxydants, des agents de protection contre la lumière et des stabilisants de mise en oeuvre (stabilisants thermiques), en particulier un phosphite organique.

**10.** Composés de formule (X)

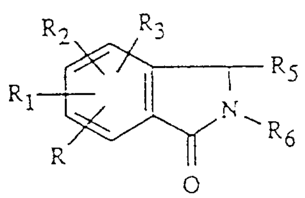

(X)

dans laquelle :

R est un hydrogène ou un radical alkyle en $C_1$-$C_{18}$,alcoxy en $C_1$-$C_{18}$, nalogéno, nitro, phényl-(alcoxy en $C_1$-$C_4$), alcanoyle en $C_2$-$C_{18}$, benzoyle, (alkyle en $C_1$-$C_6$)-benzoyle, alcénoyle en $C_2$-$C_{18}$, $-N(R_7)(R_{7a})$, -OH ou -CO-A,

$R^1$ a les mêmes possibilités de signification que R,

$R^2$ est un hydrogène ou un radical alkyle en $C_1$-$C_4$,et

$R^3$ est un hydrogène ou un halogène,

A est un radical hydroxy, alcoxy en $C_1$-$C_{18}$, cycloalcoxy en $C_5$-$C_{12}$, benzyloxy ou encore benzyloxy substitué par des substituants alkyle en $C_1$-$C_6$ et/ou halogéno, $-N(R_7)(R_{7a})$, $-NH-NH-R_8$ ou encore $-G_1-(G)_n$,

n est un nombre de 1 à 3, et G est un résidu de formule

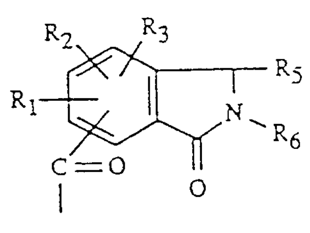

(II)

$G_1$, quand n = 1 est un radical $-O-R_{20}-O-$, $-NH-R_{21}-NH-$ ou -NH-NH-,

quand n = 2, un groupe de formule

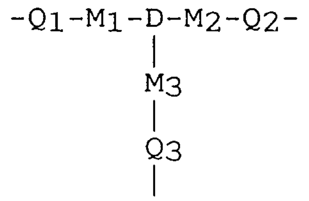

(III)

et quand n = 3, un radical $(-O-CH_2)_4C$ ou encore

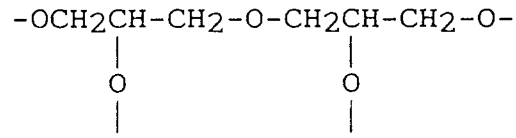

où D est N, CH ou un radical C(alkyle en $C_1$-$C_4$),$Q_1$, $Q_2$ et $Q_3$, indépendamment les uns des autres, correspondent chacun à O ou NH, et $M_1$, $M_2$ ou $M_3$, indépendamment les uns des autres, sont chacun un radical alkylène en $C_1$-$C_6$ ou encore alkylène en $C_2$-$C_{12}$ interrompu par -O-, ou encore, quand D est CH ou un radical C(alkyle en $C_1$-$C_4$), $-M_3-Q_3$ peut aussi être -O-, ou encore, quand D est N, $-M_3-Q_3$ peut aussi être une liaison directe,

$R_5$ et $R_6$ forment ensemble un groupe de formules (IV), (V) ou (VII)

(IV) 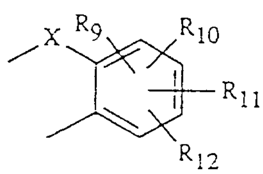 , 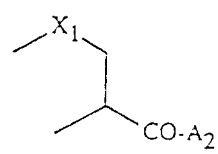 (V),

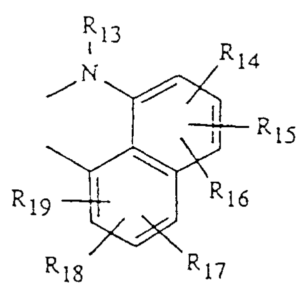 (VII)

X est -S-,

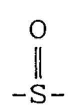

ou

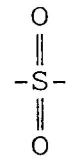

$X_1$ est -S-,

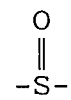

ou

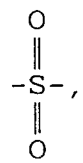

et

où, quand X est S ou S=O, et que l'un des substituants $R_9$ à $R_{12}$ est H, un radical alcoxy ou halogéno, les trois autres de ces substituants ne peuvent pas être simultanément des hydrogènes,

$R_7$ et $R_{7a}$, indépendamment l'un de l'autre, sont chacun un hydrogène ou un radical alkyle en $C_1$-$C_{18}$, phényl-(alkyle en $C_1$-$C_4$), alcanoyle en $C_2$-$C_{18}$, alcénoyle en $C_2$-$C_{18}$, hydroxyalkyle en $C_2$-$C_4$, benzoyle ou (alkyle en $C_1$-$C_6$)-benzoyle, ou encore avec l'atome d'azote auquel ils sont liés, sont des radicaux pipéridyle, morpholinyle, pipérazinyle ou 4-méthylpipérazinyle,

$R_8$ est un hydrogène ou un radical alcanoyle en $C_2$-$C_{18}$, benzoyle ou (alkyle en $C_1$-$C_6$)-benzoyle,

$R_9$, $R_{10}$, $R_{11}$ et $R_{12}$ ont les mêmes possibilités de signification que R, $R_1$, $R_2$ et $R_3$ dans cet ordre, auquel cas, on peut remplacer -CO-A par -CO-$A_1$,

$R_{13}$ est un hydrogène ou un radical alkyle en $C_1$-$C_{18}$, alcényle en $C_2$-$C_{18}$, cycloalkyle en $C_5$-$C_{12}$, phényl-(alkyle en $C_1$-$C_4$), alcanoyle en $C_2$-$C_{18}$, alcénoyle en $C_2$-$C_{18}$, benzoyle, (alkyle en $C_1$-$C_6$)-benzoyle, benzyle, (alcoxy en $C_2$-$C_{19}$)carbonyl-(alkyle en $C_1$-$C_4$), carboxy(alkyle en $C_1$-$C_4$), cyano-(alkyle en $C_1$-$C_4$), hydroxy-(alkyle en $C_2$-$C_4$) ou

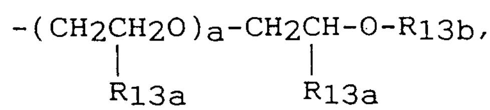

$R_{13a}$ est un hydrogéne ou le radical méthyle, et $R_{13b}$ est un hydrogène ou un radical alkyle en $C_1$-$C_{18}$, phényl-(alkyle en $C_1$-$C_4$), alcanoyle en $C_1$-$C_{18}$, benzoyle, alcénoyle en $C_2$-$C_{18}$ ou encore (alkyle en $C_1$-$C_6$)-benzoyle, et a est un nombre de 0 à 6,

$R_{14}$, $R_{15}$, $R_{16}$, $R_{17}$, $R_{18}$ et $R_{19}$, indépendamment les uns des autres, sont chacun un hydrogène ou un radical alkyle en $C_1$-$C_4$, halogéno, hydroxy, alcoxy en $C_1$-$C_{18}$ ou encore -N($R_{7b}$)($R_{7c}$), où $R_{7b}$ et $R_{7c}$ sont chacun indépendamment de l'autre un hydrogène ou un radical alkyle en $C_1$-$C_{18}$,

$R_{20}$ est un radical alkylène en $C_1$-$C_{12}$, alcénylène en $C_2$-$C_8$, alcynylène en $C_3$-$C_6$, ou encore alkylène en $C_2$-$C_{24}$ interrompu par O, S et/ou par -N($R_{22}$)-,

$R_{21}$ est un radical alkylène en $C_1$-$C_{12}$, et $R_{22}$ est un hydrogène ou un radical alkyle en $C_1$-$C_{18}$, phényle, (alkyle en $C_1$-$C_6$)-phényle, ou encore phényl-(alkyle en $C_1$-$C_4$), $A_1$ est un radical hydroxyle, alcoxy en $C_1$-$C_{24}$, cycloalcoxy en $C_5$-$C_{12}$, benzyloxy ou encore benzyloxy substitué par des substituants alkyle en $C_1$-$C_6$ et/ou halogéno, -N($R_7$)($R_{7a}$), -NH-NH-$R_8$ ou -$G_1$-$(G_2)_n$, et $A_2$ est un radical hydroxyle, alcoxy en $C_1$-$C_{24}$, cycloalcoxy en $C_5$-$C_{12}$, benzyloxy ou encore benzyloxy substitué par des substituants alkyle en $C_1$-$C_6$ et/ou halogéno, -N($R_7$)-($R_{7a}$), -NH-NH-$R_8$ ou encore -$G_1$-$(G_3)_n$, où $G_1$ et n

sont tels que définis ci-dessus,

$G_2$ est un résidu de formule

(VIII) 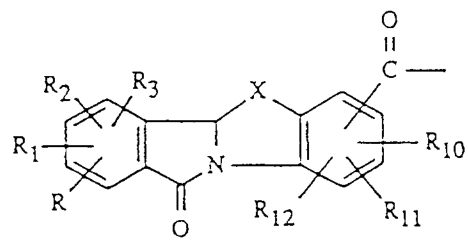

et $G_3$ est un résidu de formule

(IX) 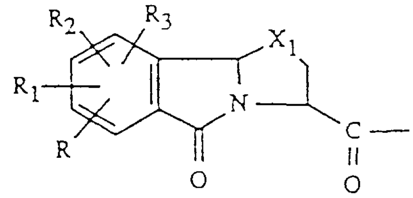

du moment que

1) dans la molécule, on n'a qu'un seul substituant -CO-A, -CO-$A_1$ ou -CO-$A_2$, où A, $A_1$, $A_2$ représentent respectivement les radicaux -$G_1$-$(G)_n$, -$G_1$-($G_2$)n et -$G_1$-$(G_3)_n$ et

2) que quand X = S, au plus l'un des substituants R et $R_1$ est un radical alcoxy en $C_1$-$C_{18}$.

**11.** Composés selon la revendication 10, dans laquelle :

R est un hydrogène ou un radical hydroxy, alkyle en $C_1$-$C_4$, chloro, nitro, alcoxy en $C_1$-$C_4$, benzoyle, -N($R_7$)-($R_{7a}$) ou -CO-A,

$R_1$ a les mêmes possibilités de signification que R,

$R_2$ est un hydrogène ou un radical alkyle en $C_1$-$C_4$, et

$R_3$ est un hydrogène ou le radical chloro,

A est un radical hydroxyle, alcoxy en $C_1$-$C_{18}$, cycloalcoxy en $C_5$-$C_6$, benzyloxy ou encore benzyloxy substitué par des substituants alkyle en $C_1$-$C_4$ et/ou chloro, -N($R_7$)($R_{7a}$) ou encore -$G_1$-$(G)_n$, n est un nombre de 1 à 3 et G est un radical de formule

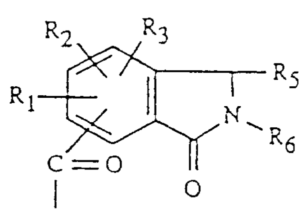 (II)

$G_1$, quand n = 1, est un radical -O-$R_{20}$-O- ou -NH-$R_{21}$-NH-,

quand n = 2, un groupe de formule

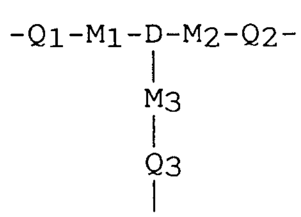 (III)

et quand n = 3, le radical $(-OCH_2)_4C$, où D est N, CH ou encore C(alkyle en $C_1$-$C_4$),

$Q_1$, $Q_2$ et $Q_3$, indépendamment les uns des autres sont chacun O ou NH et

$M_1$, $M_2$ et $M_3$, indépendamment les uns des autres, sont chacun un radical alkylène en $C_1$-$C_6$ ou un radical alkylène en $C_2$-$C_{12}$ interrompu par -O-,

ou bien encore, quand D est CH ou C(alkyle en $C_1$-$C_4$),-$M_3$-$Q_3$ peut aussi représenter -O-, ou encore, quand D est N, -$M_3$-$Q_3$ peut aussi être une liaison directe,

$R_5$ et $R_6$ forment ensemble un groupe de formules (IV), (V) ou (VII),

$R_7$ et $R_{7a}$, indépendamment l'un de l'autre, sont chacun un hydrogène ou un radical alkyle en $C_1$-$C_6$, benzyle, hydroxyalkyle en $C_2$-$C_4$, ou encore, avec l'atome d'azote auquel ils sont liés, représentent des radicaux pipéridyle, morpholinyle, pipérazinyle ou 4-méthylpipérazinyle,

$R_9$, $R_{10}$, $R_{11}$ et $R_{12}$ ont les mêmes possibilités de signification que R, $R_1$, $R_2$ et $R_3$ (dans cet ordre), auquel cas on peut utiliser à la place de -CO-A -CO-$A_1$,

$R_{13}$ est un hydrogène ou un radical alkyle en $C_1$-$C_{18}$, alcényle en $C_2$-$C_{18}$, cycloalkyle en $C_5$-$C_6$, phényl-(alkyle en $C_1$-$C_4$), alcanoyle en $C_2$-$C_{18}$, alcénoyle en $C_2$-$C_{18}$, benzoyle, (alkyle en $C_1$-$C_6$)-benzoyle (alcoxy en $C_2$-$C_{19}$)carbonyl-(alkyle en $C_1$-$C_4$), carboxy(alkyle en $C_1$-$C_4$), cyano-(alkyle en $C_1$-$C_4$) ou encore hydroxy-(alkyle en $C_2$-$C_4$),

$R_{14}$, $R_{15}$, $R_{16}$, $R_{17}$, $R_{18}$ et $R_{19}$, indépendamment les uns des autres, sont chacun un hydrogène ou un radical alkyle en $C_1$-$C_4$, chloro, hydroxy, alcoxy en $C_1$-$C_4$ ou encore -N($R_{7b}$)($R_{7c}$), où $R_{7b}$ et $R_{7c}$, indépendamment l'un de l'autre, sont chacun un hydrogène ou un radical alkyle en $C_1$-$C_6$,

$R_{20}$ est un radical alkylène en $C_1$-$C_{12}$, alcénylène en $C_2$-$C_8$, alcynylène en $C_3$-$C_6$, ou encore en $C_2$-$C_{24}$ interrompu par O, S et/ou -N($R_{22}$),

$R_{21}$ est un radical alkylène en $C_1$-$C_{12}$, et $R_{22}$ est un hydrogène ou un radical en $C_1$-$C_4$, $A_1$ est un radical hydroxy, alcoxy en $C_1$-$C_{18}$, cycloalcoxy en $C_5$-$C_6$, benzyloxy ou encore benzyloxy substitué par des substituants alkyle en $C_1$-$C_4$ et/ou halogéno, -N($R_7$)($R_{7a}$) ou - $G_1$-$(G_2)_n$, et $A_2$ est un radical hydroxy, alcoxy en $C_1$-$C_{18}$,cycloalcoxy en $C_5$-$C_6$, benzyloxy ou encore benzyloxy substitué par des substituants alkyle en $C_1$-$C_4$ et/ou halogéno, -N($R_7$)($R_{7a}$) ou -$G_1$-$(G_3)_n$, où $G_1$ et n sont tels que définis ci-dessus,

$G_2$ est un radical de formule

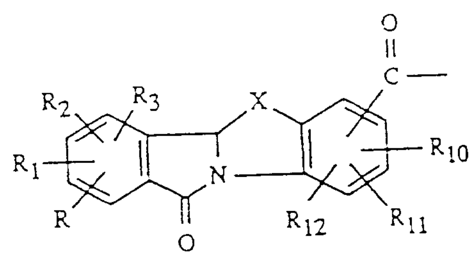

et $G_3$ est un radical de formule

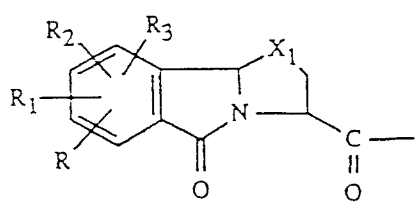

**12.** Composés selon la revendication 10, dans lesquels R est un hydrogène ou un radical chloro, hydroxy ou alkyle en $C_1$-$C_4$,

$R_1$ est un hydrogène ou un radical chloro, alkyle en $C_1$-$C_4$ ou hydroxy,

$R_2$ et $R_3$ sont des hydrogènes,

$R_5$ et $R_6$ forment ensemble un groupe de formules (IV), (V), (VII),

$R_9$, $R_{10}$, $R_{11}$ et $R_{12}$ ont les mêmes possibilités de signification que R, $R_1$, $R_2$ et $R_3$ (dans cet ordre),

$R_{13}$ est un hydrogène ou un radical alkyle en $C_1$-$C_{18}$, cycloalkyle en $C_5$-$C_6$, benzyle, (alcoxy en $C_2$-$C_{19}$)-carbonyl-(alkyle en $C_1$-$C_4$), carboxy-(alkyle en $C_1$-$C_4$), alcanoyle en $C_2$-$C_{18}$ ou benzoyle,

$R_{14}$, $R_{15}$, $R_{16}$, $R_{17}$, $R_{18}$ et $R_{19}$ sont chacun un hydrogène, et

$A_2$ est un radical hydroxy, alcoxy en $C_1$-$C_{18}$, cycloalcoxy en $C_5$-$C_6$ ou benzyloxy.

**13.** Composés selon la revendication 12, dans lesquels R est un hydrogène ou le radical méthyle ou chloro, $R_1$, $R_2$ et $R_3$ sont des hydrogènes, et $R_5$ et $R_6$ forment ensemble un groupe de formules (IV), (V) ou (VII), où X est -S-,

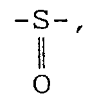

ou encore

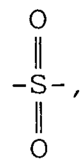

$X_1$ est -S-,

$X_2$ est $-CH_2-$, $R_9$ est un hydrogène, $-CH_3$, Cl ou -COO-(alkyle en $C_1$-$C_{24}$), $R_{10}$, $R_{11}$ et $R_{12}$ sont des hydrogènes, $R_{13}$ est un hydrogène ou un radical alkyle en $C_1$-$C_{18}$, benzoyle, (alcoxy en $C_2$-$C_{19}$)-carbonylméthyle ou encore alcanoyle en $C_2$-$C_{18}$, $R_{14}$ à $R_{19}$ sont des hydrogènes, et $A_2$ est un radical alcoxy en $C_1$-$C_{24}$.

**14.** Composés selon la revendication 10, dans lesquels R et $R_9$, indépendamment l'un de l'autre, sont chacun un hydrogène ou un radical alkyle en $C_1$-$C_4$ ou chloro, et $R_1$, $R_2$, $R_3$, $R_{10}$, $R_{11}$, $R_{12}$, $R_{14}$, $R_{15}$, $R_{16}$, $R_{17}$, $R_{18}$ et $R_{19}$ sont des hydrogènes.

**15.** Utilisation de composés de formules (I) et (X) selon les revendications 1 et 10 pour stabiliser une matière organique vis-à-vis d'une dégradation thermique, par oxydation et/ou actinique.